(19) Europäisches Patentamt
European Patent Office
Office européen des brevets

(11) EP 2 318 536 B1

(12) **FASCICULE DE BREVET EUROPEEN**

(45) Date de publication et mention de la délivrance du brevet:
**01.04.2020 Bulletin 2020/14**

(21) Numéro de dépôt: **09740394.3**

(22) Date de dépôt: **27.07.2009**

(51) Int Cl.:
*C12N 15/82* (2006.01) *C12N 15/29* (2006.01)
*A01H 1/04* (2006.01) *A01H 5/00* (2018.01)
*C12N 9/88* (2006.01) *C07K 14/415* (2006.01)
*C12N 15/60* (2006.01) *C12N 5/10* (2006.01)
*C12N 5/04* (2006.01) *C12Q 1/68* (2018.01)

(86) Numéro de dépôt international:
**PCT/FR2009/051510**

(87) Numéro de publication internationale:
**WO 2010/012948 (04.02.2010 Gazette 2010/05)**

(54) **COMBINAISON DE DEUX ELEMENTS GENETIQUES POUR LE CONTROLE DU DEVELOPPEMENT DU TYPE FLORAL D'UNE PLANTE DICOTYLEDONE, ET MISE EN OEUVRE DANS DES PROCEDES DE DETECTION ET DE SELECTION**

KOMBINATION VON ZWEI GENETISCHEN ELEMENTEN ZUR KONTROLLE DER BLÜTENENTWICKLUNG EINER ZWEIKEIMBLÄTTRIGEN PFLANZE, DEREN ANWENDUNG BEI NACHWEIS- UND SELEKTIONSVERFAHREN

COMBINATION OF TWO GENETIC ELEMENTS FOR THE CONTROL OF THE FLOWER DEVELOPMENT OF A DICOTYLDENOUS PLANT , ITS APPLICATION IN METHODS OF DETECTION AND SELECTION

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **28.07.2008 FR 0855146**

(43) Date de publication de la demande:
**11.05.2011 Bulletin 2011/19**

(73) Titulaire: **Institut national de recherche pour l'agriculture, l'alimentation et l'environnement
75007 Paris (FR)**

(72) Inventeurs:
- **BENDAHMANE, Abdelhafid
F-91830 Le Coudray Montceaux (FR)**
- **BOUALEM, Adnane
F-03300 Cusset (FR)**
- **TROADEC, Christelle
F-94200 Ivry-sur-Seine (FR)**
- **ANTOINE, Martin
F-91190 Gif sur Yvette (FR)**
- **DOGIMONT, Catherine
F-84810 Aubignan (FR)**

(74) Mandataire: **Jacobacci Coralis Harle
32, rue de l'Arcade
75008 Paris (FR)**

(56) Documents cités:
**WO-A-2005/035752 FR-A- 2 900 415**

- **DATABASE EMBL [Online] 25 septembre 2007 (2007-09-25), "Cucumis melo subsp. agrestis EST, clone A_14-B10-M13R" XP002521045 extrait de EBI accession no. EMBL:AM727647 Database accession no. AM727647**
- **SAGASSER MARTIN ET AL: "A. thaliana TRANSPARENT TESTA 1 is involved in seed coat development and defines the WIP subfamily of plant zinc finger proteins." GENES & DEVELOPMENT 1 JAN 2002, vol. 16, no. 1, 1 janvier 2002 (2002-01-01), pages 138-149, XP002521044 ISSN: 0890-9369 cité dans la demande**
- **MARTIN ANTOINE ET AL: "A transposon-induced epigenetic change leads to sex determination in melon." 22 octobre 2009 (2009-10-22), NATURE 22 OCT 2009, VOL. 461, NR. 7267, PAGE(S) 1135 - 1138 , XP002568870 ISSN: 1476-4687 le document en entier**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

### DOMAINE DE L'INVENTION

La présente invention se rapporte au domaine de la sélection de variétés de plantes, et en particulier à la sélection du type sexuel des plantes. Elle est relative à la détection génotypique du sexe des plantes par analyse du polymorphisme d'un gène A et d'un gène G, ainsi qu'à des moyens de mise en œuvre de cette détection et à des procédés d'obtention de plantes dont le phénotype sexuel est modifié.

### ART ANTERIEUR

La réalisation de plantes hybrides est d'un grand intérêt en agronomie et en agriculture. En effet, les plantes hybrides, grâce au phénomène d'hétérosis appelé également vigueur hybride, présentent une supériorité pour de nombreux caractères, par rapport à la moyenne de leurs deux parents. Cette supériorité peut s'illustrer par exemple par une meilleure vigueur, un meilleur rendement, une plus grande adaptation au milieu dans lequel l'hybride est cultivé, et une grande uniformité des hybrides par rapport à ses parents. Cette vigueur hybride est d'autant plus importante que les parents sont éloignés génétiquement.

La création de lignées pures et stables, futurs parents de l'hybride est un passage obligé pour la création de variété hybrides homogènes et reproductibles exprimant le plus d'hétérosis. Il est donc nécessaire de créer des lignées pures et stables, puis de croiser ces lignées pour obtenir des hybrides.

La création de lignées pures implique l'autofécondation d'une plante de sorte à obtenir des plantes présentant un même patrimoine génétique, fixé pour l'ensemble des caractères de productivité, régularité du rendement, ou encore de résistance aux maladies, recherchées.

Pour créer des lignées pures, Il est donc nécessaire d'utiliser des plantes dont le type sexuel permet l'autofécondation, par exemple des plantes hermaphrodites.

Or, beaucoup de plantes dicotylédones, et en particulier les cucurbitaceae peuvent être monoïque, andromonoïque, gynoïques ou hermaphrodites.

Une première technique mise en œuvre, pour l'obtention de lignées pures, consiste à réaliser un traitement chimique des plantes de sorte à obtenir des plantes aptes à s'autoféconder, par exemple des plantes hermaphrodites. Chez le melon (*cucumis melo*) par exemple, la pulvérisation d'inhibiteurs de la synthèse de l'éthylène tel que le nitrate d'argent ou le thiosulfate d'argent entraîne l'apparition temporaire d'étamines dans les fleurs femelles (Rudich *et al.*, 1969 ; Risser *et al.*, 1979). De cette manière, la transformation des plantes gynoïques en plantes hermaphrodites est utilisée pour le maintien de lignées pures.

Cependant, la production de lignées pures par cette méthode est limitée par le coût des agents chimiques, leur durée d'action, et leurs effets phytotoxiques. De plus, de tels agents peuvent ne pas être efficaces en ce qui concerne la réalisation d'hybrides à partir de plantes dont la durée de floraison est longue, car de nouvelles fleurs apparues après traitement pourraient ne pas être affectées par le traitement chimique.

Il existe donc un besoin pour un système qui permettrait de contrôler le développement du type floral d'une plante dicotylédone, et d'obtenir une plante d'un type floral déterminé.

De plus, de très nombreux croisements sont nécessaires pour obtenir des hybrides intéressants, à partir de lignées pures, et à chaque croisement, les plants présentant le phénotype le plus prometteur sont retenus.

Lors du croisement des lignées pures entre elles, il est indispensable de pouvoir choisir le sens du croisement effectué, et d'éviter l'auto pollinisation des plantes qui conduirait à des plantes ne présentant pas la vigueur hybride recherchée.

Là encore, du fait de la diversité du type sexuel des plantes dicotylédones, il est nécessaire de séparer les fleurs mâles et les fleurs femelles d'un même plant pour éviter l'autopollinisation.

Une première technique, mise en œuvre notamment pour le maïs, consiste à utiliser des moyens mécaniques pour réaliser une émasculation des plantes. Cependant cette technique s'avère extrêmement coûteuse puisqu'elle nécessite l'émasculation de chaque plante dont on veut éviter l'autopollinisation, pour chaque croisement effectué.

Une autre technique consiste à réaliser une émasculation chimique des plantes, bloquant la formation de pollen viable. Ainsi, chez le melon (*Cucumis melo*), le traitement de plantes monoïques par de l'éthrel (précurseur de l'éthylène) entraîne la disparition temporaire des fleurs mâles.

De tels agents chimiques, appelés gamétocides, utilisés pour provoquer une stérilité mâle transitoire présentent plusieurs inconvénients, comme un coût élevé ou une grande toxicité, comme cela a été rappelé ci-dessus.

Les techniques mécaniques ou chimiques de contrôle du type floral décrites ci-dessus s'avèrent donc très coûteuses, d'autant que de très nombreux croisements sont nécessaires pour obtenir des plants hybrides présentant les caractères recherchés et pouvant être commercialisés.

Pour faciliter la création de lignées pures et d'hybrides, Il existe donc également un besoin pour un système qui permettrait de contrôler le développement du type floral d'une plante dicotylédone, et d'obtenir une plante d'un type floral déterminé.

Une autre voie pour obtenir des plants capables d'autopollinisation utiles pour la création de lignées pures, ou non capables d'autopollinisation, pour la création d'hybrides, pourrait consister respectivement en une sélection d'individus exclusivement hermaphrodites, ou exclusivement femelles, présents dans une espèce. Cependant, une telle technique s'avèrerait extrêmement coûteuse elle aussi, puisqu'elle nécessiterait la culture d'un nombre très important de plantes, jusqu'au moment où il est possible d'en déterminer le type sexuel. Cette technique serait de plus aléatoire, car les mécanismes de détermination du sexe des fleurs dépendent notamment de facteurs environnementaux.

Selon encore une autre voie, on a cherché à identifier et caractériser les déterminants génétiques participant au contrôle du type floral chez le melon. Chez le melon, le contrôle génétique de détermination du sexe est gouverné par deux déterminants génétiques principaux, respectivement (1) le déterminant génétique andromonoïque (« *andromonoecious»* ou « a ») et (2) le déterminant génétique gynoïque (« *gynoecious* » ou « g »), chaque déterminant possédant au moins deux allèles, et dont les combinaisons produisent une grande variété de phénotypes sexuels. La demande internationale PCT publiée sous le n° WO2007/125264 décrit l'identification et la caractérisation du déterminant génétique (a), qui s'est avéré consister en un gène codant une aminocyclopropane carboxylate synthase (ACS). Ainsi, la demande PCT n° WO 2007/125264 fournit les moyens de détection et de contrôle permettant de sélectionner ou de générer des plantes dicotylédones possédant l'allèle (A) dominant ou l'allèle (a) récessif. Le déterminant génétique (g) restait totalement inconnu. Tout au plus, des données préliminaires suggéraient que le déterminant génétique (g), de nature et de structure inconnues, pouvait être localisé dans une large région génomique de plus de 2,4 mégabases délimitée par des marqueurs désignés M8 et M30. Du fait qu'il est communément admis qu'il existe en moyenne 12 cadres ouverts de lecture (« ORFs ») dans 100 kilobases de génome végétal, la région génomique délimitée entre les marqueurs M8 et M30 était susceptible de contenir environ 300 cadres ouverts de lecture.

Toutefois, en l'absence de caractérisation du second déterminant génétique gynoïque (ou « g »), il n'était pas possible de mettre à la disposition du public des moyens de sélection ou de contrôle du développement du type floral permettant, par exemple de discriminer ou de générer une population de plantes strictement femelles, puisque ce phénotype est contrôlé exclusivement par le déterminant génétique (g). Egalement, la sélection ou l'obtention d'une population de plantes exclusivement hermaphrodites ne serait possible qu'après l'identification et la caractérisation du déterminant génétique (g).

Il existe donc également un besoin pour un procédé qui permettrait de sélectionner des plantes dycotylédones par exemple hermaphrodites ou femelles, sans devoir pour autant les cultiver.

Ce procédé devrait permettre de sélectionner des plantes, particulièrement utiles pour la réalisation de lignées pures ou d'hybrides comme cela a été précisé ci-dessus.

## RESUME DE L'INVENTION

Selon l'invention, on a identifié et caractérisé le déterminant génétique (g) gynoïque pour le contrôle du développement floral d'une plante dicotylédone qui est un angiosperme, et plus précisément une plante de la famille des *cucurbitaceae*.

L'identification et la caractérisation du déterminant génétique (g) gynoïque a permis pour la première fois la mise au point d'une combinaison des deux déterminants génétiques (a) andromonoïque et (g) gynoïque permettant de contrôler en totalité le développement du type floral d'une plante dicotylédone, quel que soit le phénotype sexuel qui est considéré.

L'invention se rapporte donc une combinaison des deux éléments génétiques (A/a) et (G/g) qui permet de contrôler le développement du type floral d'une plante dicotylédone, en particulier d'un cucurbitacée comme le melon.

Il avait été déjà montré dans l'état de la technique que, physiologiquement, les deux allèles (A) et (a) se distinguent par des taux d'activité enzymatique différents d'une protéine, la aminocyclopropane carboxylate synthase aussi désignée ACS.

Les inventeurs ont maintenant montré que, physiologiquement, les deux allèles (G) et (g), qui ont été identifiés et caractérisés selon l'invention, se distinguent par des taux différents d'une nouvelle protéine, la protéine CmWIP1.

L'invention a donc pour objet l'utilisation d'une combinaison de deux éléments génétiques pour le contrôle du développement du type floral d'une plante dicotylédone, ladite combinaison comprenant respectivement :

a) un premier élément génétique de contrôle (A/a) présent dans ladite plante dicotylédone, sous la forme d'un allèle dominant (A), et d'un allèle récessif (a), dans lequel :

- l'allèle dominant (A), consiste en un acide nucléique (NA) permettant l'expression la protéine ACS (aminocyclopropane carboxylate synthase) de séquence SEQ ID N°3,
- l'allèle récessif (a), se distingue de l'allèle dominant en ce que ledit allèle récessif (a) est non fonctionnel dans ladite plante dicotylédone, et

b) un second élément génétique de contrôle (G/g) présent dans ladite plante dicotylédone, sous la forme d'un allèle dominant (G), et d'un allèle récessif (g), dans lequel :

- l'allèle dominant (G), consiste en un acide nucléique (NG) permettant l'expression de la protéine CmWIP1 de séquence SEQ ID N°12,
- l'allèle récessif (g), se distingue de l'allèle dominant en ce que ledit allèle récessif (g) est non fonctionnel dans ladite plante dicotylédone,

étant entendu qu'au moins le second élément génétique de contrôle a été artificiellement introduit dans ladite plante dicotylédone.

Dans certains modes de réalisation selon l'invention, pour le second élément génétique de contrôle (G/g), les caractéristiques respectives de l'allèle dominant (G) et de l'allèle récessif (g) sont les suivantes :

- l'allèle dominant (G), consiste en un acide nucléique (NG) comprenant :

  (i) un polynucléotide régulateur (PG) fonctionnel dans une plante dicotylédone, et
  (ii) un acide nucléique dont l'expression est régulée par le polynucléotide régulateur (PG), ledit acide nucléique codant pour la protéine CmWIP1 de séquence SEQ ID N°12,

- l'allèle récessif (g), se distingue de l'allèle dominant (G) par un acide nucléique non fonctionnel et comprend:

  (i) un polynucléotide régulateur (Pg) dont la séquence nucléotidique comprend au moins une mutation, une insertion ou une délétion par rapport à l'acide nucléique de séquence SEQ ID N°13, ledit acide nucléique conduisant à une expression réduite ou nulle de la protéine CmWIPI de séquence SEQ ID N°12, ou
  (ii) un acide nucléique (Ng) qui code pour une protéine qui diffère de la protéine CmWIP1 de séquence SEQ ID N°12 par la substitution, l'insertion ou la délétion d'un ou plusieurs acides aminés et qui ne possède pas l'activité biologique de ladite protéine CmWIPI dans ladite plante dicotylédone.

La demande décrit une protéine ACS englobant la protéine de séquence SEQ ID N° 3 ou une protéine ayant au moins 90% d'identité en acides aminés, préférentiellement au moins 91%, 92%, 93%, 94%, 95%, 96%, 97%, 98% ou 99% d'identité en acide aminés, avec la protéine de séquence SEQ ID N° 3.

La demande décrit une protéine CmWIP1 englobant la protéine de séquence SEQ ID N° 12 ou une protéine ayant au moins 90% d'identité en acides aminés, préférentiellement au moins 91%, 92%, 93%, 94%, 95%, 96%, 97%, 98% ou 99% d'identité en acide aminés, avec la protéine de séquence SEQ ID N° 12. La protéine CmWIP1 peut aussi englober la protéine de séquence SEQ ID N° 16 ou une protéine ayant au moins 90% d'identité en acides aminés, préférentiellement au moins 91%, 92%, 93%, 94%, 95%, 96%, 97%, 98% ou 99% d'identité en acide aminés, avec la protéine de séquence SEQ ID N° 16.

Dans certains modes de réalisation selon l'invention, pour le second élément génétique de contrôle (A/a), les caractéristiques respectives de l'allèle dominant (A) et de l'allèle récessif (a) sont les suivantes :

- l'allèle dominant (A), consiste en un acide nucléique (NA) comprenant :

  (i) un polynucléotide régulateur (PA) fonctionne! dans une plante dicotylédone, et
  (ii) un acide nucléique dont l'expression est régulée par le polynucléotide régulateur (PA), ledit acide nucléique codant pour la protéine ACS (aminocyclopropane carboxylate synthase) de séquence SEQ ID N°3,

- l'allèle récessif (a), se distingue de l'allèle dominant (A) par un acide nucléique non fonctionnel et comprend:

  (i) un polynucléotide régulateur (Pa) dont la séquence nucléotidique comprend une insertion, une substitution ou une délétion par rapport à l'acide nucléique allant du nucléotide 1 au nucléotide 5907 de la séquence SEQ ID N°1, et conduisant à une expression réduite ou nulle de la protéine ACS de séquence SEQ ID N°3, ou
  (iii) un acide nucléique (Na) codant pour une protéine qui diffère de la protéine ACS de séquence SEQ ID N°3 par la substitution, l'insertion ou la délétion d'un ou plusieurs acides aminés et qui ne possède pas l'activité biologique de ladite protéine ACS dans ladite plante dicotylédone.

La demande décrit également les polynucléotides régulateurs (PG) et (Pg) en tant que tels

L'invention est également relative à des procédés d'obtention d'une plante transformée dont le phénotype sexuel a été modifié, ainsi que les parties d'une telle plante, notamment ses semences.

L'invention a encore pour objet la protéine CmWIP1 telle que définie plus en détail ci-après, ou un fragment de cette protéine, ainsi que des anticorps dirigés contre la protéine CmWIP1.

L'invention a également trait à des procédés pour détecter la présence des allèles (A), (a), (G), et (g) dans un échantillon.

## DESCRIPTION DES FIGURES

La **figure 1** illustre le clonage positionnel du locus g.

La figure **1A** illustre les cartes physiques et génétiques du locus g sur le chromosome 4. Le locus est bordé par deux marqueurs M261 et M335. Les lignes discontinues indiquent la position de ces marqueurs génétiques dans les différents clones BAC.

La figure **1B** illustre la représentation des 8 cadres ouverts de lecture ou ORFs (flèche large) retrouvé dans le BAC 102, avec leur prédiction d'orientation. Le repère triangulaire représente l'insertion du transposon d'ADN nommé Gyno-hAT dont la sequence est décrite dans la SEQ ID N° 14. C'est l'insertion de ce transposon qui a induit la méthylation du promoteur du gène CmWIP1 et a conduit à son inactivation.

La figure **1C** est une cartographie à haute résolution des deux évènements critiques de recombinaison à proximité du locus g. Les polymorphismes SNP sont indiqués et les recombinants P63.2 et 87.94 ont déterminé une région finale de 1,4kb.

La **figure 2** illustre les résultats de l'amplification par PCR semi-quantitative sensible à l'endonucléase McrBr sur l'ADN du transposon inséré au locus g.

L'amorce sens est localisée dans la séquence du transposon, et l'amorce antisens est localisée sur la séquence génomique bordant le transposon. L'amplification par PCR sans digestion par l'endonucléase de l'ADN génomique Gynadou a résulté dans une forte amplification, signifiant la présence du transposon à ce locus. L'amplification par PCR à prédigestion par l'endonucléase McrBC ne montre aucune amplification, ce qui indique un très fort taux de méthylation du transposon. Aucune amplification n'a été obtenue pour PI124112 quelque soit le support, du fait que le transposon n'est pas inséré dans le locus G.

La **figure 3** illustre l'analyse de méthylation au locus g.

La figure **3A** illustre l'amplification PCR semi-quantitative sensible à McrBR sur les trois cadres ouverts de lecture ou ORF les plus proches du locus g pour le génotype monoïque (G-) PI124112 et le génotype gynoïque Gynadou (gg). L'absence d'amplification à prédigestion avec McrBC indique la présence de méthylation de l'ADN. Les oligonucléotides ont été dessinés afin de générer un amplicon qui borde le site d'initiation de la transcription prédit pour chaque ORF, incluant une partie du promoteur et une partie du premier exon.

La figure **3B** illustre la méthylation de l'ADN et la structure du gène CmWIP1. Les flèches noires représentent le site d'initiation à la transcription déterminé par la technique « 5' RACE », les boîtes noires représentent les deux exons, le symbole après le second exon représente la fin de la séquence « 3'UTR », déterminée par la technique de « 3'RACE ».

L'insertion du transposon est symbolisée sur la région sur extrémité 3' du gène. La méthylation de l'ADN dans la séquence complète de CmWIP1 a été déterminée par amplification PCR quantitative sensible à McrBC. Chaque valeur correspond à la moyenne d'au moins trois plantes, chaque réaction PCR ayant été réalisée en triple.

La figure **3C** illustre l'analyse de méthylation des cytosines par séquençage au bisulfite. Deux amplicons correspondant à la partie hautement méthylée du promoteur ont été amplifiés après traitement au bisulfite. Le pourcentage de cytosines méthylées est indiqué par des barres verticales.

La **figure 4** illustre l'analyse de méthylation au locus g pour différents fonds génétiques de *C.mélo.* W1998, Bulgarie 14, Paul et Gynadou sont homozygotes pour l'allèle g. PI161375, Vedrantais et PI124112 sont homozygotes pour l'allèle G.

La figure **4A** illustre l'expérience par laquelle l'insertion du transposon au locus g a été criblée par amplification par PCR dans les différents fons génétiques. L'amorce sens était localisée dans la séquence du transposon et l'amorce antisens s'était localisée dans la séquence génomique bordant le transposon, afin de vérifier la présence de l'insertion (ligne supérieure), où les deux amorces bordant le site d'insertion ont été utilisées afin de vérifier l'absence du transposon (ligne inférieure).

Sur la figure 4A sont représentés les résultats d'amplification PCR semi-quantitative sensible à McrBr sur le gène CmWIP1 dans les différents fonds génétiques. L'absence d'maplification après digestion avec McrBc indique la présence de la méthylation de l'ADN.

La **figure 5** illustre l'analyse du profil d'expression de l'ARN messager de CmWIP1.

Les niveaux d'expression de CmWIP1 ont été analysés par PCR quantitative. Chaque valeur correspond à la moyenne d'au moins trois plantes. Les niveaux d'expression de CmWIP1 ont été normalisés avec les niveaux d'expression d'un gène ubiquitaire, le gène de l'actine.

La **figure 6** illustre les résultats de l'analyse des niveaux d'expression de CmWIP1 analysés par PCR quantitative dans un pool de bourgeons floraux jusqu'au stade 6. Chaque valeur correspond à la moyenne d'au moins trois plantes. Les niveaux d'expression de CmWIP1 ont été normalisés avec les niveaux d'expression d'un gène ubiquitaire, le gène de l'actine.

La **figure 7** illustre l'observation des phénotypes floraux identifiés par la technique de TILLING. Les fleurs de la tige principale du mutant S306F comme celle du mutant P193L ont été comparées à une fleur mâle et une fleur femelle de type sauvage du parent monoique). Les fleurs des mutants S306F comme celle du mutant P193L montrent clairement un développement de l'ovaire dans la quatrième spirale Le mutant L77F est un mutant faible. Ov : ovaire ; St : étamine.

La **Figure 8A** représente un alignement d'un fragment de l'ARN messager du gène *ACS* provenant d'une plante ayant le phénotype (A) [ligne supérieure] et d'une plante ayant le phénotype (a) [ligne inférieure], et illustre une mutation ponctuelle d'un nucléotide différenciant (A) et (a).

La **Figure 8B** représente un alignement des séquences d'acides aminés codées par les acides nucléiques de la Figure 8A, y compris pour une plante ayant le phénotype (A) [ligne supérieure] et d'une plante ayant le phénotype (a) [seconde ligne] et illustre une mutation ponctuelle d'un acide aminé différenciant (A) et (a).

La **Figure 9** représente des clichés de plantes transgéniques de *Arabidopsis thaliana* portant l'allèle de melon A ou a. Sur les figures 9A et 9B, on montre que les siliques des plantes transformées avec l'allèle A (Fig 9A-Cm-A et Fig. 9B)sont plus courtes que les siliques des plantes sauvages (Col-0) et des plantes transformées avec l'allèle (a).

## DESCRIPTION DETAILLEE DE L'INVENTION

Selon la présente invention, on a identifié et caractérisé le déterminant génétique (G/g) qui contrôle, en combinaison avec le déterminant génétique (A/a) précédemment décrit dans l'état de la technique, le développement du type floral chez les cucurbitacées.

L'identification et la caractérisation du déterminant génétique (G/g) fournit pour la première fois à l'homme du métier la possibilité de sélectionner ou de générer des plantes dicotylédones ayant le phénotype sexuel désiré, en particulier des cucurbitacées comme le melon ayant le phénotype sexuel désiré.

On rappelle que l'allèle (A) contrôle le caractère andromonoïque des plantes, et l'allèle (G) contrôle le caractère gynoïque des plantes, comme cela est illustré dans le tableau 1 ci-dessous.

**Tableau 1**

| Phénotype | génotype | Type de fleurs |
|---|---|---|
| Monoïque | A-G- | Mâles et femelles |
| Andromonoïque | aaG- | Mâles et hermaphrodites |
| Hermaphrodite | aagg | Hermaphrodites |
| Gynoïque | A-gg | Femelles |

Le tableau 1 illustre la correspondance entre le génotype et le phénotype sexuel de fleurs de plantes dicotylédones.

Les inventeurs ont maintenant montré que, physiologiquement, les deux allèles (G) et (g), qui ont été identifiés et caractérisés selon l'invention, se distinguent par des taux différents d'une nouvelle protéine, la protéine CmWIP1 .

En réalisant des comparaisons de séquences avec des protéines connues, les inventeurs ont montré que la nouvelle protéine CmWIP1 pouvait être classée dans la famille des protéines à doigts de zinc de type WIP qui sont retrouvées dans une grande variété de plantes, y compris les dicotylédones, les monocotylédones, les gymnospermes et les mousses.

Du point de vue génétique, les inventeurs ont montré que l'allèle (g) se distingue de l'allèle (G) par un taux de protéine CmWIP1 faible dans la plante, comparé à celui d'une plante portant un allèle (G) ou bien par la production d'une protéine CmWIP1 mutée par rapport à la protéine CmWIP1 produite par une plante portant un allèle (G).

Les inventeurs ont aussi montré que l'allèle (G) est dominant sur l'allèle (g).

Comme cela a déjà été précisé précédemment, il avait été déjà montré dans l'état de la technique que, physiologiquement, les deux allèles (A) et (a) se distinguent par des taux différents de l'activité enzymatique d'une protéine, la aminocyclopropane carboxylate synthase aussi désignée ACS, qui est une protéine impliquée dans la synthèse de l'éthylène.

Or, différentes études ont montré que les gènes de la biologie florale des cucurbitacées codent pour des protéines impliquées dans la voie de biosynthèse ou de régulation de l'éthylène (Kamachi *et al*., 1997 ; Kahana *et al.*, 2000).

Il avait été aussi montré antérieurement que l'allèle (a) se distingue physiologiquement de l'allèle (A) par un taux d'activité enzymatique de protéine ACS faible dans la plante, comparé à celui d'une plante portant un allèle (A).

Il avait été aussi montré que l'allèle (A) s'exprime dans les promordia des carpels et c'est cette expression qui bloque le développement des étamines. L'absence d'expression de l'allele A ou l'expression d'une forme mutée de la proteine issue des criblages TILLING par exemple ou de la protéine issue de l'allele a portant la mutation A57V ne bloque pas le développement des étamines. Enfin, il avait été également montré que l'allèle (A) est dominant sur l'allèle (a).

Sans vouloir être liés par une quelconque théorie, les inventeurs pensent que le système de contrôle du développement du type floral par une combinaison d'allèles du gène (G/g) et (A/a) peut être généralisé à la famille des dicotylédones, y compris à la famille des *cucurbitaceae.*

La demande décrit donc une combinaison de deux éléments génétiques et son utilisation pour le contrôle du développement du type floral d'une plante dicotylédone, ladite combinaison comprenant respectivement :

a) un premier élément génétique de contrôle (A/a) présent dans ladite plante dicotylédone, sous la forme d'un allèle dominant (A), et d'un allèle récessif (a), dans lequel :

- l'allèle dominant (A), consiste en un acide nucléique (NA) permettant l'expression de la protéine ACS (aminocyclopropane carboxylate synthase), préférentiellement la protéine ACS de séquence SEQ ID N°3,
- l'allèle récessif (a), se distingue de l'allèle dominant par un acide nucléique (NA) non fonctionnel dans ladite plante dicotylédone, et

b) un second élément génétique de contrôle (G/g) présent dans ladite plante dicotylédone, sous la forme d'un allèle dominant (G), et d'un allèle récessif (g), dans lequel :

- l'allèle dominant (G), consiste en un acide nucléique (NG) permettant l'expression de la protéine CmVVIP1, préférentiellement la protéine CmWIP1 de séquence SEQ ID N°12,
- l'allèle récessif (g), se distingue de l'allèle dominant par un acide nucléique (NG) non fonctionnel dans ladite plante dicotylédone,

étant entendu qu'au moins le second élément génétique de contrôle a été artificiellement introduit dans ladite plante dicotylédone.

L'acide nucléique (NA) non fonctionnel qui caractérise l'allèle (a) englobe (i) un acide nucléique codant pour une protéine distincte de la protéine de SEQ ID N° 3, y compris la protéine mutée A57V, (ii) ou toute autre forme de protéine mutée par rapport à la protéine de séquence SEQ ID N° 3 et conduisant à une enzyme inactive, (ii) ou encore d'autres ACS non fonctionnelles (iv) ou encore un allèle non exprimé.

Il est notamment décrit selon l'invention l'utilisation d'une combinaison de deux éléments génétiques selon la revendication 1, caractérisée en ce que :

- l'allèle dominant (A), consistant en un acide nucléique (NA), comprend :

  (i) un polynucléotide régulateur (PA) fonctionnel dans une plante dicotylédone, et
  (ii) un acide nucléique dont l'expression est régulée par le polynucléotide régulateur (PA), ledit acide nucléique codant pour la protéine ACS (aminocyclopropane carboxylate synthase) de séquence SEQ ID N°3,

- l'allèle récessif (a) non fonctionnel, se distingue de l'allèle dominant par un acide nucléique non fonctionnel et comprend :

  (i) un polynucléotide régulateur (Pa) dont la séquence nucléotidique comprend une insertion, une substitution ou une délétion par rapport à l'acide nucléique allant du nucléotide 1 au nucléotide 5907 de la séquence SEQ ID N°1, et conduisant à une expression réduite ou nulle de la protéine ACS de séquence SEQ ID N°3
(ii) un acide nucléique (Na) codant pour une protéine qui diffère de la protéine ACS de séquence SEQ ID N°3 par la substitution, l'insertion ou la délétion d'un ou plusieurs acides aminés et qui ne possède pas l'activité biologique de ladite protéine ACS dans ladite plante dicotylédone, et

- l'allèle dominant (G), consistant en un acide nucléique (NG), comprend :

(i) un polynucléotide régulateur (PG) fonctionnel dans une plante dicotylédone, et
(ii) un acide nucléique dont l'expression est régulée par le polynucléotide régulateur (PG), ledit acide nucléique codant pour l'expression de la protéine CmWIPI *(« C. melo Zinc Finger Protein »*) de séquence SEQ ID N°12,

- l'allèle récessif non fonctionnel (g) se distingue de l'allèle dominant (G) par un acide nucléique non fonctionnel et comprend :

(i) un polynucléotide régulateur (Pg) dont la séquence nucléotidique comprend au moins une mutation, une insertion ou une délétion par rapport à l'acide nucléique de séquence SEQ ID N°13, ledit acide nucléique conduisant à une expression réduite ou nulle de la protéine CmWIPI de séquence SEQ ID N°12, ou
(ii) un acide nucléique (Ng) qui code pour une protéine qui diffère de la protéine CmWIPI de séquence SEQ ID N°12 par la substitution, l'insertion ou la délétion d'un ou plusieurs acides aminés et qui ne possède pas l'activité biologique de ladite protéine CmWIPI dans ladite plante dicotylédone.

La combinaison des deux éléments génétiques qui est décrite ci-dessus permet de contrôler, et/ou de modifier le sexe des fleurs de plantes dicotylédones, et est donc très avantageuse, par rapport aux systèmes de contrôle mécaniques, souvent coûteux, ou chimiques, souvent toxiques, utilisés dans l'art antérieur.
Par « allèle », on entend au sens de la présente invention, l'une des formes d'un gène occupant un site ou locus sur une paire de chromosomes homologues. Les allèles d'un gène se rapportent au même trait génétique mais peuvent déterminer des phénotypes différents.
Un allèle dominant est un allèle dont le niveau d'expression phénotypique est beaucoup plus important que celui de l'allèle homologue (dit récessif). La dominance peut être complète ou partielle.
Un allèle récessif est un allèle ne s'exprimant dans le phénotype que lorsque la plante reçoit les allèles identiques de chacun de ses deux parents. En revanche, l'expression de l'allèle récessif est masquée si l'allèle homologue dominant est présent.
Ainsi, la combinaison définie ci-dessus existe sous la forme de différents états correspondant chacun à un phénotype.
Lorsque le premier élément génétique de contrôle (A/a) présent dans une plante dicotylédone est sous forme d'allèle (A), la plante est de phénotype monoïque ou gynoïque.
Lorsque le premier élément génétique de contrôle (A/a) présent dans une plante est sous forme d'allèle (aa), la plante est de phénotype hermaphrodite ou andromonoïque.
Lorsque le second élément génétique de contrôle (G/g) présent dans une plante dicotylédone est sous forme d'allèle (G), la plante est de phénotype monoïque ou andromonoïque.
Lorsque le second élément génétique de contrôle (G/g) présent dans une plante est sous forme d'allèle (gg), la plante est de phénotype hermaphrodite ou gynoïque.
La correspondance entre allèles et phénotypes est résumée dans le tableau 1.
Dans certains modes de réalisation de la combinaison selon l'invention, pour le second élément génétique de contrôle (G/g), les caractéristiques respectives de l'allèle dominant (G) et de l'allèle récessif (g) sont les suivantes :

- l'allèle dominant (G), consiste en un acide nucléique (NG) comprenant :

(i) un polynucléotide régulateur (PG) fonctionnel dans une plante dicotylédone, et
(ii) un acide nucléique dont l'expression est régulée par le polynucléotide régulateur (PG), ledit acide nucléique codant pour la protéine CmWIP1 (« *C. melo* Zinc Finger Protein ») de séquence SEQ ID N°12,

- l'allèle récessif (g), se distingue de l'allèle dominant (G) par :

(i) un acide nucléique (NG) non présent dans la plante, ou
(ii) un polynucléotide régulateur (Pg) non fonctionnel dans une plante dicotylédone, ou
(iii) un acide nucléique (Ng) non fonctionnel pour l'expresssion d'une protéine CmWIP1 active.

Dans certains modes de réalisation de la combinaison selon l'invention, pour le premier élément génétique de contrôle (A/a), les caractéristiques respectives de l'allèle dominant (A) et de l'allèle récessif (a) sont les suivantes :

- l'allèle dominant (A), consiste en un acide nucléique (NA) comprenant :

  (i) un polynucléotide régulateur (PA) fonctionnel dans une plante dicotylédone, et
  (ii) un acide nucléique dont l'expression est régulée par le polynucléotide régulateur (PA), ledit acide nucléique codant pour la protéine ACS (aminocyclopropane carboxylate synthase) de séquence SEQ ID N°3,

- l'allèle récessif (a), se distingue de l'allèle dominant (A) par :

  (i) un acide nucléique (NA) non présent dans la plante, ou
  (ii) un polynucléotide régulateur (Pa) non fonctionnel dans une plante dicotylédone, ou
  (iii) un acide nucléique (Na) non fonctionnel pour l'expression d'une protéine ACS active.

La suite de la description expose des variantes, ou modes de réalisation préférés des premier et second éléments génétiques de contrôle faisant partie du système de contrôle objet de l'invention.

**Elément génétique de contrôle G/g, sous forme d'allèle dominant (G), dans la combinaison de deux éléments génétiques selon l'invention.**

De manière générale, l'élément génétique de contrôle G/g, présent dans une plante sous la forme de l'allèle dominant (G), permet d'obtenir un taux plus élevé de protéine CmWIP1, par rapport au taux observé lorsque l'allèle (G) n'est pas présent dans ladite plante.
Dans la suite de la description, on considère qu'un « taux élevé » de protéine CmWIP1 correspond au taux moyen de la protéine CmWIP1 mesuré dans une plante comprenant l'allèle dominant (G) dans son génome, et qu'un « taux faible » de protéine CmWIP1 correspond au taux moyen de protéine CmWIP1 observé dans une plante ne comprenant pas l'allèle dominant (G) dans son génome.
L'allèle dominant (G), consiste en un acide nucléique (NG) comprenant :

(i) un polynucléotide régulateur (PG) fonctionnel dans une plante dicotylédone, et
(ii) un acide nucléique dont l'expression est régulée par le polynucléotide régulateur (PG), ledit acide nucléique codant pour la protéine CmWIP1 de séquence SEQ ID N°12.

- ***polynucléotide régulateur (PG) fonctionnel***

Un polynucléotide régulateur ou promoteur (PG) fonctionnel selon l'invention consiste en un acide nucléique qui permet l'expression de la protéine CmWIP1 de séquence SEQ ID N°12 chez les plantes dicotylédones.
A titre d'exemple un tel promoteur comprend, ou consiste en, l'acide nucléique de séquence SEQ ID N° 13, qui est localisé du nucléotide en position 1 jusqu'au nucléotide en position 2999 de l'acide nucléique du gène CmWIP1 de séquence SEQ ID N° 10.
Ainsi, dans les modes de réalisation de la combinaison des deux éléments génétiques de l'invention dans lesquels le second élément génétique consiste en l'allèle G, le polynucléotide régulateur (PG) peut consister en un polynucléotide qui comprend, ou qui consiste en, (i) une séquence nucléotidique allant du nucléotide 1 au nucléotide 2999 de la séquence SEQ ID N°10 ou (ii) une séquence ayant au moins 90% d'identité en nucléotides avec la séquence 1-2999 de SEQ ID N° 10 et qui est fonctionnelle ou (iii) un fragment des séquences (i) et (ii) précédentes et qui est fonctionnelle.
L'invention a également pour objet le polynucléotide régulateur (PG) en tant que tel, défini ci-dessus, ainsi que des fragments de cet acide nucléique, comme cela sera décrit plus en détails dans la partie intitulée « Acides nucléiques selon l'invention »
Un polynucléotide régulateur (PG) fonctionnel selon l'invention peut également consister en un promoteur connu pour diriger l'expression de la séquence d'acide nucléique codant pour la protéine CmWIP1 de façon constitutive ou de façon tissu spécifique.
Un polynucléotide régulateur (PG) fonctionnel selon l'invention peut ainsi être choisi parmi des promoteurs tissu spécifiques tels que ceux des gènes de la famille des « MADS box » de la classe A B C D et E, tels que décrits par Theiβen et al., 2001 ou tout autre promoteur de gènes homéotiques.
Un polynucléotide régulateur (PG) fonctionnel selon l'invention peut ainsi être choisi parmi :

- le promoteur 35S du virus de la mosaïque du chou-fleur, ou le promoteur 19S ou avantageusement le promoteur constitutif double 35S (pd35S), décrits dans l'article de Kay et al., 1987 ;
- le promoteur actine du ri3 suivi de l'intron actine de ri3 (pAR-IAR) contenu dans le plasmide pAct1-F4 décrit par Mc Elroy et al., 1991 ;
- le promoteur constitutif EF-1$\alpha$ du gène codant pour le facteur d'élongation végétale décrit dans la demande PCT n°WO 90/02172 ou encore dans l'article de AXELOS et al. (1989) ;
- le superpromoteur chimérique PSP (NI et al., 1995) constitué de la fusion de trois copies de l'élément d'activité transcriptionnelle du promoteur du gène de la octopin synthase de *Agrobacterium tumefaciens* et de l'élément d'activation de la transcription du promoteur du gène de la mannopin synthase de *Agrobacterium tumefaciens* ; et
- le promoteur ubiquitine du tournesol (BINET et al., 1991) ;
- le promoteur de l'ubiquitine 1 de maïs (CHRISTENSEN et al., 1996).

le promoteur de l'ubiquitine 1 de maïs (Christensen et al., 1996)

Un polynucléotide régulateur (PG) fonctionnel selon l'invention peut également consister en un promoteur inductible.

Ainsi, l'invention a pour objet une combinaison de deux éléments génétiques pour le contrôle du type floral d'une plante dicotylédone, tel que défini précédemment, dans lequel le polynucléotide régulateur (PG) est sensible à l'action d'un signal inducteur, et de préférence, dans lequel le polynucléotide régulateur (PG) est un polynucléotide activateur inductible de la transcription ou de la traduction.

Lorsque le polynucléotide régulateur activateur de la transcription ou de la traduction est sensible, directement ou indirectement, à l'action d'un signal inducteur activateur, il s'agit d'un polynucléotide « activateur inductible » au sens de l'invention.

Selon l'invention, un polynucléotide régulateur du type « activateur inductible » est une séquence régulatrice qui n'est activée qu'en présence d'un signal externe. Un tel signal externe peut être la fixation d'un facteur de transcription, la fixation d'un facteur de transcription pouvant être induite sous l'effet du signal inducteur activateur auquel le polynucléotide régulateur est directement ou indirectement sensible.

Lorsqu'une telle construction d'acides nucléiques est utilisée dans un hôte cellulaire, l'expression du polynucléotide codant pour la protéine CmWIP1 selon l'invention peut être induite en mettant en contact l'hôte cellulaire transformé avec le signal inducteur activateur auquel le polynucléotide régulateur activateur est, directement ou indirectement, sensible.

Lorsque l'on recherche l'absence d'expression du polynucléotide codant pour un polypeptide CmWIP1 chez cet hôte cellulaire transformé, il suffit alors d'éliminer ou supprimer la présence du signal inducteur activateur auquel le polynucléotide régulateur de la transcription ou de la traduction est sensible.

L'homme du métier aura recours à ses connaissances générales techniques dans le domaine des polynucléotides régulateurs, en particulier ceux actifs chez les végétaux, pour définir les constructions répondant à la définition du mode de réalisation ci-dessus.

La séquence régulatrice capable de contrôler l'acide nucléique codant pour une protéine CmWIP1 peut être une séquence régulatrice inductible par un métabolite particulier, tel que :

- une séquence régulatrice inductible par les glucocorticoïdes telle que décrite par AOYAMA et al. (1997) ou telle que décrit par McNELLYS et al. (1998);
- une séquence régulatrice inductible par l'éthanol, telle que celle décrite par SALTER et al. (1998) ou encore telle que décrite par CADDICK et al. (1998);
- une séquence régulatrice inductible par la tétracycline telle que celle commercialisée par la Société CLONTECH.
- une séquence de promoteur inductible par un pathogène ou par un métabolite produit par un pathogène.
- une séquence régulatrice de gènes de type PR, inductible par l'acide salicylique ou le BTH ou l'aliette (Gorlach et al., 1996, Molina et al., 1998) ;
- une séquence régulatrice de type récepteur Ecdysone (Martinez et al., 1999) inductible par le tebufeno3ide (référence produit RH5992, commercialisé par ROHM & HAAS) par exemple, appartenant à la famille des diben3oylhydra3ines.

***-Acides nucléiques codant pour la protéine CmWIP1***

De préférence, l'acide nucléique codant pour la protéine CmWIP1 comprend, de l'extrémité 5' vers l'extrémité 3', au moins :

(i) une séquence ayant au moins 95% d'identité avec le polynucléotide allant du nucléotide 3000 au nucléotide 3617 de la séquence SEQ ID N°10, et

(ii) une séquence ayant au moins 95% d'identité avec le polynucléotide allant du nucléotide 5458 au nucléotide 5901 de la séquence SEQ ID N°10.

Dans d'autres modes de réalisation, l'acide nucléique codant la protéine CmWIP1 comprend la séquence nucléotidique SEQ ID N° 11.

**Elément génétique de contrôle G/g, sous forme d'allèle récessif (g) du système selon l'invention**

De manière générale, l'élément génétique de contrôle (G/g) sous la forme de l'allèle récessif (g), lorsqu'il est présent dans une plante qui ne possède pas l'allèle dominant (G) dans son génome, ne permet pas d'obtenir un taux de protéine CmWIP1, aussi élevé que celui obtenu lorsque l'allèle (G) est présent.
On peut donc définir l'allèle (g) comme toute altération du génotype correspondant à l'allèle (G), ne permettant pas d'obtenir un taux de CmWIP1 aussi élevé que l'allèle (G).
L'allèle récessif (g), se distingue de l'allèle dominant (G) par :

(i) un acide nucléique (NG) non présent dans la plante, ou
(ii) un polynucléotide régulateur (Pg) non fonctionnel dans une plante dicotylédone, ou
(iii) un acide nucléique (Ng) non fonctionnel pour l'expression d'une protéine CmWIP1 non active.

A titre d'exemple, un mode de réalisation d'un élément de contrôle G/g sous la forme d'allèle récessif (g) est illustré par l'acide nucléique de séquence SEQ ID N° 15, qui est un allèle de la séquence codant CmWIP1 dans lequel est présent un acide nucléique transposon désigné « Gyno-hAT ». Dans l'acide nucléique de séquence SEQ ID N° 15, le transposon Gyno-hAT est localisé du nucléotide en position 7167 jusqu'au nucléotide en position 15412 de la séquence SEQ ID N° 15.
Dans l'acide nucléique de séquence SEQ ID N° 14, le transposon Gyno-hAT est localisé du nucléotide en position 10 jusqu'au nucléotide en position 8246 de la séquence SEQ ID N° 14.

***- Polynucléotide régulateur (Pg) non fonctionnel***

Un polynucléotide régulateur (Pg), ou promoteur non fonctionnel selon l'invention est un acide nucléique qui :

(i) ne permet pas l'expression de la protéine CmWIP1 de séquence SEQ ID N°12 dans une cellule hôte, ou
(ii) permet l'expression de cette protéine à un taux faible en comparaison du taux observé avec le polynucléotide régulateur (PG), ou
(iii) permet l'expression de la protéine CmWIP1 au cours de la vie de la plante, pendant une durée inférieure, en comparaison de celle observée avec le polynucléotide régulateur (PG).

Dans certains modes de réalisation du polynucléotide régulateur (Pg) non fonctionnel, ledit polynucléotide (Pg) est méthylé. Par exemple, le polynucléotide régulateur (Pg) peut consister en un polynucléotide régulateur (PG) qui se présente sous la forme d'un acide nucléique méthylé.
Par « acide nucléique méthylé » ou « polynucléotide régulateur méthylé », on entend selon l'invention l'acide nucléique correspondant dont le rapport (nombre de bases méthylées)/(nombre de bases non méthylées) est d'au moins 5/1. Ainsi, selon l'invention, un acide nucléique méthylé englobe un acide nucléique possédant un rapport (nombre de bases méthylées)/(nombre de bases non méthylées) d'au moins, 6/1, 7/1, 8/1, 9/1, 10/1, 11/1, 12/1, 13/1, 14/1, 15/1, 16/1, 17/1, 18/1 et 20/1.
La détermination du rapport (nombre de bases méthylées)/(nombre de bases non méthylées) peut être réalisée aisément par l'homme du métier par toute technique connue. L'homme du métier peut notamment utiliser la méthode de séquençage au bisulfite, comme décrit dans les exemples de la présente description.
Pour comparer le niveau d'expression de plusieurs promoteurs, une technique simple, connue de l'homme du métier consiste à placer un gène marqueur de sélection sous le contrôle des promoteurs à tester. Un gène marqueur de sélection peut être par exemple le gène de résistance à l'herbicide BASTA, bien connu de l'homme du métier.
Une autre technique peut consister à mesurer le taux de la protéine CmWIP1 obtenue lorsque la séquence codant pour cette protéine est sous le contrôle de différents promoteurs, en utilisant des anticorps dirigés à l'encontre cette protéine, et les procédés décrits dans la partie « polypeptides selon l'invention ».
Comme cela est montré dans les exemples, une illustration d'un promoteur (Pg) non fonctionnel consiste en un promoteur ayant une séquence nucléotidique identique à la séquence nucléotidique d'un promoteur fonctionnel (PG) mais qui se présente *in cellulo* ou *in vivo* sous une forme méthylée non fonctionnelle. Dans le mode de réalisation spécifique illustré dans les exemples, l'état méthylé du promoteur (Pg) est provoqué par la présence d'une élément transposable (TE) localisé à une distance inférieure à 1 kilobases de l'extrémité 3' du gène *CmWIP1.*

Un polynucléotide (Pg) non fonctionnel peut également être tout polynucléotide dérivé du polynucléotide (PG) tel que défini ci-dessus dont la séquence nucléotidique comprend une insertion, une substitution ou une délétion d'un ou plusieurs nucléotides, par rapport à la séquence nucléotidique du polynucléotide régulateur.

Ainsi, l'invention a également pour objet un acide nucléique comprenant une séquence nucléotidique portant au moins une altération choisie parmi une mutation, une insertion ou une délétion, par rapport à l'acide nucléique allant du nucléotide 1 au nucléotide 2999 de la séquence SEQ ID N°10, ledit acide nucléique altéré conduisant à une expression réduite de la protéine CmWIP1, lorsqu'il contrôle l'expression de ladite protéine, par rapport à l'expression de la protéine CmWIP1 contrôlée par l'acide nucléique allant du nucléotide 1 au nucléotide 2999 de la séquence SEQ ID N°10.

L'invention a également pour objet le polynucléotide régulateur (Pg) en tant que tel, tel que défini ci-dessus.

L'invention a également pour objet un acide nucléique comprenant un polynucléotide régulateur (Pg) et un acide nucléique codant pour la protéine CmWIP1 de séquence SEQ ID N°12.

L'invention a également pour objet une combinaison de deux éléments génétiques pour le contrôle du développement du type floral d'une plante dicotylédone telle que définie de manière générale dans la présente description, dans laquelle le polynucléotide régulateur (Pg) est sensible à l'action d'un signal inducteur, et de préférence, dans lequel le polynucléotide régulateur (Pg) est un polynucléotide répresseur inductible de la transcription ou de la traduction.

Par polynucléotide régulateur « répresseur », on entend selon l'invention une séquence régulatrice dont l'activité constitutive peut être bloquée par un signal externe. Un tel signal externe peut être l'absence de fixation d'un facteur de transcription reconnu par le polynucléotide régulateur répresseur. L'absence de fixation du facteur de transcription peut être induite sous l'effet du signal inducteur répresseur auquel le polynucléotide régulateur répresseur est sensible.

Selon ce premier mode de réalisation particulier, l'expression de la séquence codant pour une protéine CmWIP1 est constitutive dans l'hôte cellulaire choisi, en l'absence du signal inducteur répresseur auquel le polynucléotide régulateur répresseur est directement ou indirectement sensible.

La mise en contact de l'hôte cellulaire avec le signal inducteur répresseur a pour effet, grâce à une action directe ou indirecte sur le polynucléotide régulateur répresseur, d'inhiber et/ou de bloquer l'expression du polynucléotide codant pour la protéine CmWIP1.

Pour réaliser les constructions d'ADN selon l'invention comprenant un polynucléotide régulateur répresseur, l'homme du métier aura recours à ses connaissances générales techniques dans le domaine de l'expression de gènes chez les végétaux.

Un procédé d'obtention d'une plante transformée, mettant en œuvre ce type de polynucléotide régulateur est décrit dans la partie intitulée « procédés d'obtention d'une plante transformée selon l'invention ».

- ***acide nucléique (Ng) non fonctionnel pour l'expression d'une protéine CmWIP1 active***

Un acide nucléique (Ng) englobe les acides nucléiques comprenant au moins une partie d'une séquence codant une protéine CmWIP1 active mais qui ne permettent pas, lorsqu'ils sont placés sous le contrôle d'un polynucléotide régulateur fonctionnel dans des cellules de plantes dicotylédone, la production dans lesdites plantes d'une protéine CmWIP1 active.

Un acide nucléique (Ng) englobe essentiellement les acides nucléiques (NG) dans lesquels sont présentes une ou plusieurs mutations dans au moins un intron ou un exon, chaque mutation étant choisie parmi (i) la substitution d'un nucléotide ou plus d'un nucléotide, (ii) la délétion d'un nucléotide ou d'au moins deux nucléotides consécutifs et (ii) la délétion d'un nucléotide ou d'au moins deux nucléotides consécutifs, par rapport à l'acide nucléique (NG) de référence. Un acide nucléique (Ng) englobe notamment les acides nucléiques codant pour une protéine CmWIP1 non active.

Par acide nucléique codant pour une protéine CmWIP1 non active, on entend au sens de la présente invention, un acide nucléique qui code pour une protéine qui diffère de la protéine CmWIP1 de séquence SEQ ID N°12, par la substitution, la délétion, ou l'insertion d'un ou plusieurs acides aminés, et qui ne possède pas l'activité biologique de la protéine CmWIP1 de séquence SEQ ID N°12.

Est englobé également un acide nucléique qui code pour une protéine qui diffère de la protéine CmWIP1 de séquence SEQ ID N°16, par la substitution, la délétion, ou l'insertion d'un ou plusieurs acides aminés, et qui ne possède pas l'activité biologique de la protéine CmWIP1 de séquence SEQ ID N°16.

En particulier, une telle protéine CmWIP1 non active, lorsqu'elle est exprimée dans une plante qui n'exprime pas de protéine CmWIP1 active, en particulier pas de protéine CmWIP1 de séquence SEQ ID N° 12, ou de protéine CmWIP1 de séquence SEQ ID N° 16, induit respectivement :

- un phénotype de plante hermaphrodite en combinaison avec la présence sous forme homozygote des allèles (a/a) dans ladite plante,
- un phénotype de plante femelle en combinaison avec (i) la présence sous forme homozygote des allèles (A/A) dans ladite plante ou en combinaison avec (ii) la présence sous forme hétérozygote des allèles (A/a) dans ladite plante.

On a montré dans les exemples que des plantes possédant l'allèle (g) de l'élément génétique (G/g) étaient obtenues avec des acides nucléiques codant une protéine CmWIP1 inactive. Notamment, on a montré dans les exemples que des plantes possédant l'allèle (g) de l'élément génétique (G/g) étaient obtenues avec des acides nucléiques codant une protéine CmWIP1 possédant une substitution d'un seul nucléotide par rapport à la séquence nucléotidique SEQ ID N° 11 codant la protéine CmWIP1 de séquence SEQ ID N° 12.

A titre illustratif, on a montré dans les exemples des plantes possédant l'allèle (g) et dont l'acide nucléique correspondant code une protéine CmWIP1 mutée possédant une substitution d'un acide aminé choisie parmi L77F, P193L et S306F, selon la numérotation d'acides aminés utilisée pour la séquence SEQ ID N° 12.

L'élément génétique de contrôle A/a, sous forme d'allèle dominant (A) ou sous forme d'allèle dominant (a) a déjà été décrit dans la demande de brevet français n° FR 2 900 415 et dans la demande PCT n° WO 2007/125264.

Toutefois, du fait que l'élément génétique de contrôle (A/a) est un élément important de la combinaison de deux éléments génétiques pour le contrôle du développement du type floral selon l'invention, ses caractéristiques principales sont à nouveau décrites ci-dessous.

**Elément génétique de contrôle A/a, sous forme d'allèle dominant (A) de la combinaison selon l'invention**

De manière générale, l'élément génétique de contrôle A/a, présent dans une plante sous la forme de l'allèle dominant (A), permet d'obtenir un taux plus élevé de protéine ACS active, par rapport au taux observé lorsque l'allèle (A) n'est pas présent dans ladite plante.

Dans la suite de la description, on considère qu'un « taux élevé » de protéine ACS correspond au taux moyen de la protéine ACS mesuré dans une plante comprenant l'allèle dominant (A) dans son génome, et qu'un « taux faible » de protéine ACS correspond au taux moyen de protéine ACS active observé dans une plante ne comprenant pas l'allèle dominant (A) dans son génome. Un taux faible de la protéine ACS englobe un taux nul de protéine ACS active, par exemple dans le cas d'une expression d'ACS non active, y compris le produit de l'allele a portant la mutation A57V.

L'allèle dominant (A), consiste en un acide nucléique (NA) comprenant :

(i) un polynucléotide régulateur (PA) fonctionnel dans une plante dicotylédone, et
(ii) un acide nucléique dont l'expression est régulée par le polynucléotide régulateur (PA), ledit acide nucléique codant pour la protéine ACS de séquence SEQ ID N°3.

***- polynucléotide régulateur (PA) fonctionnel***

Un polynucléotide régulateur ou promoteur (PA) fonctionnel selon l'invention consiste en un acide nucléique qui permet l'expression de la protéine ACS de séquence SEQ ID N°3 chez les plantes dicotylédones.

A titre d'exemple un tel promoteur comprend une séquence nucléotidique allant du nucléotide 1 au nucléotide 5906 de la séquence SEQ ID N°1.

Ainsi, dans les modes de réalisation de la combinaison des deux éléments génétiques de l'invention dans lesquels le premier élément génétique consiste en l'allèle A, le polynucléotide régulateur (PA) peut consister en un polynucléotide qui comprend, ou qui consiste en, (i) une séquence nucléotidique allant du nucléotide 1 au nucléotide 5906 de la séquence SEQ ID N°1 ou (ii) une séquence ayant au moins 90% d'identité en nucléotides avec la séquence 1-5906 de SEQ ID N° 1 et qui est fonctionnelle ou (iii) un fragment des séquences (i) et (ii) précédentes et qui est fonctionnelle.

Ainsi, dans certains modes de réalisation de la combinaison de deux éléments génétiques de l'invention, le polynucléotide régulateur (PA) comprend ou consiste en une séquence nucléotidique allant du nucléotide 1 au nucléotide 5906 de la séquence SEQ ID N°1.

Un polynucléotide régulateur (PA) fonctionnel qui est inclus dans une combinaison de deux éléments génétiques selon l'invention peut également consister en un promoteur connu pour diriger l'expression de la séquence d'acide nucléique codant pour la protéine ACS de façon constitutive ou de façon tissu spécifique.

Un polynucléotide régulateur (PA) fonctionnel inclus dans une combinaison de deux éléments génétiques selon l'invention peut ainsi être choisi parmi l'un quelconque des promoteurs constitutifs ou spécifiques de tissu décrit précédemment pour certains modes des réalisation du polynucléotide régulateur (PG).

Un polynucléotide régulateur (PA) fonctionnel selon l'invention peut également consister en un promoteur inductible.

Ainsi, l'invention a pour objet une combinaison de deux éléments génétiques telle que définie ci-dessus, dans laquelle le polynucléotide régulateur (PA) est sensible à l'action d'un signal inducteur, et de préférence, dans lequel le polynucléotide régulateur (PA) est un polynucléotide activateur inductible de la transcription ou de la traduction, qui peut être choisi parmi l'un quelconque des polynucléotides activateurs inductibles décrits dans certains modes de réalisation du polynucléotide régulateur (PG).

Lorsqu'une telle construction d'acides nucléiques est utilisée dans un hôte cellulaire, l'expression du polynucléotide codant pour la protéine ACS selon l'invention peut être induite en mettant en contact l'hôte cellulaire transformé avec le signal inducteur activateur auquel le polynucléotide régulateur activateur est, directement ou indirectement, sensible.
Lorsque l'on recherche l'absence d'expression du polynucléotide codant pour un polypeptide ACS chez cet hôte cellulaire transformé, il suffit alors d'éliminer ou supprimer la présence du signal inducteur activateur auquel le polynucléotide régulateur de la transcription ou de la traduction est sensible.
L'homme du métier aura recours à ses connaissances générales techniques dans le domaine des polynucléotides régulateurs, en particulier ceux actifs chez les végétaux, pour définir les constructions répondant à la définition du mode de réalisation ci-dessus, et en particulier ceux décrits pour le polynucléotide régulateur (PG).

***-Acides nucléiques codant pour la protéine ACS***

De préférence, l'acide nucléique codant pour la protéine ACS comprend, de l'extrémité 5' vers l'extrémité 3', au moins :

(i) une séquence ayant au moins 95% d'identité avec le polynucléotide allant du nucléotide 5907 au nucléotide 6086 de la séquence SEQ ID N°1,
(ii) une séquence ayant au moins 95% d'identité avec le polynucléotide allant du nucléotide 6181 au nucléotide 6467 de la séquence SEQ ID N°1, et
(iii) une séquence ayant au moins 95% d'identité avec le polynucléotide allant du nucléotide 7046 au nucléotide 7915 de la séquence SEQ ID N°1.

**Elément génétique de contrôle A/a, sous forme d'allèle récessif (a) de la combinaison selon l'invention**

De manière générale, l'élément génétique de contrôle (A/a) sous la forme de l'allèle récessif (a), lorsqu'il est présent dans une plante qui ne possède pas l'allèle dominant (A) dans son génome, permet d'obtenir un taux de protéine ACS, aussi élevé que celui obtenu lorsque l'allèle (A) est présent.
On peut donc définir l'allèle (a) comme toute altération du génotype correspondant à l'allèle (A), ne permettant pas d'obtenir un taux d'ACS active.
L'allèle récessif (a), se distingue de l'allèle dominant (A) par :

(i) un acide nucléique (NA) non présent dans la plante, ou
(ii) un polynucléotide régulateur (Pa) non fonctionnel dans une plante dicotylédone, ou
(iii) un acide nucléique codant pour une protéine ACS non active, ou
(iV) un polynucléotide régulateur (Pa) non fonctionnel dans une plante dicotylédone, et un acide nucléique codant pour une protéine ACS non active.

***- Polynucléotide régulateur (Pa) non fonctionnel***

Un polynucléotide régulateur (Pa), ou promoteur non fonctionnel selon l'invention est un acide nucléique qui :

(i) ne permet pas l'expression de la protéine ACS de séquence SEQ ID N°3 dans une cellule hôte, ou
(ii) permet l'expression de cette protéine à un taux faible en comparaison du taux observé avec le polynucléotide régulateur (PA), ou
(iii) permet l'expression de la protéine ACS au cours de la vie de la plante, pendant une durée inférieure, en comparaison de celle observée avec le polynucléotide régulateur (PA).

Dans certains modes de réalisation du polynucléotide régulateur (Pa) non fonctionnel, ledit polynucléotide (Pa) est méthylé. Par exemple, le polynucléotide régulateur (Pa) peut consister en un polynucléotide régulateur (PA) qui se présente sous la forme d'un acide nucléique méthylé.
Par « acide nucléique méthylé » ou « polynucléotide régulateur méthylé », on entend selon l'invention l'acide nucléique correspondant dont le rapport (nombre de bases méthylées)/(nombre de bases non méthylées) est d'au moins 5/1. Ainsi, selon l'invention, un acide nucléique méthylé englobe un acide nucléique possédant un rapport (nombre de bases méthylées)/(nombre de bases non méthylées) d'au moins, 6/1, 7/1, 8/1, 9/1, 10/1, 11/1, 12/1, 13/1, 14/1, 15/1, 16/1, 17/1, 18/1 et 20/1.
La détermination du rapport (nombre de bases méthylées)/(nombre de bases non méthylées) peut être réalisée aisément par l'homme du métier par toute technique connue. L'homme du métier peut notamment utiliser la méthode de séquençage au bisulfite, comme décrit dans les exemples de la présente description.

Pour comparer le niveau d'expression de plusieurs promoteurs, une technique simple, connue de l'homme du métier consiste à placer un gène marqueur de sélection sous le contrôle des promoteurs à tester. Un gène marqueur de sélection peut être par exemple le gène de résistance à l'herbicide BASTA, bien connu de l'homme du métier.

Une autre technique peut consister à mesurer le taux de la protéine ACS obtenue lorsque la séquence codant pour cette protéine est sous le contrôle de différents promoteurs, en utilisant des anticorps dirigés à l'encontre cette protéine, et les procédés décrits dans la partie « polypeptides selon l'invention ».

A titre d'exemple, un polynucléotide régulateur (Pa) non fonctionnel comprend une séquence nucléotidique allant du nucléotide 1 au nucléotide 3650 de la séquence SEQ ID N°2.

Egalement à titre d'exemple, un polynucléotide régulateur (Pa) non fonctionnel comprend certaines séquences nucléotidiques comprenant une ou plusieurs substitutions, délétions ou additions de bases, par rapport à la séquence nucléotidique allant du nucléotide 1 au nucléotide 3650 de la séquence SEQ ID N°1.

Ainsi, dans la combinaison de deux éléments génétiques pour le contrôle du développement du type floral objet de l'invention, un polynucléotide régulateur (Pa) non fonctionnel peut comprendre une séquence nucléotidique allant du nucléotide 1 au nucléotide 3650 de la séquence SEQ ID N°2 altéré par l'une des methodes de l homme de l'art

L'invention a également pour objet une combinaison de deux éléments génétiques pour le contrôle du développement du type floral telle que définie dans la présente description, et dans laquelle l'allèle (a) est un acide nucléique comprenant la séquence SEQ ID N°2. Un tel acide nucléique de séquence SEQ ID N° 2 comprend un polynucléotide régulateur (Pa) et un acide nucléique codant pour la protéine ACS de séquence SEQ ID N°3.

Un polynucléotide (Pa) non fonctionnel peut également être tout polynucléotide dérivé du polynucléotide (PA) tel que défini ci-dessus dont la séquence nucléotidique comprend une insertion, une substitution ou une délétion d'un ou plusieurs nucléotides, par rapport à la séquence nucléotidique du polynucléotide régulateur.

Ainsi, dans certains modes de réalisation de la combinaison selon l'invention, un polynucléotide (Pa) consiste en un acide nucléique comprenant une séquence nucléotidique portant au moins une altération choisie parmi une mutation, une insertion ou une délétion, par rapport à l'acide nucléique allant du nucléotide 1 au nucléotide 5907 de la séquence SEQ ID N°1, ledit acide nucléique altéré conduisant à une expression réduite de la protéine ACS, lorsqu'il contrôle l'expression de ladite protéine, par rapport à l'expression de la protéine ACS contrôlée par l'acide nucléique allant du nucléotide 1 au nucléotide 5907 de la séquence SEQ ID N°1.

L'invention a également pour objet une combinaison de deux éléments génétiques pour le contrôle du développement du type floral telle que définie ci-dessus, dans laquelle le polynucléotide régulateur (Pa) est sensible à l'action d'un signal inducteur, et de préférence, dans lequel le polynucléotide régulateur (Pa) est un polynucléotide répresseur inductible de la transcription ou de la traduction.

Selon ce premier mode de réalisation particulier, l'expression de la séquence codant pour une protéine ACS est constitutive dans l'hôte cellulaire choisi, en l'absence du signal inducteur répresseur auquel le polynucléotide régulateur répresseur est directement ou indirectement sensible.

La mise en contact de l'hôte cellulaire avec le signal inducteur répresseur a pour effet, grâce à une action directe ou indirecte sur le polynucléotide régulateur répresseur, d'inhiber et/ou de bloquer l'expression du polynucléotide codant pour la protéine ACS.

Pour réaliser les constructions d'ADN selon l'invention comprenant un polynucléotide régulateur répresseur, l'homme du métier aura recours à ses connaissances générales techniques dans le domaine de l'expression de gènes chez les végétaux.

Un procédé d'obtention d'une plante transformée, mettant en œuvre ce type de polynucléotide régulateur est décrit dans la partie intitulée « procédés d'obtention d'une plante transformée selon l'invention ».

***- acide nucléique (Na) non fonctionnel pour l'expression d'une protéine ACS active***

Un acide nucléique (Na) englobe les acides nucléiques comprenant au moins une partie d'une séquence codant une protéine ACS active mais qui ne permettent pas, lorsqu'ils sont placés sous le contrôle d'un polynucléotide régulateur fonctionnel dans des cellules de plantes dicotylédone, la production dans lesdites plantes d'une protéine CmWIP1 active.

Un acide nucléique (Na) englobe essentiellement les acides nucléiques (NA) dans lesquels sont présentes une ou plusieurs mutations dans au moins un intron ou un exon, chaque mutation étant choisie parmi (i) la substitution d'un nucléotide ou plus d'un nucléotide, (ii) la délétion d'un nucléotide ou d'au moins deux nucléotides consécutifs et (ii) la délétion d'un nucléotide ou d'au moins deux nucléotides consécutifs, par rapport à l'acide nucléique (NA) de référence. Un acide nucléique (Na) englobe notamment les acides nucléiques codant pour une protéine ACS non active.

Par acide nucléique codant pour une protéine ACS non active, on entend au sens de la présente invention, un acide nucléique qui code pour une protéine qui diffère de la protéine ACS de séquence SEQ ID N°3, par la substitution, la délétion, ou l'insertion d'un ou plusieurs acides aminés, et qui ne possède pas l'activité biologique de la protéine ACS de séquence SEQ ID N°3. Un exemple illustratif d'un tel acide nucléique est présenté dans la figure 8.

En particulier, une telle protéine ACS non active ne permet pas de transformer la S-adénosyl méthionine en ACC (1-aminocyclopropane-1-carboxylate).

**Acides nucléiques selon l'invention**

Comme indiqué ci-dessus, il a été caractérisé deux variants alléliques (G) et (g) du second élément génétique de contrôle (G/g) inclus dans une combinaison de deux éléments génétiques pour le contrôle du développement floral de l'invention.

Les inventeurs ont identifié l'acide nucléique de séquence SEQ ID N°10 comme étant un acide nucléique correspondant au variant allélique dominant (G) et sa forme méthylée *in planta*, comme correspondant au variant allélique récessif (g), du second élément génétique de contrôle sous forme d'un gène (G/g).

Dans la combinaison de deux éléments génétiques pour le contrôle du développement floral de l'invention, au moins le second des deux éléments génétiques de contrôle a été introduit artificiellement dans une plante.

Comme cela a été exposé ci-dessus, une telle introduction provoque un changement du sexe de la fleur de la plante, ce qui est un des buts recherchés selon l'invention.

En conséquence l'acide nucléique de séquence SEQ ID N°10 fait partie des objets de l'invention.

La présente invention a donc pour objet un acide nucléique comprenant un polynucléotide possédant au moins 95% d'identité en nucléotides avec la séquence nucléotidique SEQ ID N°10, ou avec un fragment de la séquence SEQ ID N°10, à condition qu'un tel acide nucléique possède les caractéristiques fonctionnelles de l'allèle (G) tel que défini ci-dessus.

Fait également partie de l'invention un acide nucléique de séquence complémentaire à l'acide nucléique tel que défini ci-dessus.

Un autre objet de l'invention est un acide nucléique consistant en un polynucléotide possédant au moins 95% d'identité en nucléotides avec la séquence SEQ ID N°10, ou avec un fragment de la séquence SEQ ID N°10, ou un acide nucléique de séquence complémentaire, à condition qu'un tel acide nucléique possède les caractéristiques fonctionnelles de l'allèle (G) tel que défini ci-dessus.

L'invention est aussi relative à un acide nucléique comprenant au moins 12, de préférence au moins 15 et de manière tout à fait préférée au moins 20 nucléotides consécutifs de l'acide nucléique de séquence SEQ ID N°10, étant entendu qu'un tel acide nucléique englobe dans sa définition les « fragments » d'un acide nucléique selon l'invention tel que défini dans la présente description.

L'invention est également relative à l'acide nucléique comprenant ou consistant en la séquence SEQ ID N°10.

L'invention est aussi relative à un acide nucléique comprenant au moins 12, de préférence au moins 15 et de manière tout à fait préférée au moins 20 nucléotides consécutifs de l'acide nucléique de séquence SEQ ID N°10, étant entendu qu'un tel acide nucléique englobe dans sa définition les « fragments » d'un acide nucléique selon l'invention tel que défini dans la présente description.

L'allèle (G) défini par la séquence SEQ ID N°10 comprend, de l'extrémité 5' vers l'extrémité 3', respectivement :

a) une séquence non codante portant des éléments régulateurs de la transcription et/ou de la traduction de ce gène, localisée en amont du premier exon, du nucléotide en position 1 jusqu'au nucléotide en position 2999 de la séquence SEQ ID N°10;
b) une région dite « codante » qui comprend les deux exons et l'intron du gène (G/g), cette région codante étant localisée du nucléotide en position 3000 jusqu'au nucléotide en position 5901 de la séquence SEQ ID N°10; et
c) une région non codante localisée en aval de la région codante, du nucléotide en position 5902 jusqu'au nucléotide en position 7621 de la séquence SEQ ID N°10.

Les caractéristiques structurales des deux exons et de l'intron du gène G/g sont détaillées dans le tableau 2 ci-après. Les caractéristiques structurales des deux exons et de l'intron des allèles (G) et (g) du gène (G/g) sont très proches, de sorte que les exons des allèles (G) et (g) peuvent, dans certains modes de réalisation, coder pour une même protéine de séquence SEQ ID N°12. Comme cela a été rappelé ci-dessus, la différence majeure entre les séquences nucléotidiques correspondant aux allèles (G) et (g) peut se trouver :

(i) soit dans les séquences régulatrices amont correspondant à ces deux allèles, qui sont non méthylées *in planta* pour l'allèle (G) et qui sont méthylées *in planta* pour l'allèle (g),
(ii) soit dans la séquence des exons, puisque une seule substitution de nucléotide provoquant la substitution d'un acide aminé dans la séquence de la protéine CmWIP1 est suffisante pour l'obtention de l'allèle (g).

**TABLEAU 2**

| Séquences des exons du gène G/g | | |
|---|---|---|
| Exon n° | Position du nucléotide en 5' sur SEQ ID N°10 (allèle G) | Position du nucléotide en 3' sur SEQ ID N°10 (Allèle G) |
| 1 | 3000 | 3617 |
| 2 | 5458 | 5901 |

L'invention est également relative à un acide nucléique comprenant au moins 12 nucléotides consécutifs d'un polynucléotide exonique du gène *G/g*, tel que les polynucléotides 1 et 2 décrits dans le tableau 2 ci-dessus, qui sont inclus dans l'acide nucléique de séquence SEQ ID N°10

Un tel acide nucléique code pour au moins une partie de la protéine CmWIP1 et peut notamment être inséré dans un vecteur recombinant destiné à l'expression du produit de traduction correspondant dans une cellule hôte ou dans une plante transformée avec ce vecteur recombinant, en vue de d'obtenir une plante de génotype (G).

Un tel acide nucléique peut aussi être utilisé pour la synthèse de sondes et d'amorces nucléotidiques destinées à la détection ou à l'amplification de séquences nucléotidiques comprises dans le gène (G/g) dans un échantillon.

Le cas échéant les séquences décrites ci-dessus peuvent porter une ou plusieurs mutations, préférentiellement une ou plusieurs mutations de nature à induire la synthèse d'une protéine CmWIP1 non active, et à modifier le type sexuel d'une plante portant un tel gène (G/g) muté. De telles séquences répondent à la définition d'acides nucléiques codant pour une protéine CmWIP1 non active, définis de manière générale ci-dessus.

**TABLEAU 3**

| Séquence de l'intron du gène (G/g) | | |
|---|---|---|
| Intron n° | Position du nucléotide en 5' sur SEQ ID N°10 (allèle G) | Position du nucléotide en 3' sur SEQ ID N°10 (allèle G) |
| 1 | 3618 | 5457 |

L'invention est également relative à un acide nucléique comprenant au moins 12 nucléotides consécutifs d'un polynucléotide intronique du gène (G/g), décrit dans le tableau 3 ci-dessus, qui sont inclus dans l'acide nucléique de séquence SEQ ID N°10.

Un tel acide nucléique peut être utilisé comme sonde ou amorce oligonucléotidique pour détecter la présence d'au moins une copie du gène (G/g) dans un échantillon, ou encore pour amplifier une séquence cible déterminée au sein du gène (A/a).

Un tel acide nucléique peut aussi être utilisé pour amplifier une séquence cible déterminée au sein du gène (G/g) ou l'inhiber par une approche sens ou co-suppression, ou par l'utilisation d'ARN double brin (Wassenegger et al. 1996 ; Kooter et al. 1999) pour interférence. Un tel acide nucléique peut également être utilisé pour rechercher des variants alléliques fonctionnels du gène (G/g), qui pourront être utilisés dans une méthode de sélection de plantes possédant un type sexuel déterminé.

***Autres acides nucléiques selon l'invention, codant pour la protéine CmWIP1***

L'invention a également pour objet un acide nucléique comprenant un polynucléotide possédant au moins 95% d'identité en nucléotides avec la séquence nucléotidique débutant au nucléotide en position 3000 et se terminant au nucléotide en position 5901 de la séquence SEQ ID N°10 ainsi qu'un acide nucléique de séquence complémentaire.

L'invention concerne aussi un acide nucléique possédant au moins 95% d'identité en nucléotides avec la séquence nucléotidique débutant au nucléotide en position 3000 et se terminant au nucléotide en position 5901 de la séquence SEQ ID N°10, ainsi qu'un acide nucléique de séquence complémentaire.

L'invention a encore pour objet un acide nucléique comprenant la séquence nucléotidique débutant au nucléotide en position 3000 et se terminant au nucléotide en position 5901 de la séquence SEQ ID N°10 ou un acide nucléique de séquence complémentaire.

L'invention a également trait à un acide nucléique consistant en la séquence nucléotidique débutant au nucléotide en position 3000 et se terminant au nucléotide en position 5901 de la séquence SEQ ID N°10 ou un acide nucléique de séquence complémentaire.

Un autre objet de l'invention consiste en un acide nucléique comprenant, au moins :

(i) une séquence ayant au moins 95% d'identité avec le polynucléotide allant du nucléotide 3000 au nucléotide 3617 de la séquence SEQ ID N°10,

(ii) une séquence ayant au moins 95% d'identité avec le polynucléotide allant du nucléotide 5458 au nucléotide 5901 de la séquence SEQ ID N°10, et

L'invention a encore pour objet un acide nucléique comprenant, de l'extrémité 5' vers l'extrémité 3' :

(i) une séquence allant du nucléotide 3000 au nucléotide 3617 de la séquence SEQ ID N°10, et

(ii) une séquence allant du nucléotide 5458 au nucléotide 5901 de la séquence SEQ ID N°10.

Un acide nucléique codant pour la protéine CmWIP1, peut comprendre en outre des séquences leader et terminateur, classiques pour l'homme du métier.

**Produits de transcription et de traduction du gène (G/g) et polypeptides selon l'invention.**

L'expression de l'acide nucléique génomique codant la protéine CmWIP1 conduit à la synthèse d'un ARN messager dont l'ADNc est l'acide nucléique de séquence SEQ ID N° 11, qui est aussi l'un des objets de la présente invention.

L'invention a donc encore pour objet le polypeptide comprenant la séquence d'acides aminés SEQ ID N°12, également nommé « protéine CmWIP1 » dans la présente description, ainsi qu'un polypeptide possédant au moins 95% d'identité en acides aminés avec la séquence SEQ ID N°12, ou un fragment ou un variant de celui-ci.

Une illustration selon l'invention d'une protéine CmWIP1 possédant au moins 95% d'identité en acides aminés avec la séquence SEQ ID N° 12 consiste en la protéine de séquence SEQ ID N° 16, qui diffère de la protéine de séquence SEQ ID N° 12 par la délétion d'un résidu Sérine..

Un fragment d'une protéine CmWIP1 selon l'invention comprend au moins 10, 50, 100, 200, 300, 320, 330, 340, 345 ou 353 acides aminés consécutifs d'un polypeptide de séquence SEQ ID N°12.

L'invention est également relative à un polypeptide comprenant une séquence en acides aminés ayant au moins 95% d'identité en acides aminés avec la séquence d'une protéine CmWIP1 de séquence SEQ ID N°12.

Avantageusement, fait aussi partie de l'invention un polypeptide ayant au moins 96%, 97%, 98%, 99%, 99,1%, 99,2%, 99,3%, 99,4%, 99,5%, 99,6%, 99,7%, 99,8% ou 99,9% d'identité en acides aminés avec la séquence d'un polypeptide de séquence SEQ ID N°12, ou un fragment peptidique de ce dernier.

De manière générale, les polypeptides selon la présente invention se présentent sous une forme isolée ou purifiée.

Un polypeptide selon l'invention peut être obtenu par recombinaison génétique selon des techniques bien connues de l'homme du métier, par exemple des techniques décrites dans AUSUBEL et al. (1989).

Un polypeptide selon l'invention peut être également préparé par des techniques classiques de synthèse chimique, indifféremment en solution homogène ou en phase solide.

A titre illustratif, un polypeptide selon l'invention pourra être préparé par la technique en solution homogène décrite par HOUBEN WEIL (1974) ou encore par la technique de synthèse en phase solide décrite par MERRIFIELD (1965a; 1965b).

De préférence, les polypeptides variants d'un polypeptide selon l'invention conservent leur capacité à être reconnus par des anticorps dirigés contre les polypeptides de séquences SEQ ID N°12.

Un polypeptide codé par le gène (G/g) selon l'invention, tel qu'un polypeptide de séquence en acides aminés SEQ ID N°12, ou encore un variant ou un fragment peptidique de ce dernier est utile notamment pour la préparation d'anticorps destinés à la détection de la présence et/ou de l'expression d'un polypeptide de séquences SEQ ID N°12 ou d'un fragment peptidique de ce dernier dans un échantillon.

Outre la détection de la présence d'un polypeptide codé par le gène (G/g) ou encore d'un fragment peptidique d'un tel polypeptide dans un échantillon, des anticorps dirigés contre ces polypeptides sont utilisés pour quantifier la synthèse d'un polypeptide de séquences SEQ ID N°12, par exemple dans des cellules d'une plante, et déterminer ainsi le sexe de la plante, sans pour autant devoir la cultiver.

Par "anticorps" au sens de la présente invention, on entendra notamment des anticorps polyclonaux ou monoclonaux ou des fragments (par exemple les fragments $F(ab)'_2$, F(ab)) ou encore tout polypeptide comprenant un domaine de l'anticorps initial reconnaissant le polypeptide ou le fragment de polypeptide cible selon l'invention.

Des anticorps monoclaux peuvent être préparés à partir d'hybridomes selon la technique décrite par KOHLER et MILSTEIN (1975)

La présente invention concerne également des anticorps dirigés contre un polypeptide tel que décrit ci-dessus ou un fragment ou un variant de ce dernier, tel que produit dans la technique du trioma ou encore la technique d'hybridome décrite par KOZBOR et al. (1983).

L'invention a également trait à des fragments d'anticorps simple chaîne Fv (ScFv) tels que décrits dans le brevet US N°4,946,778 ou encore par MARTINEAU et al. (1998).

Les anticorps selon l'invention comprennent également des fragments d'anticorps obtenus à l'aide de banques de phages telles que décrites par RIDDER et al. (1995) ou encore des anticorps humanisés tels que décrits par REINMANN et al. (1997) et LEGER et al. (1997). Les préparations d'anticorps selon l'invention sont utiles dans des tests de détection immunologiques destinés à l'identification de la présence et/ou de la quantité d'un polypeptide de séquences SEQ ID N°3, ou d'un fragment peptidique de celui-ci, présent dans un échantillon.

Un anticorps selon l'invention pourra comprendre en outre un marqueur détectable isotopique ou non isotopique, par exemple fluorescent, ou encore être couplé à une molécule telle que la biotine, selon des techniques bien connues de l'homme du métier.

Ainsi, l'invention a en outre pour objet un procédé pour détecter la présence d'un polypeptide conforme à l'invention dans un échantillon, ledit procédé comprenant les étapes de:

a) mettre en contact l'échantillon à tester avec un anticorps tel que décrit ci-dessus;
b) détecter le complexe antigène/anticorps formé.

L'invention est également relative à un nécessaire ou kit de diagnostic pour la détection de la présence d'un polypeptide conforme à l'invention dans un échantillon, ledit nécessaire comprenant:

a) un anticorps tel que défini ci-dessus;
b) le cas échéant, un ou plusieurs réactifs nécessaires à la détection du complexe antigène/anticorps formé.

Un autre objet de l'invention consiste en l'utilisation d'un acide nucléique ou d'un variant allélique d'un acide nucléique tel que défini ci-dessus dans des programmes de sélection de plantes pour l'obtention de plantes dont le type floral a été modifié.

***Acides nucléiques comprenant un polynucléotide régulateur (PG) fonctionnel***

Un polynucléotide régulateur ou promoteur (PG) fonctionnel selon l'invention consiste en un acide nucléique qui permet l'expression de la protéine CmWIP1 de séquence SEQ ID N°12 chez les plantes dicotylédones.

Un tel polynucléotide régulateur (PG) fonctionnel permet ainsi, lorsqu'il est introduit artificiellement dans une plante, de modifier le sexe des fleurs d'une telle plante, et en particulier permet d'obtenir des plantes mâles et femelles ou mâles et hermaphrodites, capables d'autopollinisation

L'invention a donc également pour objet un acide nucléique comprenant un polynucléotide possédant au moins 95% d'identité en nucléotides avec la séquence nucléotidique débutant au nucléotide en position 1 et se terminant au nucléotide en position 2999 de la séquence SEQ ID N°10 ainsi qu'un acide nucléique de séquence complémentaire.

L'invention concerne aussi un acide nucléique possédant au moins 95% d'identité en nucléotides avec la séquence nucléotidique débutant au nucléotide en position 1 et se terminant au nucléotide en position 2999 de la séquence SEQ ID N°10, ainsi qu'un acide nucléique de séquence complémentaire.

L'invention a encore pour objet un acide nucléique comprenant la séquence nucléotidique débutant au nucléotide en position 1 et se terminant au nucléotide en position 2999 de la séquence SEQ ID N°10 ou un acide nucléique de séquence complémentaire.

L'invention a également trait à un acide nucléique consistant en la séquence nucléotidique débutant au nucléotide en position 1 et se terminant au nucléotide en position 2999 de la séquence SEQ ID N°10 ou un acide nucléique de séquence complémentaire.

Le polynucléotide régulateur allant du nucléotide en position 1 jusqu'au nucléotide en position 2999 de la séquence SEQ ID N° 10 est également référencé comme l'acide nucléique de séquence SEQ ID N° 13 dans la présente description.

L'invention a également trait à un acide nucléique comprenant au moins 12 nucléotides consécutifs d'un polynucléotide régulateur, tel que défini ci-dessus.

Un tel acide nucléique peut être utilisé comme sonde ou amorce oligonucléotidique pour détecter la présence d'au moins une copie de l'allèle (G) du gène (G/g) dans un échantillon, pour amplifier une séquence cible déterminée au sein du gène (G/g). Un tel acide nucléique peut également être utilisé pour rechercher des variants alléliques fonctionnels du gène (G/g), ou pourront être utilisés dans une méthode de sélection de plantes possédant un type sexuel déterminé.

Des procédés de détection mettant en œuvre des acides nucléiques tels que décrits ci-dessus sont décrits dans la partie intitulée « Procédés de sélection selon l'invention »

Un tel acide nucléique peut aussi être utilisé pour inhiber une séquence cible déterminée au sein du gène (G/g) par une approche antisens ou co-suppression, ou par l'utilisation d'ARN double brin (Wassenegger et al. 1996 ; Kooter et al. 1999) pour interférence.

***Acides nucléiques comprenant un polynucléotide régulateur Pg non fonctionnel***

Un polynucléotide régulateur (Pa), ou promoteur non fonctionnel selon l'invention est un acide nucléique qui :

(i) ne permet pas l'expression de la protéine CmWIP1 de séquence SEQ ID N°12 dans une cellule hôte, ou
(ii) permet l'expression de cette protéine à un niveau très faible en comparaison du niveau observé avec le polynucléotide régulateur (PG), ou
(iii) permet l'expression de la protéine CmWIP1 au cours de la vie de la plante, pendant une durée inférieure, en comparaison de celle observée avec le polynucléotide régulateur (PG).

Un tel polynucléotide régulateur (Pg) non fonctionnel permet ainsi, lorsqu'il est introduit artificiellement dans une plante, par exemple en remplacement d'un polynucléotide (G) de modifier le sexe des fleurs d'une telle plante, et en particulier permet d'obtenir des plantes hermaphrodites, capables d'autopollinisation, ou bien des plantes femelles.
Un tel acide nucléique peut être utilisé comme sonde ou amorce oligonucléotidique pour détecter la présence d'au moins une copie de l'allèle (a) du gène (G/g) dans un échantillon, ou encore pour amplifier une séquence cible déterminée au sein du gène (G/g).
L'invention concerne enfin des acides nucléiques comprenant l'association un ou plusieurs acides nucléiques tels que définis ci-dessus, par exemple un acide nucléique codant pour une protéine CmWIP1 fonctionnelle sous le contrôle d'un promoteur de type (PG) ou (Pg).

**Définitions générales**

Selon l'invention, toute technique classique de biologie moléculaire, de microbiologie et d'ADN recombinant connue de l'homme du métier peut être utilisée. De telles techniques sont décrites par exemple par SAMBROOK et al. (1989), GLOVER (1985), GAIT (1984), HAMES et HIGGINS (1984), BERBAL (1984) et AUSUBEL et al. (1994) .
De manière préférée, tout acide nucléique et tout polypeptide selon l'invention se présente sous une forme isolée ou purifiée.
Le terme " isolé " au sens de la présente invention désigne un matériel biologique qui a été soustrait à son environnement originel (l'environnement dans lequel il est localisé naturellement). Par exemple, un polynucléotide présent à l'état naturel dans une plante n'est pas isolé. Le même polynucléotide séparé des acides nucléiques adjacents au sein desquels il est naturellement inséré dans le génome de la plante est isolé. Un tel polynucléotide peut être inclus dans un vecteur et/ou un tel polynucléotide peut être inclus dans une composition et demeurer néanmoins à l'état isolé du fait que le vecteur ou la composition ne constitue pas son environnement naturel.
Le terme " purifié " ne nécessite pas que le matériel soit présent sous une forme de pureté absolue, exclusive de la présence d'autres composés. Il s'agit plutôt d'une définition relative.
Un polynucléotide ou un polypeptide est à l'état purifié après purification du matériel de départ ou du matériel naturel d'au moins un ordre de grandeur, de préférence 2 ou 3 et préférentiellement quatre ou cinq ordres de grandeur.
Aux fins de la présente description, l'expression " séquence nucléotidique " peut être employée pour désigner indifféremment un polynucléotide ou un acide nucléique. L'expression " séquence nucléotidique " englobe le matériel génétique lui-même et n'est donc pas restreinte à l'information concernant sa séquence.
Les termes " acide nucléique ", "polynucléotide ", " oligonucléotide " ou encore " séquence nucléotidique " englobent des séquences d'ARN, d'ADN, d'ADNc ou encore des séquences hybrides ARN/ADN de plus d'un nucléotide, indifféremment sous la forme simple brin ou sous la forme de duplex.
Le terme " nucléotide " désigne à la fois les nucléotides naturels (A, T, G, C) ainsi que des nucléotides modifiés qui comprennent au moins une modification telle que (i) un analogue d'une purine, (ii) un analogue d'une pyrimidine, ou (iii) un sucre analogue, de tels nucléotides modifiés étant décrits par exemple dans la demande PCT N°WO 95/04064.
Aux fins de la présente invention, un premier polynucléotide est considéré comme étant " complémentaire " d'un second polynucléotide lorsque chaque base du premier nucléotide est appariée à la base complémentaire du second polynucléotide dont l'orientation est inversée. Les bases complémentaires sont A et T (ou A et U), et C et G.
Selon l'invention, un premier acide nucléique ayant au moins 95% d'identité avec un second acide nucléique de référence, possédera au moins 95%, de préférence au moins 96%, 97%, 98%, 98,5%, 99%, 99,1%, 99,2%, 99,3%, 99,4%, 99,5%, 99,6%, 99,7%, 99,8% ou 99,9% d'identité en nucléotides avec ce second polynucléotide de référence, le pourcentage d'identité entre deux séquences étant déterminé comme décrit ci-dessous.
Le « pourcentage d'identité » entre deux séquences d'acides nucléiques, au sens de la présente invention, est déterminé en comparant les deux séquences alignées de manière optimale, à travers une fenêtre de comparaison.

La partie de la séquence nucléotidique dans la fenêtre de comparaison peut ainsi comprendre des additions ou des délétions (par exemple des « gaps ») par rapport à la séquence de référence (qui ne comprend pas ces additions ou ces délétions) de manière à obtenir un alignement optimal entre les deux séquences.

Le pourcentage d'identité est calculé en déterminant le nombre de positions auxquelles une base nucléique identique est observée pour les deux séquences comparées, puis en divisant le nombre de positions auxquelles il y a identité entre les deux bases nucléiques par le nombre total de positions dans la fenêtre de comparaison, puis en multipliant le résultat par cent afin d'obtenir le pourcentage d'identité en nucléotides des deux séquences entre elles.

L'alignement optimal des séquences pour la comparaison peut être réalisé de manière informatique à l'aide d'algorithmes connus.

De manière tout à fait préférée, le pourcentage d'identité de séquence est déterminé à l'aide du logiciel CLUSTAL W (version 1.82) les paramètres étant fixés comme suit : (1) CPU MODE = ClustalW mp ; (2) ALIGNMENT = « full » ; (3) OUTPUT FORMAT = « aln w/numbers » ; (4) OUTPUT ORDER = « aligned » ; (5) COLOR ALIGNMENT = « no » ; (6) KTUP (word size) = « default » ; (7) WINDOW LENGTH = « default » ; (8) SCORE TYPE = « percent » ; (9) TOPDIAG = « default » ; (10) PAIRGAP = « default » ; (11) PHYLOGENETIC TREE/TREE TYPE = « none » ; (12) MATRIX = « default » ; (13) GAP OPEN = « default » ; (14) END GAPS = « default » ; (15) GAP EXTENSION = « default » ; (16) GAP DISTANCES = « default » ; (17) TREE TYPE = « cladogram » et (18) TREE GRAP DISTANCES = « hide ».

Un acide nucléique possédant au moins 95% d'identité en nucléotides avec un acide nucléique selon l'invention englobe les " variants " d'un acide nucléique selon l'invention.

Par " variant " d'un acide nucléique selon l'invention, on entend un acide nucléique qui diffère de l'acide nucléique de référence par une ou plusieurs substitutions, additions ou délétions d'un nucléotide, par rapport à l'acide nucléique de référence. Un variant d'un acide nucléique selon l'invention peut être d'origine naturelle, tel qu'un variant allélique qui existe naturellement. Un tel acide nucléique variant peut être également un acide nucléique non naturel obtenu, par exemple, par des techniques de mutagenèse.

En général, les différences entre l'acide nucléique de référence et l'acide nucléique " variant " sont réduites de telle sorte que l'acide nucléique de référence et l'acide nucléique variant ont des séquences nucléotidiques très similaires et, dans de nombreuses régions, identiques. Les modifications nucléotidiques présentes dans un acide nucléique variant peuvent être silencieuses, ce qui signifie qu'elles n'affectent pas la séquence d'acides aminés qui peut être codée par cet acide nucléique variant.

Les modifications de nucléotides dans l'acide nucléique variant peuvent aussi résulter en des substitutions, additions ou délétions d'un ou plusieurs acides aminés dans la séquence du polypeptide qui peut être codé par cet acide nucléique variant.

De manière tout à fait préférée, un acide nucléique variant selon l'invention comportant une phase de lecture ouverte, code pour un polypeptide qui conserve la même fonction ou la même activité biologique que le polypeptide codé par l'acide nucléique de référence.

De manière tout à fait préférée, un acide nucléique variant selon l'invention et qui comporte une phase de lecture ouverte, code pour un polypeptide qui conserve la capacité d'être reconnu par des anticorps dirigés contre le polypeptide codé par l'acide nucléique de référence.

Font partie des « variants » d'un acide nucléique codant la protéine ACS les acides nucléiques des gènes orthologues à la protéine CmWIP1 inclus dans le génome de plantes, et possédant une identité en nucléotides d'au moins 95% avec un acide nucléique codant la protéine CmWIP1.

Par "fragment" d'un acide nucléique selon l'invention, on entend une séquence nucléotidique d'une longueur réduite par rapport à l'acide nucléique de référence, le fragment d'acide nucléique possédant une séquence nucléotidique identique à la séquence nucléotidique de l'acide nucléique de référence sur la partie commune. De tels fragments d'un acide nucléique selon l'invention possèdent au moins 12, 15, 18, 20, 25, 30, 35, 40, 45, 50, 60, 70, 80, 90, 100, 150, 200, 300, 400, 500, 1000, 2000 ou 3000 nucléotides consécutifs de l'acide nucléique de référence, la longueur maximale en nucléotides d'un fragment d'un acide nucléique selon l'invention étant bien entendu limitée par la longueur maximale en nucléotides de l'acide nucléique de référence.

## Sondes et amorces

Les acides nucléiques selon l'invention, et en particulier les séquences nucléotidiques SEQ ID N°10 et SEQ ID N°11, leurs fragments d'au moins 12 nucléotides, les polynucléotides régulateurs (PG) et (Pg), ainsi que les acides nucléiques de séquence complémentaire, sont utiles pour la détection de la présence d'au moins une copie d'une séquence nucléotidique du gène (G/g) ou encore d'un fragment ou d'un variant allélique de cette dernière dans un échantillon.

En particulier, les sondes et amorces ci-dessus dérivés des séquence SEQ ID N°10, et en particulier dérivées du polynucléotide régulateur (PG) peuvent être utilisées pour détecter la présence de l'allèle (G) dans une plante dicotylédone.

Font également partie de l'invention les sondes et amorces nucléotidiques hybridant, dans des conditions d'hybridation de forte stringence, avec un acide nucléique choisi parmi les séquences SEQ ID N°10 et SEQ ID N°11, ou avec un polynucléotide régulateur (PG) ou (Pg).

L'invention a donc également pour objet un acide nucléique, utilisable en tant que sonde ou amorce, hybridant spécifiquement avec un acide nucléique tel que défini ci-dessus.

Les conditions d'hybridation ci-dessous sont mises en œuvre pour l'hybridation d'un acide nucléique, sonde ou amorce, de 20 bases de longueur.

Le niveau et la spécificité d'hybridation dépend de différents paramètres, tels que :

a) la pureté de la préparation de l'acide nucléique sur lequel la sonde ou l'amorce doit s'hybrider ;
b) la composition en bases de la sonde ou de l'amorce, les paires de base G-C possédant une plus grande stabilité thermique que les paires de bases A-T ou A-U ;
c) la longueur de la séquence de bases homologues entre la sonde ou l'amorce et l'acide nucléique ;
d) la force ionique : le taux d'hybridation augmente avec l'accroissement de la force ionique et la durée du temps d'incubation ;
e) la température d'incubation ;
f) la concentration de l'acide nucléique sur lequel la sonde ou l'amorce doit s'hybrider ;
g) la présence d'agents dénaturants tels que des agents favorisant la rupture des liaisons hydrogène, comme le formamide ou l'urée, qui accroissent la stringence de l'hybridation ;
h) le temps d'incubation, le taux d'hybridation augmentant avec la durée de l'incubation ;
i) la présence d'agents d'exclusion de volume, tels que le dextran ou le sulfate de dextran, qui augmentent le taux d'hybridation du fait qu'ils accroissent les concentrations effectives de la sonde ou l'amorce et de l'acide nucléique qui doit s'hybrider, au sein de la préparation.

Les paramètres définissant les conditions de stringence dépendent de la température à laquelle 50% des brins appariés se séparent (Tm).

Pour les séquences comprenant plus de 360 bases, Tm est définie par la relation:

Tm= 81,5 + 0,41 (%G+C)+16,6 Log(concentration en cations) - 0,63 (% formamide)-(600/nombre de bases) (SAMBROOK et al., (1989), pages 9.54-9.62).

Pour les séquences de longueur inférieure à 30 bases, Tm est définie par la relation: Tm= 4(G+C) + 2(A+T).

Dans des conditions de stringence appropriées, dans lesquelles les séquences aspécifiques n'hybrident pas, la température d'hybridation est approximativement de 5 à 30°C, de préférence de 5 à 10 °C en-dessous de Tm.

Par « conditions d'hybridation de forte stringence » selon l'invention, on entend des conditions d'hybridation telles que l'on se place à une température d'hybridation de 5°C au-dessous du Tm.

Les conditions d'hybridation ci-dessus décrites peuvent être adaptées en fonction de la longueur et de la composition en bases de l'acide nucléique dont l'hybridation est recherchée ou du type de marquage choisi, selon les techniques connues de l'homme du métier.

Les conditions convenables d'hybridation peuvent par exemple être adaptées selon l'enseignement contenu dans l'ouvrage de HAMES et HIGGINS (1985) ou encore dans l'ouvrage de AUSUBEL et al. (1989).

A titre illustratif, les conditions d'hybridation utilisées pour un acide nucléique de 200 bases de longueur sont les suivantes :

*Préhybridation:*

mêmes conditions que pour l'hybridation
durée: 1 nuit.

*Hybridation:*

5 x SSPE (0.9 M NaCl, 50 mM phosphate de sodium pH 7.7, 5 mM EDTA)
5 x Denhardt's (0.2% PVP, 0.2% Ficoll, 0.2% SAB)
100 μg/ml ADN de sperme de saumon
0.1% SDS
durée: 1 nuit.

*Lavages:*

2 x SSC, 0.1% SDS 10 min 65°C
1 x SSC, 0.1 % SDS 10 min 65 °C
0.5 x SSC, 0.1 % SDS 10 min 65 °C
0.1 x SSC, 0.1% SDS 10 min 65 °C.

Les sondes ou les amorces nucléotidiques selon l'invention comprennent au moins 12 nucléotides consécutifs d'un acide nucléique selon l'invention, en particulier d'un acide nucléique de séquences SEQ ID N°10 ou SEQ ID N°11 ou de sa séquence complémentaire, d'un acide nucléique ayant 95% d'identité en nucléotides avec une séquence choisie parmi les séquences SEQ ID N°10 ou SEQ ID N° 11 ou de sa séquence complémentaire ou encore d'un acide nucléique hybridant dans des conditions d'hybridation de forte stringence avec une séquence choisie parmi les séquences SEQ ID N°10 ou SEQ ID N° 11 ou de sa séquence complémentaire.

De préférence, des sondes ou amorces nucléotidiques selon l'invention auront une longueur d'au moins 12, 15, 18, 20, 25, 30, 35, 40, 45, 50, 60, 100, 150, 200, 300, 400, 500, 1000, 2000 ou 3000 nucléotides consécutifs d'un acide nucléique selon l'invention.

Alternativement, une sonde ou une amorce nucléotidique selon l'invention consistera et/ou comprendra les fragments d'une longueur de 12, 15, 18, 20, 25, 30, 35, 40, 45, 50, 60, 100, 150, 200, 300, 400, 500, 1000, 2000 ou 3000 nucléotides consécutifs d'un acide nucléique selon l'invention.

Une amorce ou une sonde nucléotidique selon l'invention peut être préparée par toute méthode adaptée bien connue de l'homme du métier, y compris par clonage et action d'enzymes de restriction ou encore par synthèse chimique directe selon des techniques telles que la méthode au phosphodiester de NARANG et al. (1979) ou de BROWN et al. (1979), la méthode au diéthylphosphoramidites de BEAUCAGE et al. (1980) ou encore la technique sur support solide décrite dans le brevet européen n°EP 0 707 592. Chacun des acides nucléiques selon l'invention, y compris les sondes et amorces oligonucléotidiques décrites ci-dessus, peut être marqué, si désiré, en incorporant une molécule détectable, c'est-à-dire un marqueur détectable, par des moyens spectroscopiques, photochimiques, biochimiques, immunochimiques ou encore chimiques.

Par exemple, de tels marqueurs peuvent consister en des isotopes radioactifs ($^{32}P$ $^{3}H$, $^{35}S$), des molécules fluorescentes (5-bromodéoxyuridine, fluorescéine, acétylaminofluorène) ou encore des ligands tels que la biotine.

Le marquage des sondes est fait de préférence par incorporation de molécules marquées au sein des polynucléotides par extension d'amorces, ou bien par rajout sur les extrémités 5' ou 3'.

Des exemples de marquage non radioactif de fragments d'acides nucléiques sont décrits notamment dans le brevet français n°FR 78 10 975 ou encore dans les articles de URDEA et al. (1988) ou SANCHEZ PESCADOR et al. (1988).

De manière avantageuse, les sondes selon l'invention peuvent avoir des caractéristiques structurelles de nature à permettre une amplification du signal, telles que les sondes décrites par URDEA et al; (1991) ou encore dans le brevet européen n°EP 0 225 807 (Chiron).

Les sondes oligonucléotidiques selon l'invention peuvent être utilisées notamment dans des hybridations de type Southern à tout acide nucléique codant pour la protéine CmWIP1, en en particulier les acides nucléiques de séquences SEQ ID N°10 ou SEQ ID N° 11, ou encore dans des hybridations à l'ARN lorsque l'expression du transcrit correspondant est recherchée dans un échantillon.

Les sondes selon l'invention peuvent aussi être utilisées pour la détection de produits d'amplification PCR ou encore pour la détection de mésappariements.

Des sondes ou amorces nucléotidiques selon l'invention peuvent être immobilisées sur un support solide. De tels supports solides sont bien connus de l'homme du métier et comprennent des surfaces des puits de plaques de micro-titration, des billes de polystyrène, des billes magnétiques, des bandes de nitrocellulose ou encore des microparticules telles que des particules de latex.

En conséquence, l'invention a encore pour objet un acide nucléique utilisable en tant que sonde ou amorce nucléotidique caractérisé en ce qu'il comprend au moins 12 nucléotides consécutifs d'un acide nucléique tel que défini ci-dessus, en particulier d'un acide nucléique de séquences nucléotidiques SEQ ID N°10 et SEQ ID N°11.

L'invention est également relative à un acide nucléique utilisable en tant que sonde ou amorce nucléotidique caractérisé en ce qu'il consiste en un polynucléotide d'au moins 12 nucléotides consécutifs d'un acide nucléique selon l'invention, de manière tout à fait préférée d'un acide nucléique de séquences choisies parmi les séquences nucléotidiques SEQ ID N°10 et SEQ ID N°11.

Comme décrit ci-dessus, un tel acide nucléique peut en outre être caractérisé en ce qu'il est marqué par une molécule détectable.

Un acide nucléique utilisable en tant que sonde ou amorce nucléotidique pour la détection ou l'amplification d'une séquence génomique, de l'ARNm ou de l'ADNc du gène (G/g) peut en outre être caractérisé en ce qu'il est choisi parmi les séquences suivantes :

a) les séquences nucléotidiques hybridant, dans des conditions d'hybridation de forte stringence, avec un acide nucléique de séquence SEQ ID N°10 ou SEQ ID N°11; et

b) les séquences comprenant au moins 12 nucléotides consécutifs d'un acide nucléique de séquence SEQ ID N°10 ou SEQ ID N°11.

## Vecteurs, cellules et plantes selon l'invention

Dans la combinaison de deux éléments génétiques pour le contrôle du développement du type floral d'une plante dicotylédone objet de l'invention, pour permettre qu'au moins un des éléments génétiques de contrôle soit artificiellement introduit dans la plante dicotylédone, les acides nucléiques, et les polynucléotides régulateurs définis ci-dessus doivent être introduits dans des vecteurs, puis dans des cellules.

Ainsi, l'invention a également pour objet des vecteurs, des cellules et des plantes transformées, qui comprennent les polynucléotides régulateurs (PG) et (Pg), les acides nucléiques codant pour des protéines CmWIP1 actives ou non actives, ainsi que les acides nucléiques correspondant aux allèles (G) et (g) tels que décrits ci-dessus, et les amorces définies ci-dessus.

### *Vecteurs*

Un acide nucléique tel que défini ci-dessus, nommé ci-après acide nucléique d'intérêt, peut être inséré dans un vecteur approprié.

Par "vecteur" au sens de la présente invention, on entendra une molécule d'ADN ou d'ARN circulaire ou linéaire qui est indifféremment sous forme simple brin ou double brin.

Un vecteur recombinant selon l'invention est de préférence un vecteur d'expression, ou plus spécifiquement un vecteur d'insertion, un vecteur de transformation ou un vecteur d'intégration.

Il peut s'agir notamment d'un vecteur d'origine bactérienne ou virale.

Dans tous les cas, l'acide nucléique d'intérêt est placé sous le contrôle d'une ou plusieurs séquences contenant des signaux de régulation de son expression dans la plante considérée, soit que les signaux de régulation soient tous contenus dans l'acide nucléique d'intérêt, comme c'est le cas dans les constructions d'acides nucléiques décrites dans la section précédente, soit que l'un, plusieurs d'entre eux, ou encore tous les signaux de régulation soient contenus dans le vecteur receveur dans lequel l'acide nucléique d'intérêt a été inséré.

Un vecteur recombinant selon l'invention comprend avantageusement des séquences d'initiation et d'arrêt de la transcription appropriées.

En outre, les vecteurs recombinants selon l'invention peuvent inclure une ou plusieurs origines de réplication fonctionnelles dans les cellules hôtes dans lesquelles leur expression est recherchée, ainsi que, le cas échéant, des séquences nucléotidiques marqueurs de sélection.

Les vecteurs recombinants selon l'invention peuvent aussi inclure un ou plusieurs des signaux de régulation de l'expression tels que définis ci-dessus dans la description.

Les vecteurs bactériens préférés selon l'invention sont par exemple les vecteurs pBR322 (ATCC n°37 017) ou encore les vecteurs tels que pAA223-3 (Pharmacia, Uppsala, Suède) et pGEM1 (Promega Biotech, Madison, WI, Etats-Unis).

On peut encore citer d'autres vecteurs commercialisés tels que les vecteurs pQE70, pQE60, pQE9 (Quiagen), psiX174, pBluescript SA, pNH8A, pMH16A, pMH18A, pMH46A, pWLNEO, pSV2CAT, pOG44, pXTI et pSG (Stratagene).

Il peut s'agir également de vecteurs du type *Baculovirus* tel que le vecteur pVL1392/1393 (Pharmingen) utilisé pour transfecter les cellules de la lignée Sf9 (ATCC N°CRL 1711) dérivée de *Spodoptera frugiperda.*

De manière préférée, et pour l'application principale des vecteurs de l'invention consistant à obtenir une expression stable, et préférentiellement inductible, d'une séquence codant pour une protéine CmWIP1 dans une plante, on aura recours à des vecteurs spécialement adaptés pour l'expression de séquences d'intérêt dans des cellules de plantes, tels que les vecteurs suivants:

- vecteur pBIN19 (BEVAN et al.), commercialisé par la Société CLONTECH (Palo Alto, Californie, USA);
- vecteur pBI 101 (JEFFERSON, 1987), commercialisé par la Société CLONTECH ;
- vecteur pBI121 (JEFFERSON, 1987), commercialisé par la Société CLONTECH;
- vecteur pEGFP; Yang et al. (1996), commercialisé par la Société CLONTECH;
- vecteur pCAMBIA 1302 (HAJDUKIIEWICZ et al., 1994)
- vecteurs intermédiaires et superbinaires dérivés des vecteurs pSB12 et pSB1 décrits par Japan Tobacco (EP 672 752 et Ishida et al., 1996).

*Cellules*

Les méthodes les plus répandues pour introduire des acides nucléiques dans des cellules bactériennes peuvent être utilisées dans le cadre de cette invention. Ce peut être la fusion de cellules réceptrices avec des protoplastes bactériens contenant l'ADN, l'électroporation, le bombardement par projectiles, l'infection par des vecteurs viraux, etc. Les cellules bactériennes sont souvent utilisées pour amplifier le nombre de plasmides contenant le construit comprenant la séquence nucléotidique, objet de l'invention. Les bactéries sont mises en culture et les plasmides sont ensuite isolés selon des méthodes bien connus de l'homme du métier (se reporter aux manuels de protocoles déjà cités), incluant les kits de purification de plasmides vendus dans le commerce comme par exemple EasyPrepl de Pharmacia Biotech ou QIAexpress Expression System de Qiagen. Les plasmides ainsi isolés et purifiés sont ensuite manipulés pour produire d'autres plasmides qui seront utilisés pour transfecter les cellules végétales.

Pour permettre l'expression d'un acide nucléique d'intérêt selon l'invention placé sous le contrôle d'une séquence régulatrice appropriée, les acides nucléiques ou les vecteurs recombinants définis dans la présente description doivent être introduits dans une cellule hôte. L'introduction des polynucléotides selon l'invention dans une cellule hôte peut être réalisée *in vitro*, selon les techniques bien connues de l'homme du métier.

L'invention a en outre pour objet une cellule hôte transformée par un acide nucléique selon l'invention ou par un vecteur recombinant tel que défini ci-dessus.

Une telle cellule hôte transformée est préférentiellement d'origine bactérienne, fongique ou végétale.

Ainsi, peuvent être notamment utilisées des cellules bactériennes de différentes souches de *Escherichia coli* ou encore d'*Agrobacterium tumefaciens.*

Avantageusement, la cellule hôte transformée est une cellule de plante ou encore un protoplaste de plante.

Parmi les cellules susceptibles d'être transformées selon le procédé de l'invention, on peut citer à titre d'exemples des cellules de plantes dicotylédone, de préférence appartenant à la famille des *cucurbitaceae,* dont les membres sont détaillées ci-après dans la partie intitulée « plantes selon l'invention ».

Préférentiellement, il s'agit d'une cellule ou d'un protoplaste d'une plante appartenant à l'espèce *cucumis melo.*

L'invention a également pour objet l'utilisation d'un acide nucléique d'intérêt, pour fabriquer une plante transformée dont le phénotype sexuel est modifié.

L'invention est également relative à l'utilisation d'un vecteur recombinant tel que défini dans la présente description pour fabriquer une plante transformée dont le phénotype sexuel est modifié.

L'invention concerne aussi l'utilisation d'un hôte cellulaire transformé par un acide nucléique d'intérêt, pour fabriquer une plante transformée dont le phénotype sexuel est modifié.

L'invention concerne aussi une plante transformée comprenant une pluralité de cellules hôtes telles que définies ci-dessus.

*Plantes transformées selon l'invention*

L'invention concerne aussi un organisme multicellulaire végétal transformé, caractérisé en ce qu'il comprend une cellule hôte transformée ou une pluralité de cellules hôtes transformées par au moins l'un des acides nucléiques tels que définis ci-dessus, ou encore par un vecteur recombinant comprenant un tel acide nucléique.

La plante transformée peut contenir une pluralité de copies d'un acide nucléique codant pour la protéine CmWIP1, dans les situations dans lesquelles une surexpression de la protéine CmWIP1 est recherchée. Une surexpression de la protéine CmWIP1 est recherchée notamment lorsque l'on souhaite obtenir des plantes produisant des fleurs mâles et femelles ou bien des plantes produisant des fleurs mâles et hermaphrodites.

Une plante surexprimant la protéine CmWIP1 produit des fleurs mâles et femelles dans les modes de réalisation de la combinaison de deux éléments génétiques de l'invention dans lesquels la protéine ACS est également surexprimée (allèle A présent à l'état homozygote ou hétérozygote).

Une plante surexprimant la protéine CmWIP1 produit des fleurs mâles et hermaphrodites dans les modes de réalisation de la combinaison de deux éléments génétiques de l'invention dans il y a absence d'expression d'une protéine ACS active.

L'invention est donc également relative à une plante transformée telle que définie ci-dessus dont les fleurs sont mâles et femelles et à une plante transformée telle que définie ci-dessus dont les fleurs sont mâles et hermaphrodites.

La plante transformée peut contenir une pluralité de copies d'un acide nucléique codant pour la protéine ACS, dans les situations dans lesquelles une surexpression de la protéine ACS est recherchée. Une surexpression de la protéine ACS est recherchée notamment lorsque l'on souhaite obtenir des plantes produisant des fleurs femelles, non capables d'auto-pollinisation.

Une plante surexprimant la protéine ACS produit des fleurs mâles et femelles dans les modes de réalisation de la combinaison de deux éléments génétiques de l'invention dans lesquels la protéine CmWIP1 est également surexprimée (allèle G présent à l'état homozygote).

Une plante surexprimant la protéine ACS produit des fleurs femelles dans les modes de réalisation de la combinaison de deux éléments génétiques de l'invention dans il y a absence d'expression d'une protéine CmWIP1 active.

L'invention est donc également relative à une plante transformée telle que définie ci-dessus dont les fleurs sont mâles et femelles et à une plante transformée telle que définie ci-dessus dont les fleurs sont exclusivement femelles.

Les plantes transformées selon l'invention, comprennent toutes au moins le second élément génétique de la combinaison selon l'invention, choisi parmi les acides nucléiques, et les polynucléotides régulateurs définis ci-dessus sous une forme artificiellement introduit dans leur génome.

L'invention concerne aussi toute partie d'une plante transformée telle que définie dans la présente description, telle que la racine, mais aussi les parties aériennes comme la tige, la feuille, la fleur et surtout la graine ou le fruit.

L'invention a encore pour objet une semence ou une graine de plante produit par une plante transformée telle que définie ci-dessus.

Typiquement, une telle semence transformée ou un tel grain transformé comprend une ou plusieurs cellules comprenant dans leur génome une ou plusieurs copies des premiers et seconds éléments génétiques de contrôle tels que définis ci-dessus, artificiellement introduits dans ladite plante dicotylédone permettant la synthèse de la protéine CmWIP1 à un taux élevé ou à un taux faible, le cas échéant de manière contrôlée et inductible.

Selon un mode de réalisation préférentiel d'une plante transformée selon l'invention, on cherche à exprimer de manière contrôlée la protéine CmWIP1, ce qui implique que la plante transformée ne contient, en tant que copie fonctionnelle d'un polynucléotide codant pour la protéine CmWIPI, uniquement la ou les copies qui ont été artificiellement introduites dans leurs cellules, et préférentiellement dans leur génome, alors que les séquences du gène (G/g) codant pour CmWIP1, retrouvées naturellement dans la plante sauvage portent au moins une mutation provoquant un défaut dans l'expression du gène (G/g).

Les plantes transformées selon l'invention sont des dicotylédones, de préférence appartenant à la famille *cucurbitaceae,* et en particulier aux genres choisis parmi :

*Abobra, Acanthosicyos, Actinostemma, Alsomitra, Ampelosicyos, Anacaona, Apat3ingania, Apodanthera, Bambekea, Benincasa, Biswarea, Bolbostemma, Brandegea, bryonia, Calycophysum, Cayaponia, Cephalopentandra, Ceratosanthes, Chalema, Cionosicyos, Citrullus, Coccinia, Cogniauxia, Corallocarpus, Cremastopus, Ctenolepis, Cucumella, Cucumeropsis, Cucumis, Cucurbita,, Cucurbitella, Cyclanthera, Cyclantheropsis, Dactyliandra, Dendrosicyos, Dicoelospermum, Dieterlea, Diplocyclos, Doyerea, Ecballium, Echinocystis, Echinopepon, Edgaria, Elateriopsis, Eureiandra, Fevillea, Gerrardanthus, Gomphogyne, Gurania, Guraniopsis, Gymnopetalum, Gynostemma, Halosicyos, Hanburia, Helmontia, Hemsleya, Herpetospermum, Hodgsonia, Ibervillea, Indofevillea, Kedrostis, Lagenaria,, Lemurosicyos, Luffa, Marah, Melancium, Melothria, Melothrianthus, Microsechium, Momordica, Muellerargia, Mukia, Myrmecosicyos, Neoalsomitra, Nothoalsomitra, Odosicyos, Oreosyce, Parasicyos, Penelopeia, Peponium, Peponopsis, Polyclathra, Posadaea, Praecitrullus, Pseudocyclanthera, Pseudosicydium, Psiguria, Pteropepon, Pterosicyos, Raphidiocystis, Ruthalicia, Rytidostylis, Schi3ocarpum, Schi3opepon, Sechiopsis, Sechium,, Selysia, Seyrigia, Sicana, Sicydium, Sicyos, Sicyosperma, Siolmatra, Siraitia, Solena, Tecunumania, Telfairia, Thladiantha, Trichosanthes, Tricyclandra, Trochomeria, Trochomeriopsis, Tumamoca, Vaseyanthus, Wilbrandia, Xerosicyos, 3anonia, 3ehneria, 3ombitsia, ou 3ygosicyos.*

De préférence, les plantes transformées appartiennent au genre *cucumis,* et à l'espèce *Cucumis Melo.*

## Procédés de détection selon l'invention

L'identification du système de contrôle du développement du type floral par les inventeurs, a permis de mettre au point des procédés de détection du phénotype sexuel des plantes extrêmement simples, dont les principales caractéristiques sont détaillées ci-après.

L'invention concerne aussi un procédé de détection de la présence d'un allèle (G) ou (g), ladite méthode comprenant les étapes de :

1) mettre en contact une sonde ou une pluralité de sondes nucléotidiques telles que définies ci-dessus avec l'échantillon à tester ; et
2) détecter le complexe éventuellement formé entre la ou les sondes et l'acide nucléique présent dans l'échantillon.

Le procédé de détection ci-dessus peut-être mis en oeuvre conjointement avec le procédé de détection de la présence d'un allèle (A) ou (a) décrit dans la demande PCT n° WO 2007/125264, qui comprend les étapes de :

1) mettre en contact une sonde ou une pluralité de sondes nucléotidiques telles que définies ci-dessus avec l'échantillon à tester ; et
2) détecter le complexe éventuellement formé entre la ou les sondes et l'acide nucléique présent dans l'échantillon.

Ces deux procédés de détection permettent de sélectionner des plantes dont les phénotypes et génotypes ont été résumés dans le tableau 1.

La détection du complexe entre un acide nucléique et une sonde peut se faire par toute technique connue de l'homme du métier, et en particulier en utilisant des sondes ou amorces marquées, telles que décrites dans la partie « Sondes et amorces selon l'invention ».

Ces procédés sont particulièrement avantageux car ils évitent de devoir cultiver une plante dicotylédone, pour connaître son phénotype sexuel. Il est ainsi possible de réaliser la détection du phénotype sexuel d'un très grand échantillon de plantes, à moindre coût.

**Procédés de sélection selon l'invention**

Les procédés de détection ci-dessus peuvent être mis en œuvre dans les procédés de sélection détaillés ci-après.

L'invention a également pour objet un procédé de sélection du type floral d'une plante appartenant au genre *cucurbitaceae,* caractérisé en ce qu'il comprend les étapes de :

a) déterminer la présence des allèles (G) et (g), dans une plante d'intérêt appartenant à la famille des *cucurbitaceae,* par exemple en utilisant les acides nucléiques tels que définis ci-dessus, et
b) sélectionner positivement la plante qui possède l'allèle (G) ou l'allèle (g) dans son génome.

Le procédé de sélection ci-dessus peut être mis en oeuvre conjointement avec le procédé de sélection du type floral d'une plante appartenant au genre *cucurbitaceae* qui est décrit dans la demande PCT n° WO 2007/125264, et qui est caractérisé en ce qu'il comprend les étapes de :

a) déterminer la présence des allèles (A) et (a), dans une plante d'intérêt appartenant à la famille des *cucurbitaceae,* par exemple en utilisant les acides nucléiques tels que définis ci-dessus, ou un anticorps dirigé contre la protéine ACS et
b) sélectionner positivement la plante qui possède l'allèle (A) ou l'allèle (a) dans son génome.

La détermination de la présence des allèles (A), (a), (G) et (g) peut donc être avantageusement effectuée en mettant en œuvre les procédés de détection ci-dessus.

L'homme du métier peut aisément combiner les procédés de sélection définis ci-dessus, en se référant au tableau 1, donnant la correspondance entre génotype et phénotype, pour obtenir des plantes de phénotype uniquement femelle ou hermaphrodites, par exemple.

**Procédés d'obtention d'une plante transformée selon l'invention**

Les plantes transformées selon l'invention peuvent être obtenues grâce à l'un quelconque des nombreux procédés qui font aujourd'hui partie des connaissances générales de l'homme du métier.

Des procédés d'obtention de plantes transformées selon l'invention sont décrits ci-après.

*Procédé d'obtention de plantes transformées par « TILLING »*

Le « TILLING » (pour « Targeted Induced Local Lésions IN Genomes ») est une méthode de génétique inverse qui est basée sur la capacité d'une endonucléase à détecter les mésappariements dans un double brin d'ADN et à réaliser une coupure de l'ADN au niveau des bases non appariées. Cette technique permet de détecter des points de mutation uniques générés par l'exposition des plantes à un composé chimique mutagène. La technique de TILLING permet ainsi l'identification d'une série d'allèles d'un gène donné et est particulièrement bien adaptée à la mise en oeuvre de procédés de criblages à haut débit permettant la sélection, dans des gènes cibles d'intérêt, de mutations induites par mutagénèse chimique.

Le procédé de Tilling est bien connu de l'homme du métier ; elle est décrite notamment par Mc Callum et al. (2000, Plant Physiology, Vol. 123 : 439-442).

Pour les besoins de la présente invention, la technique de Tilling est particulièrement bien adaptée à l'obtention et à la sélection des plantes dans lesquelles on a artificiellement introduit l'allèle (g) du second élément de contrôle génétique (G/g).

Selon l'invention, la technique de Tilling peut être aussi utilisée avec succès pour l'obtention et la sélection de plantes dans lesquelles on a artificiellement introduit l'allèle (a) du premier élément génétique (A/a).

Par définition, au vu de ce qui précède, la technique de Tilling est adaptée à l'obtention et à la sélection des plantes dans lesquelles on a artificiellement introduit à la fois l'allèle (g) du second élément de contrôle génétique (G/g) et l'allèle (a) du premier élément génétique (A/a).

L'obtention de plantes dicotylédones artificiellement mutées dans le gène codant la protéine CmWIP1 peut être réalisée en utilisant la technique de Tilling, par exemple selon un procédé comprenant les étapes suivantes :

a) générer une collection de plantes dicotylédones mutantes par mutagénèse chimique ;
b) sélectionner dans la collection de plantes mutantes générée à l'étape a), les plantes possédant une mutation ou plus d'une mutation dans le gène codant la protéine CmWIP1,
c) sélectionner, parmi les plantes mutantes sélectionnées à l'étape b), les plantes qui expriment le phénotype (g).

Dans certains modes de réalisation du procédé ci-dessus, l'étape a) est réalisée par mutagénèse chimique des semences des plantes dicotylédone d'intérêt, par exposition des semences à un agent mutagène, par exemple à de l'éthyl méthanesulfonate, par exemple en utilisant la méthode décrite par Koornbeef et al. (1982, Mutat Res, Vol. 93 : 109-123).

Puis, on génère une collection de plantes M1 à partir des semences préalablement exposées à l'agent mutagène. Les plantes M1 sont ensuite auto-fécondées afin de générer une collection de plantes M2 qui est la collection de plantes mutantes générée à l'étape a) du procédé.

Puis, à l'étape b), on extrait l'ADN de chaque plante de la collection de plantes générée à l'étape a) et on réalise une amplification de l'acide nucléique du gène cible, ici le gène codant la protéine CmWIP1, et on recherche la présence de mutation(s) dans la séquence du gène cible, par comparaison avec la séquence du gène cible non muté. Puis, on sélectionne les plantes mutées dans la séquence du gène codant le gène cible, ici le gène codant la protéine CmWIP1.

Dans certains modes de réalisation de l'étape b), l'ADN extrait à partir de plusieurs plantes M2, par exemple 20 plantes M2, est mélangé dans un premier temps et la détection de mutations dans la séquence du gène cible est réalisée sur le mélange (« pool ») d'ADN extrait afin de réduire le nombre d'étapes de détection de mutations qui doit être effectué.

Puis, on réalise une étape d'amplification des séquences cibles par PCR, en utilisant des amorces nucléiques appropriées et les amplicons qui sont générés sont chauffés, puis refroidis afin de générer des hétéroduplexes d'ADN entre l'ADN originaire d'une plante non mutée sur l'acide nucléique cible et l'ADN originaire d'une plante mutée sur l'acide nucléique cible.

Puis, les hétéroduplexes d'ADN sont incubés en présence d'une endonucléase coupant au niveau des mésappariements. Puis les hétéroduplexes clivés sont dénaturés et séparés. Puis les brins d'ADN séparés sont soumis à l'étape de détection de mutation(s) proprement dites, par exemple par électrophorèse ou bien par HPLC en conditions dénaturantes (DHPLC).

Dans certains modes de réalisation de l'étape b), la détection de mutation(s) dans le gène cible est réalisée par la technique de HPLC en conditions dénaturantes (« DHPLC »), comme décrit par exemple par Mc Callum et al. (2000, Plant Physiol., Vol. 123 : 439-442).

Puis, à l'étape c), on sélectionne, parmi les plantes mutées sur le gène cible, ici les plantes mutées dans le gène codant la protéine CmWIP1, les plantes qui expriment le phénotype associé à l'allèle (g) de l'élément génétique (G/g).

Comme déjà mentionné ci-dessus, la technique de Tilling peut-être mise en oeuvre, selon la présente invention, pour l'obtention de plantes transformées comprenant l'une quelconque des combinaisons d'allèles (G/g) et (A/a) décrites dans la présente description.

En conséquence, dans certains modes de réalisation du procédé ci-dessus, on peut sélectionner, à l'étape b), les plantes (i) possédant une mutation ou plus d'une mutation dans le gène codant la protéine CmWIP1 et (ii) possédant une mutation ou plus d'une mutation dans le gène codant la protéine ACS.

Dans ces modes de réalisation particuliers on sélectionne alors à l'étape c), parmi les plantes mutées sur les deux gènes cibles, c'est-à-dire les plantes mutées dans le gène codant la protéine CmWIP1 et dans le gène codant la protéine ACS, les plantes qui expriment à la fois le phénotype associé à l'allèle (g) de l'élément génétique (G/g) et le phénotype associé à l'allèle (a) de l'élément génétique (A/a).

La présente invention a également pour objet des plantes dicotylédones à type floral modifié dans le génome desquelles a été introduite au moins une mutation dans le gène codant la protéine CmWIP1.

La présente invention est aussi relative à des plantes dicotylédones à type floral modifié qui ont été artificiellement mutées dans la séquence du gène codant la protéine CmWIP1, lesdites plantes exprimant le phénotype associé à l'allèle (g) de l'élément génétique (G/g).

La présente invention a également pour objet des plantes dicotylédones à type floral modifié dans le génome desquelles a été introduite (i) au moins une mutation dans le gène codant la protéine CmWIP1 et (ii) au moins une mutation dans le gène codant la protéine ACS.

La présente invention est aussi relative à des plantes dicotylédones à type floral modifié qui ont été artificiellement mutées (i) dans la séquence du gène codant la protéine CmWIP1 et (ii) dans la séquence du gène codant la protéine ACS, lesdites plantes exprimant à la fois (i) le phénotype associé à l'allèle (g) de l'élément génétique (G/g) et le phénotype associé à l'allèle (a) de l'élément génétique (G/g).

*Autres procédés d'obtention de plantes transformées*

L'invention concerne tout d'abord un procédé pour l'obtention d'une plante transformée visant à insérer l'allèle (G) dans une plante ne comprenant pas cet allèle.

L'invention a ainsi pour objet un procédé pour l'obtention d'une plante transformée, appartenant à la famille *cucurbitaceae*, portant des fleurs femelles, caractérisé en ce qu'il comporte les étapes suivantes :

a) transformation d'au moins une cellule végétale d'une plante d'intérêt ne portant pas l'allèle (G), dans son génome, par une séquence nucléotidique (NG); ou un vecteur recombinant comprenant un tel acide nucléique ;
b) sélection des cellules transformées obtenues à l'étape a) ayant intégré dans leur génome, l'acide nucléique (NG) ;
c) Régénération d'une plante transformée à partir des cellules transformées obtenues à l'étape b).

Ce type de procédé est particulièrement utile en ce qu'il permet de d'insérer l'allèle (G) dans le génome d'une plante, qui présentera ainsi un phénotype monoïque ou andromonoïque.

L'invention a également pour objet un procédé de transformation de plantes visant à supprimer l'allèle (G) dans une plante, ou a remplacer l'allèle (G) par un allèle (g) de sorte à obtenir une plante de phénotype hermaphrodite ou une plante de type femelle.

L'invention a donc pour objet un procédé pour l'obtention d'une plante transformée, appartenant à la famille *cucurbitaceae*, portant des fleurs hermaphrodites ou des fleurs femelles, caractérisé en ce qu'il comporte les étapes suivantes :

a) le remplacement dans une plante, de l'allèle (G), par un allèle (g),

La présente invention a également pour objet des plantes dicotylédones à type floral modifié dans le génome desquelles a été introduite au moins une mutation dans le gène codant la protéine CmWIP1.

La présente invention est aussi relative à des plantes dicotylédones à type floral modifié qui ont été artificiellement mutées dans la séquence du gène codant la protéine CmWIP1, lesdites plantes exprimant le phénotype associé à l'allèle (g) de l'élément génétique (G/g).

La présente invention a également pour objet des plantes dicotylédones à type floral modifié dans le génome desquelles a été introduite (i) au moins une mutation dans le gène codant la protéine CmWIP1 et (ii) au moins une mutation dans le gène codant la protéine ACS.

La présente invention est aussi relative à des plantes dicotylédones à type floral modifié qui ont été artificiellement mutées (i) dans la séquence du gène codant la protéine CmWIP1 et (ii) dans la séquence du gène codant la protéine ACS, lesdites plantes exprimant à la fois (i) le phénotype associé à l'allèle (g) de l'élément génétique (G/g) et le phénotype associé à l'allèle (a) de l'élément génétique (G/g).

*Autres procédés d'obtention de plantes transformées*

L'invention concerne tout d'abord un procédé pour l'obtention d'une plante transformée visant à insérer l'allèle (G) dans une plante ne comprenant pas cet allèle.

L'invention a ainsi pour objet un procédé pour l'obtention d'une plante transformée, appartenant à la famille *cucurbitaceae*, portant des fleurs femelles, caractérisé en ce qu'il comporte les étapes suivantes :

a) transformation d'au moins une cellule végétale d'une plante d'intérêt ne portant pas l'allèle (G), dans son génome, par une séquence nucléotidique (NG); ou un vecteur recombinant comprenant un tel acide nucléique ;
b) sélection des cellules transformées obtenues à l'étape a) ayant intégré dans leur génome, l'acide nucléique (NG) ;
c) Régénération d'une plante transformée à partir des cellules transformées obtenues à l'étape b).

Ce type de procédé est particulièrement utile en ce qu'il permet de d'insérer l'allèle (G) dans le génome d'une plante, qui présentera ainsi un phénotype monoïque ou andromonoïque.

L'invention a également pour objet un procédé de transformation de plantes visant à supprimer l'allèle (G) dans une plante, ou a remplacer l'allèle (G) par un allèle (g) de sorte à obtenir une plante de phénotype hermaphrodite ou une plante de type femelle.

L'invention a donc pour objet un procédé pour l'obtention d'une plante transformée, appartenant à la famille *cucurbitaceae*, portant des fleurs hermaphrodites ou des fleurs femelles, caractérisé en ce qu'il comporte les étapes suivantes :

a) le remplacement dans une plante, de l'allèle (G), par un allèle (g),
b) sélection des cellules transformées obtenues à l'étape a) ayant intégré dans leur génome l'allèle (g).
c) Régénération d'une plante transformée à partir des cellules transformées obtenues à l'étape b).

Sans que cela ne fasse partie de l'invention, le procédé peut comprendre encore une étape d) de croisement de plantes obtenues à l'étape c) pour l'obtention d'une ne portant plus d'allèle (G).
Dans un premier mode de réalisation, du procédé ci-dessus, l'étape a) consiste à transformer une plante comportant l'allèle (G) dans son génome, par un acide nucléique de type « antisens » tel que défini ci-dessus, et en sélectionnant les plantes ne comportant plus l'allèle (G).
Un résultat identique peut être obtenu en mettant en œuvre des phénomènes de recombinaison homologue visant à remplacer tout ou partie de l'acide nucléique (NG) par un acide nucléique de structure altérée, qui ne permet pas d'obtenir un phénotype correspondant à l'allèle (G).
Un tel acide nucléique de structure altérée, peut consister en un polynucléotide régulateur (Pg), ou un acide nucléique codant pour une protéine CmWIP1 altérée dans sa séquence.
L'invention a ainsi pour objet un procédé pour l'obtention d'une plante transformée, appartenant à la famille *cucurbitaceae*, portant des fleurs hermaphrodites, caractérisé en ce qu'il comporte les étapes suivantes :

a) transformation d'au moins une cellule végétale d'une plante d'intérêt comportant un allèle (G) par un polynucléotide régulateur (Pg) ou par un acide nucléique codant pour une protéine CmWIP1 altérée; ou un vecteur recombinant comprenant un tel acide nucléique ;
b) sélection des cellules transformées obtenues à l'étape a) ayant intégré dans leur génome au moins une copie d'un polynucléotide régulateur (Pg) ou un acide nucléique codant pour une protéine CmWIP1 altérée ;
c) Régénération d'une plante transformée à partir des cellules transformées obtenues à l'étape b).

Sans que cela ne fasse partie de l'invention, le procédé peut comprendre encore une étape d) de croisement de plantes obtenues à l'étape c) pour l'obtention d'une ne portant plus d'allèle (G).
Ce type de procédé est particulièrement utile en ce qu'il permet d'obtenir des plantes ne comprenant plus d'allèle (G), et qui sont de type hermaphrodite ou gynoïque.
Le procédé ci-dessus peut être mis en oeuvre avec le procédé de transformation de plantes visant à insérer l'allèle (A) qui est décrit dans la demande PCT n° WO 2007/125364.
Ce procédé antérieur pour l'obtention d'une plante transformée, appartenant à la famille *cucurbitaceae*, portant des fleurs femelles, caractérisé en ce qu'il comporte les étapes suivantes :

a) transformation d'au moins une cellule végétale d'une plante d'intérêt ne comprenant pas l'allèle (A), dans son génome, par une séquence nucléotidique (NA); ou un vecteur recombinant comprenant un tel acide nucléique ;
b) sélection des cellules transformées obtenues à l'étape a) ayant intégré dans leur l'acide nucléique (NA) ;
c) Régénération d'une plante transformée à partir des cellules transformées obtenues à l'étape b).

L'invention concerne également un procédé de transformation de plantes visant à remplacer l'allèle (A) par l'allèle (a).
Le procédé ci-dessus peut être mis en oeuvre avec le procédé de transformation de plantes visant à insérer l'allèle (A) qui est décrit dans la demande PCT n° WO 2007/125364.
Ce procédé antérieur pour l'obtention d'une plante transformée, appartenant à la famille *cucurbitaceae*, portant des fleurs hermaphrodites, caractérisé en ce qu'il comporte les étapes suivantes :

a) le remplacement dans une plante, de l'allèle (A), par un allèle (a),
b) sélection des cellules transformées obtenues à l'étape a) ayant intégré dans leur génome l'allèle (a),
c) régénération d'une plante transformée à partir des cellules transformées obtenues à l'étape b).

Sans que cela ne fasse partie de l'invention, le procédé peut comprendre encore une étape d) de croisement de plantes obtenues à l'étape c) pour l'obtention d'une ne portant plus d'allèle (A).
Les procédés ci-dessus peuvent donc être combinés entre eux en se fondant sur le tableau 1, de sorte à obtenir des plantes exclusivement femelles ou exclusivement hermaphrodites, dont l'intérêt industriel a été discuté ci-dessus.
Pour simplifier les procédés pour l'obtention d'une plante transformée exclusivement femelle ou exclusivement hermaphrodite, il est possible de réaliser une étape préalable dans les procédés définis ci-dessus, au cours de laquelle des mutations des gènes (A/a) et (G/g) présents naturellement dans la plante sont effectuées, par exemple par insertion au hasard du transposon *Mutator* dans une population de plantes de phénotype sauvage, puis en détectant chez les mutants obtenus, ceux d'entre ces mutants qui sont de génotype (aagg), par exemple à l'aide des sondes ou des amorces nucléotidiques décrites dans les exemples.

Selon ce mode de réalisation préférentiel, la plante transformée selon l'invention est caractérisée en ce qu'elle possède un génotype (aagg) et présente des fleurs exclusivement hermaphrodites.

Dans un mode de réalisation des procédés d'obtention d'une plante transformée définis ci-dessus, le polynucléotide (NG) lorsqu'il est utilisé comprend un polynucléotide régulateur (PG) activateur inductible.

Les plantes obtenues selon le procédé ci-dessus portent exclusivement des fleurs hermaphrodites, et sont donc particulièrement intéressantes d'un point de vue industriel, puisqu'elles ne sont capables d'auto pollinisation. Ces plantes peuvent donc être utilisées dans des procédés de création de lignées pures.

## Procédés de transformation des plantes selon l'invention

Les méthodes les plus répandues pour introduire des acides nucléiques dans des cellules végétales peuvent être utilisées dans le cadre de cette invention.

La transformation de cellules végétales peut être réalisée par diverses méthodes telles que, par exemple, le transfert des vecteurs susmentionnés dans les protoplastes végétaux après incubation de ces derniers dans une solution de polyéthylèneglycol en présence de cations divalents (Ca 2+), l'électroporation (Fromm et al. 1985), l'utilisation d'un canon à particules, ou la micro-injection cytoplasmique ou nucléaire (Neuhaus et al, 1987).

Une des méthodes de transformation de cellules végétales pouvant être utilisée dans le cadre de l'invention est l'infection des cellules végétales par un hôte cellulaire bactérien comprenant le vecteur contenant la séquence d'intérêt. L'hôte cellulaire peut être *Agrobacterium tumefaciens* (An *et al.* 1986), ou *A. rhizogenes* (Guerche ***et al.*** 1987).

De manière préférentielle, la transformation des cellules végétales est réalisée par le transfert de la région T du plasmide circulaire extrachromosomique inducteur de tumeurs Ti d'*A. tumefaciens,* en utilisant un système binaire (Watson et al., 1994). Pour ce faire, deux vecteurs sont construits. Dans un de ces vecteurs, la région d'ADN-T a été éliminée par délétion, à l'exception des bords droit et gauche, un gène marqueur étant inséré entre eux pour permettre la sélection dans les cellules de plantes. L'autre partenaire du système binaire est un plasmide Ti auxiliaire, plasmide modifié qui n'a plus d'ADN-T mais contient toujours les gènes de virulence *vir* nécessaires à la transformation de la cellule végétale. Ce plasmide est maintenu dans *Agrobacterium.*

Selon un mode préféré, la méthode décrite par Ishida et al. (1996) peut être appliquée pour la transformation des dicotylédones. Selon un autre protocole, la transformation est réalisée selon la méthode décrite par Finer et al. (1992) utilisant le canon à particules de tungstène ou d'or.

La présente invention est également illustrée, sans pour autant y être limitée, par les exemples suivants.

## Exemples

### Exemple 1 : Identification de l'élément génétique de contrôle (G/g)

Le clonage du locus g basé sur la cartographie a été réalisé par criblage des éléments de recombinaison dans une population de 12 660 plantes ségrégeant pour l'allèle g. Une plante de génération F1 issue du croisement de PI124112 (génotype monoïque A-G-) x Gynadou (génotype gynoïques A-gg) a été rétrocroisée avec Gynadou afin de générer une population de génération F2, aux fins d'analyses.

Le locus g a été localisé dans un intervalle entre les marqueurs M261 et M365 sur le chromosome 4 (voir figure 1A).

Les marqueur M261 et M365 sont ancrés dans quatre clones BAC différents provenant d'une librairie génomique de génotype monoïque, afin de générer une carte physique du locus.

Une analyse des SNP dans cet intervalle a révélé deux recombinants très critiques, ce qui a permis de réduire de manière ultime le locus g à une région de 1,4kb dans le clone BAC monoïque 102 (voir figure 1C). L'annotation de ce clone a permis d'identifier 8 cadres ouverts de lecture (ORFs) dans cette région (voir figure 1B). Aucun polymorphisme significatif n'a été retrouvé dans les 8 cadres ouverts de lecture entourant cet intervalle, lorsque la région correspondante a été séquencée dans le génotype gynoïque portant l'allèle récessif.

Cependant, une insertion de 8kb a été trouvée à l'intérieur de la région de 1,4kb délimitée par le clonage positionnel (figure 1B). Cette insertion correspond au transposon ADN de la famille *hAT,* nomé Gyno-hAT (Seqence N° 14) un groupe très répandu d'éléments transposables (TEs).

Du fait que ce transposon ADN a été retrouvé par clonage positionnel dans l'intervalle le plus court, et du fait que ce transposon correspond au seul polymorphisme significatif entre les génotypes monoïques et gynoïques à ce locus, cet élément transposable (TE) a une très forte probabilité d'être responsable du phénotype de détermination du sexe.

**Exemple 2 : Caractérisation de l'élément génétique de contrôle (G/g)**

Du fait que les éléments transposables (TEs) sont sujets à une inhibition (« silencing ») épigénétique induisant une suppression de la transposition et du réarrangement génomique illégitime, on a examiné le statut de méthylation de l'ADN du transposon au locus g, par amplification PCR sensible à l'endonucléase McrBC.

On a observé que le transposon est hautement méthylé (voir figure 2).

On a analysé l'extension potentielle de la méthylation de l'ADN aux gènes environnant le locus g en réalisant une amplification PCR sensible à l'endonucléase McrBC sur les trois cadres ouverts de lecture (ORFs) les plus proches du transposon.

Cette analyse a révélé que seul le cadre ouvert de lecture référencé ORF3, qui est localisé à une distance inférieure à 1kb du locus g, était spécifiquement méthylé dans le génotype gynoïque portant le transposon (figure 3A). Le cadre ouvert de lecture ORF3 code un facteur de transcription à doigts de zinc C2H2 et est homologue aux membres de la sous famille WIP spécifique des plantes (Sagasser et al., 2002).

En vue d'obtenir une information plus précise sur les profils de méthylation de l'ADN de la séquence génomique *CmWIP1,* on a criblé la totalité du gène *CmWIP1* par PCR quantitative sensible à l'endonucléase McrBC dans les plantes PI124112 (G-) et Gynadou (gg) (voir figure 3B). On a montré que l'ADN génomique de Gynadou était hautement méthylé dans la région promotrice à proximité du site d'initiation de la transcription, en comparaison au génotype monoïque (figure 3B).

La méthylation de l'ADN était aussi plus forte dans le premier exon, l'intron et à proximité de la séquence 3'UTR du génotype gynoïque, mais de manière moindre.

Afin de poursuivre l'étude des profils de méthylation de l'ADN de *CmWIP1,* on a réalisé un séquençage au bisulfite sur la région hautement méthylée du promoteur *CmWIP1.* Le séquençage au bisulfite a confirmé le phénotype d'hyperméthylation des plantes gynoïques.

Bien entendu, la méthylation était toujours plus grande dans le promoteur des plantes portant le transposon au locus g, et on a aussi observé la méthylation dans les résidus cytosine qui étaient complètement non méthylés dans le génotype monoïque (voir figure 3C).

Cependant, de manière intéressante, on a noté une méthylation significative de l'ADN dans le promoteur de PI124112.

Le fait qu'une méthylation significative de l'ADN ait été détectée dans le promoteur *CmWIP1* pourrait expliquer l'extension importante de l'inhibition d'expression (« silencing ») lorsqu'un élément transposable (TE) était inséré à proximité. L'hyperméthylation de *CmWIP1* a été confirmée dans différents fonds génétiques récessifs pour l'allèle g, ce qui renforce la corrélation entre la présence de l'ADN de transposon et l'état de méthylation plus élevé de ce gène (figure 4).

Ces résultats suggèrent fortement que l'hyperméthylation de *CmWIP1,* qui est due à une extension de l'inhibition due au transposon (« silencing »), pourraient être la cause de la détermination du sexe chez les plantes portant l'allèle g.

On a associé la méthylation excessive des résidus cytosine dans la région promotrice à une expression génique réduite, ce qui pourrait conduire à une diminution des niveaux de production de transcrits de *CmWIP1.*

**Exemple 3 : Profils de régulation d'expression de l'élément de contrôle (G/g)**

Afin de tester l'hypothèse selon laquelle l'hyperméthylation de la région promotrice du gène *CmWIP1* pourrait induire une diminution de l'expression de ce gène, on a analysé les profils de régulation de l'ARNm de *CmWIP1.* Les niveaux d'expression de *CmWIP1* ont été déterminés par PCR quantitative durant la détermination du sexe et le développement floral.

Les niveaux relatifs de transcrits ont été comparés chez les fleurs mâles de PI124112 (G-) et chez les fleurs femelles de Gynadou (gg).

De manière générale la détermination du sexe et du développement floral chez les cucurbitacées ont été divisés en 12 stades, allant de l'initiation du méristème floral à l'anthesis (Bai et al., 2004). Les processus du détermination du sexe, c'est-à-dire l'arrêt de l'organe sexuel inapproprié dans le bouton floral bisexuel initial, a lieu aux environs des stades 7 et 8.

Dans le cas présent, on a montré que le gène *CmWIP1* était exprimé fortement dans les bourgeons floraux mâles au stade 6, et que son expression décroît rapidement aux stages ultérieurs (figure 5). Au contraire, dans les bourgeons floraux femelles, l'ARNm de *CmWIP1* a été détecté à un niveau très bas, quelque soit le stade de développement floral. De manière remarquable, l'expression haute et transitoire de *CmWIP1* dans les bourgeons floraux mâles au stage précoce du développement reproductif coïncide avec l'arrêt du développement des carpelles qui a été décrit dans des travaux récents chez les cucurbitacées (Hao et al., 2003 ; Bai et al., 2004).

Afin de déterminer le profil de l'expression spatio-temporelle de l'ARNm de *CmWIP1,* on a réalisé des hydridations *in situ* des fleurs mâles de PI124112 (G-) et des fleurs femelles de Gynadou (gg). L'ARNm de *CmWIP1* a été détecté de manière précoce pendant le développement floral autour du stade 6 chez les bourgeons floraux mâles, ce qui est en accord avec les résultats d'analyse par PCR quantitative.

On a trouvé que la localisation d'ARNm de *CmWIP1* était fortement confinée dans la quatrième spirale des bourgeons floraux mâles. Cette localisation correspond au carpelle primaire qui s'arrêtera de se développer au stade immédiatement ultérieur, chez les fleurs mâles.

L'ARNm de *CmWIP1* n'était pas détectable dans les bourgeons floraux femelles, à aucun des stades de développement. Ces résultats confirment la très faible expression qui était déjà révélée par l'analyse par PCT quantitative chez les fleurs femelles de Gynadou (voir figure 7).

Les niveaux d'expression de *CmWIP1* étaient également très faibles durant les stades de développement liés à la détermination du sexe (jusqu'au stade 6) dans les bourgeons floraux hermaphrodites (aagg) et dans les bourgeons floraux femelles de génotype monoïque (figure 6). En d'autres termes, l'expression de *CmWIP1* au stade précoce du développement floral était toujours faible dans les fleurs qui se développeront en structure femelle mature, dans tous les génotypes. L'ensemble de ces résultats suggère fortement que *CmWIP1* a un rôle dans la détermination du sexe chez le melon, en particulier dans l'arrêt du développement des carpelles chez les bourgeons floraux mâles.

**Exemple 4 : Détermination du type floral sous le contrôle de l'élément (G/g)**

Afin d'approfondir l'étude du rôle de *CmWIP1* dans la détermination du sexe et du développement floral chez le melon, on a développé une approche de génétique inverse afin d'identifier la fonction de cette protéine.

On a utilisé une stratégie utilisant la technique de TILLING (pour « Targeted Induced Local-scale Lesions in Genome ») afin de cribler une population de génotype monoïque (A-G-) mutagénéisé avec l'éthylméthanesulfonate (EMS).

Le criblage de la population a ciblé les deux exons de *CmWIP1.* Selon l'alignement avec les homologues proches, la protéine *CmWIP1* est composée de deux différents domaines, respectivement un domaine N-terminal spécifique et un domaine C-terminal conservé au sein des protéines WIP à doigts de zinc.

Trois mutants dans la séquence codante de *CmWIP1* ayant une substitution unique de base ont été identifiés. Les substitutions nucléotidiques résultent en les modifications d'acides aminés suivants : L77F, P193L, et S306F. Les substitutions d'acides aminés étaient localisées soit dans la partie spécifique N-terminal de *CmWIP1,* ou dans le domaine C-terminal hautement conservé de la protéine.

On a observé les phénotypes floraux dans les plantes mutantes homozygotes M2, en comparaison avec les plantes homozygotes de type sauvage de la même famille EMS.

Les trois allèles mutants ont montré un redéveloppement des carpelles, en comparaison des fleurs mâles de type sauvage (figure 7).

Les mutants P193L, et S306F sont devenus complètement gynoiques, ce qui confirme que CmWIP1 est le gène de la gynoecie. Le mutant L77F est un mutant faible.

Les résultats des exemples montrent que l'épiallèle de *CmWIP1* qui a été trouvé dans les plantes gynoïques est clairement transmissible, et co-ségrège avec le phénotype, puisque ont été observées des relations génotypes-phénotypes dans différents fonds génétiques (voir figure 4) et dans plus de douze mille gamètes par des expériences de clonage positionnel.

De manière intéressante, et en accord avec la régulation épigénétique, on a observé occasionnellement des phénotypes révertants durant le développement somatique, qui étaient corrélés avec la déméthylation de la séquence d'ADN de *CmWIP1.*

Les résultats des exemples, qui démontrent l'implication de la régulation du gène *CmWIP1* dans la détermination du sexe floral, sont d'autant plus intéressants que des gènes codant des protéines à doigts de zinc de type WIP sont retrouvés dans une large variété de plantes, y compris les monocotylédones, les gymnospermes et les mousses. De plus, on rappelle que la conservation des domaines C-terminaux des protéines à doigts de zinc de type WIP est très grande.

En conséquence, la réalisation d'un contrôle de régulation de *CmWIP1,* ou de gènes de la même famille dans les génomes de plantes dicotylédones, monocotylédones, gymnospermes et mousses, au niveau transcriptionnel ou post-transcriptionnel durant le développement floral précoce, est de nature à orienter de manière contrôlée le développement dy type floral chez les plantes.

**Exemple 5 : Expression spatiale et temporelle de l'élément génétique de contrôle (A/a)**

Pour étudier l'expression de l'élément génétique de contrôle *A/a* sous la forme de l'allèle *A,* des hybridations *in situ* ont été effectuées en utilisant des sondes spécifiques de l'allèle *A* sur des plantes, et plus précisément sur des méristèmes floraux de plantes mâles, femelles et hermaphrodites, de génotype *AA GG*, *aa GG*, *AA gg* et *aa gg.* Dans les méristèmes floraux *A* l'expression est localement forte et le signal d'hybridation est détecté de manière spécifique dans les primordia des carpelles des fleurs femelles et hermaphrodites des plantes monoïques, andromonoïques, gynoïques et hermaphrodites. En se referant aux différents stades de développement de la fleur décrit pour le concombre (Bai et al., 2004), il apparaît que chez le melon le gène (*A*/*a*) est exprimé à un stade précoce du développement des méristèmes floraux avant qu'une distinction morphologique puisse être effectuée entre les fleurs mâles et les fleurs femelles.

Dans les fleurs mâles et hermaphrodites, aucune expression n'est détectée dans les anthères. Ces données indiquent que l'expression de l'allèle (*A*) dans les carpelles des fleurs femelles empêche le développement des étamines. Du fait que, dans les fleurs hermaphrodites, l'allèle récessif (*a*) présente le même profil d'expression que l'allèle *A* dans les fleurs femelles, on peut conclure que la fonction du gène *A* depend de sa tissu-spécificité d'expression ainsi que de la nature de la protéine ACS synthétisée.

**Exemple 6 : Transgénèse chez Arabidopsis thaliana**

Les effets potentiels du gène A/a, et de la protéine ACS sur le phénotype sexuel floral et l'architecture de la fleur de plantes n'appartenant pas aux cucurbitacées ont été étudiés par transformation *d'Arabidopsis thaliana* par *Agrobacterium.* Les plantes transgéniques d'*Arabidopsis* portantnt l'allèle de melon *A* ou *a* présentent un phenotype au niveau de l' architecture florale et des siliques. (figures 9A et 9B). En effet les siliques des transformants d'*Arabidopsis* sont plus courtes que celles d'une plante Arabidopsis sauvage et l'architecture des fleurs des transformants d'Arabidopsis est tres affectée. Ces résultats permettent d'étendre l'utilisation du gène de melon (A/a) à des plantes dicotylédones n'appartement pas à la famille des cucurbitacées.

En résumé, grâce au clonage du gène A (nommé CmACS-7) et du gène G (nommé CmWIP1) on a démontré que l'expression de la protéine CmWIP1 dans le carpelle inhibe le développement du carpelle et l'expression de CmACS-7 ou toute autre enzyme capable de produire de l'éthylène dans le carpelle inhibe le développement des étamines. Un homme de l'art peut donc exprimer la protéine CmWIP1 dans le carpelle d'une plante, préférablement une cucurbitaceae, pour bloquer le développement du carpelle ou au contraire inhibé l'expression de la protéine CmWIP1 dans le carpelle pour favorisé le développement du carpelle.

Un homme de l'art peut aussi exprimer la protéine CmACS-7 dans le carpelle d'une plante, préférablement une cucurbitaceae pour bloquer le développement des étamines ou au contraire inhiber l'expression de la protéine CmACS-7 dans le carpelle pour favoriser le développement des étamines. Ainsi la combinaison de l'expression des protéines active ou mutantes de CmACS-7 et CmWIP1 va permettre de générer les différents types floraux décris dans le TABLEAU 1.

Les auteurs ont aussi identifié les orthologues de CmACS-7 et CmWIP1 chez d'autres espèces. Chez le concombre (Cucumis sativa) le locus M code pour l'othologue de CmACS-7.

**Tableau des Séquences**

| SEQ ID | Type | Désignation |
|---|---|---|
| 1 | Acide Nucléique | Séquence génomique codant ACS |
| 2 | Acide Nucléique | Séquence génomique « a » avec polynucléotide régulateur |
| 3 | Peptide | Protéine ACS |
| 4 | Acide Nucléique | Amorce-marqueur M8 |
| 5 | Acide Nucléique | Amorce-marqueur M30 |
| 6 | Acide Nucléique | Sonde pour amplicon M8/M30 |
| 7 | Acide Nucléique | Sonde pour SED ID N° 1 |
| 8 | Acide Nucléique | Sonde pour SEQ ID N°2 |
| 9 | Acide Nucléique | Vecteur PEC2 avec gène A |
| 10 | Acide Nucléique | Séquence génomique codant CmWIP1 |
| 11 | Acide Nucléique | ADNc codant CmWIP1 |
| 12 | peptide | Protéine CmWIP1 |
| 13 | Acide Nucléique | Promoteur de CmWIP1 |
| 14 | Acide Nucléique | Transposon Gyno-hAT |

(suite)

| SEQ ID | Type | Désignation |
|---|---|---|
| 15 | Acide Nucléique | Gène CmWIP1 et le transposon Gyno-hAT inséré acoté |
| 16 | Peptide | Protéine homologue à CmWIP1 fonctionnelle |

## Références

An et al., (1986) Plant Physiol. 81, 86-91
Aoyama T et al., (1997) The Plant Journal, vol.11 (3):605-612.
Ausubel et al., (1997) Current protocols in molecular biology
Beaucage et al. (1981), Tetrahedron Lett., 22:1859-1862.
Berbal, 1984 .
Bevan et al., Nucleic Acids Research, vol.12:8711-8721.
Brown et al. (1979), Methods Enzymol., 68:109-151.
Causse et al. (1995) Molecular Breeding 1: 259-272.
Christensen et al. (1996), Transgenic. Res., 5:213
Finer et al. (1992) Plant Cell Report, 11, 323-328
FROMM M. et al. (1990), Biotechnology, 8:833-839
GAIT (ed.), (1984). Nucleic Acid Hybridization.
GORLACH J, VOLRATH S, KNAUF-BEITER G, HENGY G, BECKHOVE U, KOGEL KH, OOSTENDORP M, STAUB T, WARD E, KESSMANN H, RYALS J. (1996) Benzothiadiazole, a novel class of inducers of systemic acquired resistance, activates gene expression and disease resistance in wheat. Plant Cell 8:629-43
GLOVER (ed.), 1985. DNA Cloning: A Practical Approach, Volumes I and II Oligonucleotide Synthesis, MRL Press, Ltd., Oxford, U.K.
Guerche et al. (1987), Mol Gen. Genet 206, 382
HAMES and HIGGINS, 1985. Nucleic Acid Hybridization: a practical approach, Hames & Higgins Ed. IRL Press, Oxford.
HAJDUKIEWICZ, P. SVAB. Z. AND MALIGA P. Plant Mol. Biol. 25 (6), 989-994 (1994).
Ishida et al. (1996) Nature biotechnology 14, 745-750
JEFFERSON, 1987, Plant Molecular Biology Reporter, vol.5:387-405.
Kay et al., (1987) Science 236, 4805
Kahana, A., Silberstein, L., Kessler, N., Goldstein, R. S. and Perl-Treves, R. (2000) expression of ACC oxidase genes differs among sex genotypes and sex phases in cucumber. Plant Mol Biol. Nov ; 41 (4): 517-528.
Kamachi, S., Sekimoto, H., Kondo, N. & Sakai, S., (1997). Cloning of a cDNA for a 1-aminocyclopropane-1-carboxylate synthase that is expressed durig development of female flowers at the apices of Cucumis sativus L. The Plant Cell Physiol., 38:1197-206.
Kohler G and Milstein C;, (1975), Nature, volume 256:495
KOOTER, JM., MATZKE, MA., AND MEYER, P. (1999) Listening to the silent genes: transgene silencing, gene regulation and pathogen control. Trends Plant Sci. 4, 430-437
Kozbor et al., (1983), Hybridoma, vol.2 (1):7-16.
Leger OJ et al., (1997), Hum Antibodies, vol.8 (1):3-16
Martineau P et al., (1998), J. Mol. Biol. vol.280(1):117-127.
Martinez, A., Sparks, C., Hart, CA., tompson, J., and Jepson, I. (1999) Ecdysone agonist inducible transcription in transgenic tobacco plants. Plant J. 19:97-106
McNELLIS T W, 1998, The Plant Journal, vol.14 (2): 247-257
Molina A, Hunt MD, Ryals JA (1998) Impaired fungicide activity in plants blocked in disease resistance signal transduction. Plant Cell 10:1903-14 NARANG et al. (1979), Methods Enzymol., 68: 90-98.
Neuhaus et al., (1987).Theor. Appl. Genet. 75(1), 30-36
Reinmann KA et al. (1997), Aids Res. Hum retroviruses, vol.13 (11):933-943.
Ridder R. et al., (1995), Biotechnology (NY), vol.13 (3):255-260.
SALTER MG et al., 1998, vol.16 (1): 127-132
Risser, G.and Rode, J.C., 1979. Induction par le nitrate d'argent de fleurs staminées chez des plantes gynoïques de melon (Cucumis melo L.). Annales de l'Amélioration des Plantes, 29:349-352.

Rudich, J., Halevy, A.H. and Kedar, N., 1969. Increase of femaleness of three cucurbits by treatment with Ethrel, an ethylene-releasing compound. Planta, 86:69-76.
Sambrook et al., (2001), Molecular Cloning A-laboratory manual
Sanchez Pescador, (1988), J. Clin. Microbiol., 26 (10):1934-1938.
Urdea et al. (1988), Nucleic Acids Research, 11:4937-4957.
WASSENEGGER, M., AND PÉLISSIER, T. (1998) A model for RNA-mediated gene silencing in higher plants Plant Mol. Biol. 37, 349-362
Watson et al. (1994) ADN recombinant, Ed. De Boek Université, 273-292
YANG F. MOSS LG, PHILIPS GN JR. Nat. Biotechnol. 1996 Oct. 14(10):1246-51.
YANG TT, CHENG L. KAIN SR, Nucleic acids Res. 1996, Novembre 15; 24(22):4592-3.

## Revendications

**1.** Utilisation d'une combinaison de deux éléments génétiques pour le contrôle du développement du type floral d'une plante dicotylédone, ladite combinaison comprenant respectivement :

a) un premier élément génétique de contrôle (A/a) présent dans ladite plante dicotylédone, sous la forme d'un allèle dominant (A), et d'un allèle récessif (a), dans lequel :

- l'allèle dominant (A), consiste en un acide nucléique (NA) permettant l'expression de la protéine ACS (aminocyclopropane carboxylate synthase) de séquence SEQ ID N°3,
- l'allèle récessif (a), se distingue de l'allèle dominant en ce que ledit allèle récessif (a) est non fonctionnel dans ladite plante dicotylédone, et

b) un second élément génétique de contrôle (G/g) présent dans ladite plante dicotylédone, sous la forme d'un allèle dominant (G), et d'un allèle récessif (g), dans lequel :

- l'allèle dominant (G), consiste en un acide nucléique (NG) permettant l'expression de la protéine CmWIPI ("C. melo Zinc Finger Protein") de séquence SEQ ID N°12,
- l'allèle récessif (g), se distingue de l'allèle dominant en ce que ledit allèle récessif (g) est non fonctionnel dans ladite plante dicotylédone,
étant entendu qu'au moins le second élément génétique de contrôle a été artificiellement introduit dans ladite plante dicotylédone.

**2.** Utilisation d'une combinaison de deux éléments génétiques selon la revendication 1, **caractérisée en ce que** :

- l'allèle dominant (A), consistant en un acide nucléique (NA), comprend :

(i) un polynucléotide régulateur (PA) fonctionnel dans une plante dicotylédone, et
(ii) un acide nucléique dont l'expression est régulée par le polynucléotide régulateur (PA), ledit acide nucléique codant pour la protéine ACS (aminocyclopropane carboxylate synthase) de séquence SEQ ID N°3,

- l'allèle récessif (a) non fonctionnel, se distingue de l'allèle dominant par un acide nucléique non fonctionnel et comprend :

(i) un polynucléotide régulateur (Pa) dont la séquence nucléotidique comprend une insertion, une substitution ou une délétion par rapport à l'acide nucléique allant du nucléotide 1 au nucléotide 5907 de la séquence SEQ ID N°1, et conduisant à une expression réduite ou nulle de la protéine ACS de séquence SEQ ID N°3
(ii) un acide nucléique (Na) codant pour une protéine qui diffère de la protéine ACS de séquence SEQ ID N°3 par la substitution, l'insertion ou la délétion d'un ou plusieurs acides aminés et qui ne possède pas l'activité biologique de ladite protéine ACS dans ladite plante dicotylédone, et

- l'allèle dominant (G), consistant en un acide nucléique (NG), comprend :

(i) un polynucléotide régulateur (PG) fonctionnel dans une plante dicotylédone, et
(ii) un acide nucléique dont l'expression est régulée par le polynucléotide régulateur (PG), ledit acide nucléique codant pour l'expression de la protéine CmWIPI (« *C. melo Zinc Finger Protein* ») de séquence SEQ ID N°12,

- l'allèle récessif non fonctionnel (g) se distingue de l'allèle dominant (G) par un acide nucléique non fonctionnel et comprend :

(i) un polynucléotide régulateur (Pg) dont la séquence nucléotidique comprend au moins une mutation, une insertion ou une délétion par rapport à l'acide nucléique de séquence SEQ ID N°13, ledit acide nucléique conduisant à une expression réduite ou nulle de la protéine CmWIPI de séquence SEQ ID N°12, ou
(ii) un acide nucléique (Ng) qui code pour une protéine qui diffère de la protéine CmWIPI de séquence SEQ ID N°12 par la substitution, l'insertion ou la délétion d'un ou plusieurs acides aminés et qui ne possède pas l'activité biologique de ladite protéine CmWIPI dans ladite plante dicotylédone.

3. Utilisation d'une combinaison de deux éléments génétiques selon l'une des revendications 1 et 2, **caractérisée en ce que** l'acide nucléique codant pour la protéine CmWIPI comprend, de l'extrémité 5' vers l'extrémité 3', au moins :

(i) une séquence ayant au moins 95% d'identité avec le polynucléotide allant du nucléotide 3000 au nucléotide 3617 de la séquence SEQ ID N°10, et
(ii) une séquence ayant au moins 95% d'identité avec le polynucléotide allant du nucléotide 5458 au nucléotide 5901 de la séquence SEQ ID N° 10.

4. Utilisation d'une combinaison de deux éléments génétiques selon l'une des revendications 2 et 3, **caractérisée en ce que** le polynucléotide régulateur (PG) comprend la séquence nucléotidique SEQ ID N°11.

5. Acide nucléique comprenant, de l'extrémité 5' vers l'extrémité 3', au moins:

(i) une séquence ayant au moins 98,5% d'identité avec le polynucléotide allant du nucléotide 3000 au nucléotide 3617 de la séquence SEQ ID N°10, et
(ii) une séquence ayant au moins 99,5% d'identité avec le polynucléotide allant du nucléotide 5458 au nucléotide 5901 de la séquence SEQ ID N°10.

6. Acide nucléique sous forme d'allèle (G), tel que défini dans la revendication 5 de séquence SEQ ID N°10.

7. Acide nucléique comprenant une séquence nucléotidique allant du nucléotide 3000 au nucléotide 5901 de la séquence SEQ ID N°10.

8. Acide nucléique comprenant une séquence nucléotidique portant au moins une altération choisie parmi une mutation, une insertion ou une délétion, par rapport à l'acide nucléique allant du nucléotide 1 au nucléotide 2999 de la séquence SEQ ID N°10, ledit acide nucléique altéré conduisant à une expression altérée de la protéine CmWIPI de séquence SEQ ID N°12, lorsqu'il contrôle l'expression de ladite protéine, par rapport à l'expression de la protéine CmWIPI contrôlée par l'acide nucléique allant du nucléotide 1 au nucléotide 2999 de la séquence SEQ ID N°10.

9. Vecteur recombinant comprenant un acide nucléique, destiné à être artificiellement introduit dans une plante dicotylédone, lequel acide nucléique est choisi parmi :

a) un élément génétique de contrôle (G/g),
lequel élément génétique de contrôle (G/g) se présente sous la forme d'un allèle dominant (G) ou d'un allèle récessif (g) :

- l'allèle dominant (G), consistant en un acide nucléique (NG), qui comprend :

(i) un polynucléotide régulateur (PG) fonctionnel dans une plante dicotylédone, et
(ii) un acide nucléique dont l'expression est régulée par le polynucléotide régulateur (PG), ledit acide nucléique codant pour l'expression de la protéine CmWIPI (« *C. melo Zinc Finger Protein* ») de séquence SEQ ID N°12,

- l'allèle récessif non fonctionnel (g) qui se distingue de l'allèle dominant (G) par un acide nucléique non fonctionnel et qui comprend :

(i) un polynucléotide régulateur (Pg) dont la séquence nucléotidique comprend au moins une mutation, une insertion ou une délétion par rapport à l'acide nucléique de séquence SEQ ID N°13, ledit acide nucléique conduisant à une expression réduite ou nulle de la protéine CmWIPI de séquence SEQ ID N°12, ou
(ii) un acide nucléique (Ng) qui code pour une protéine qui diffère de la protéine CmWIPI de séquence SEQ ID N°12 par la substitution, l'insertion ou la délétion d'un ou plusieurs acides aminés et qui ne possède pas l'activité biologique de ladite protéine CmWIPI dans ladite plante dicotylédone,
ou

b) un acide nucléique selon l'une quelconque des revendications 5 à 8.

10. Cellule hôte transformée par un vecteur recombinant selon la revendication 9, laquelle cellule hôte comprend, dans son génome, un acide nucléique artificiellement introduit par ledit vecteur recombinant, lequel acide nucléique est choisi parmi :

a) un élément génétique de contrôle (G/g), lequel élément génétique de contrôle (G/g) se présente sous la forme d'un allèle dominant (G) ou d'un allèle récessif (g) :

- l'allèle dominant (G), consistant en un acide nucléique (NG), qui comprend :

(i) un polynucléotide régulateur (PG) fonctionnel dans une plante dicotylédone, et
(ii) un acide nucléique dont l'expression est régulée par le polynucléotide régulateur (PG), ledit acide nucléique codant pour l'expression de la protéine CmWIPI (« *C.* melo *Zinc Finger Protein* ») de séquence SEQ ID N°12,

- l'allèle récessif non fonctionnel (g) qui se distingue de l'allèle dominant (G) par un acide nucléique non fonctionnel et qui comprend :

(i) un polynucléotide régulateur (Pg) dont la séquence nucléotidique comprend au moins une mutation, une insertion ou une délétion par rapport à l'acide nucléique de séquence SEQ ID N°13, ledit acide nucléique conduisant à une expression réduite ou nulle de la protéine CmWIPI de séquence SEQ ID N°12, ou
(ii) un acide nucléique (Ng) qui code pour une protéine qui diffère de la protéine CmWIPI de séquence SEQ ID N°12 par la substitution, l'insertion ou la délétion d'un ou plusieurs acides aminés et qui ne possède pas l'activité biologique de ladite protéine CmWIPI dans ladite plante dicotylédone,
ou

b) un acide nucléique selon l'une quelconque des revendications 5 à 8.

**11.** Cellule hôte selon la revendication 10, **caractérisée en ce qu'**il s'agit d'une cellule de plante appartenant à la famille *cucurbitaceae,* et de préférence à l'espèce *cucumis melo*.

**12.** Plante appartenant à la famille *cucurbitaceae* transformée par un vecteur recombinant selon la revendication 9, ladite plante comprenant, dans son génome, un acide nucléique artificiellement introduit par ledit vecteur recombinant, lequel acide nucléique est choisi parmi :

a) un élément génétique de contrôle (G/g), lequel élément génétique de contrôle (G/g) se présente sous la forme d'un allèle dominant (G) ou d'un allèle récessif (g) :

- l'allèle dominant (G), consistant en un acide nucléique (NG), qui comprend :

(i) un polynucléotide régulateur (PG) fonctionnel dans une plante dicotylédone, et
(ii) un acide nucléique dont l'expression est régulée par le polynucléotide régulateur (PG), ledit acide nucléique codant pour l'expression de la protéine CmWIPI (« *C. melo Zinc Finger Protein* ») de séquence SEQ ID N°12,

- l'allèle récessif non fonctionnel (g) qui se distingue de l'allèle dominant (G) par un acide nucléique non fonctionnel et qui comprend :

(i) un polynucléotide régulateur (Pg) dont la séquence nucléotidique comprend au moins une mutation, une insertion ou une délétion par rapport à l'acide nucléique de séquence SEQ ID N°13, ledit acide nucléique conduisant à une expression réduite ou nulle de la protéine CmWIPI de séquence SEQ ID N°12, ou
(ii) un acide nucléique (Ng) qui code pour une protéine qui diffère de la protéine CmWIPI de séquence SEQ ID N°12 par la substitution, l'insertion ou la délétion d'un ou plusieurs acides aminés et qui ne possède pas l'activité biologique de ladite protéine CmWIPI dans ladite plante dicotylédone,
ou

b) un acide nucléique selon l'une quelconque des revendications 5 à 8.

**13.** Plante transformée selon la revendication 12, **caractérisée en ce qu'**elle comprend au moins un allèle (G) tel que défini dans la revendication 1.

**14.** Cellule hôte transformée par:

(i) un vecteur recombinant comprenant un acide nucléique choisi parmi :

- un acide nucléique déterminant un allèle A ou a tel que défini dans la revendication 1,
- un acide nucléique comprenant, de l'extrémité 5' vers l'extrémité 3', au moins:

(i) une séquence ayant au moins 95% d'identité avec le polynucléotide allant du nucléotide 5907 au nucléotide 6086 de la séquence SEQ ID N°1,
(ii) une séquence ayant au moins 95% d'identité avec le polynucléotide allant du nucléotide 6181 au nucléotide 6467 de la séquence SEQ ID N°1, et
(iii) une séquence ayant au moins 95% d'identité avec le polynucléotide allant du nucléotide 7046 au nucléotide 7915 de la séquence SEQ ID N°1,

- un acide nucléique sous forme d'allèle (A), tel que défini dans la revendication 2, de séquence SEQ ID N°1 ;
- un acide nucléique sous forme d'allèle (a), tel que défini dans la revendication 2, de séquence SEQ ID N°2,
- un acide nucléique comprenant une séquence nucléotidique allant du nucléotide 1 au nucléotide 5906 de la séquence SEQ ID N°1,
- un acide nucléique comprenant une séquence nucléotidique portant au moins une altération choisie parmi une mutation, une insertion ou une délétion, par rapport à l'acide nucléique allant du nucléotide 1 au nucléotide 5907 de la séquence SEQ ID N°1, ledit acide nucléique altéré conduisant à une expression altérée de la protéine ACS de séquence SEQ ID N°3, lorsqu'il contrôle l'expression de ladite protéine, par rapport à l'expression de la protéine ACS contrôlée par l'acide nucléique allant du nucléotide 1 au nucléotide 5907 de la séquence SEQ ID N°1, et
- un acide nucléique comprenant une séquence allant du nucléotide 1 au nucléotide 3650 de la séquence SEQ ID N°2, et

(ii) un vecteur recombinant selon la revendication 9,
laquelle cellule hôte comprend, dans son génome, un acide nucléique artificiellement introduit par ledit vecteur recombinant.

**15.** Cellule hôte selon la revendication 14, **caractérisée en ce qu'**il s'agit d'une cellule de plante appartenant à la famille *cucurbitaceae,* et de préférence à l'espèce *cucumis melo.*

**16.** Acide nucléique, utilisable en tant que amorce, hybridant spécifiquement avec un acide nucléique selon l'une quelconque des revendications 5 à 8.

**17.** Procédé de détection de la présence d'un allèle (G) ou (g) tel que défini dans la revendication 1, ladite méthode comprenant les étapes suivantes :

1) mettre en contact une sonde ou une pluralité de sondes nucléotidiques hybridant spécifiquement avec un acide nucléique selon l'une quelconque des revendications 5 à 8, avec l'échantillon à tester ;
2) détecter le complexe éventuellement formé entre la ou les sondes et l'acide nucléique présent dans l'échantillon.

**18.** Procédé d'obtention de plantes artificiellement mutées dans le gène codant la protéine CmWIPI comprenant les étapes suivantes :

a) générer une collection de plantes dicotylédones mutantes par mutagénèse chimique ;
b) sélectionner dans la collection de plantes mutantes générée à l'étape a), les plantes possédant une mutation ou plus d'une mutation dans le gène codant la protéine CmWIPI,
c) sélectionner, parmi les plantes mutantes sélectionnées à l'étape b), les plantes qui expriment le phénotype associé à l'allèle (g) de l'élément génétique (G/g) tel que défini dans la revendication 1.

**19.** Procédé selon la revendication 18, **caractérisé en ce que** les plantes sont en outre mutées dans le gène codant la protéine ACS et **en ce que** :

- on sélectionne à l'étape b) les plantes (i) possédant une mutation ou plus d'une mutation dans le gène codant la protéine CmWIPI et (ii) possédant une mutation ou plus d'une mutation dans le gène codant la protéine ACS,
- on sélectionne à l'étape c), parmi les plantes mutées dans le gène codant la protéine CmWIPI et dans le gène codant la protéine ACS, les plantes qui expriment à la fois le phénotype associé à l'allèle (g) de l'élément génétique (G/g) et le phénotype associé à l'allèle (a) de l'élément génétique (A/a).

**20.** Procédé pour l'obtention d'une plante transformée, appartenant à la famille *cucurbitaceae,* **caractérisé en ce qu'**il comporte les étapes suivantes :

a) transformation d'au moins une cellule végétale d'une plante d'intérêt ne comprenant pas l'allèle (G) tel que défini dans la revendication 1, dans son génome, par une séquence nucléotidique (NG) que définie dans la revendication 1, ou un vecteur recombinant comprenant un tel acide nucléique;
b) sélection des cellules transformées obtenues à l'étape a) ayant intégré dans leur l'acide nucléique (NG) ;
c) régénération d'une plante transformée à partir des cellules transformées obtenues à l'étape b).

**21.** Procédé pour l'obtention d'une plante transformée, appartenant à la famille *cucurbitaceae,* **caractérisé en ce qu'**il comporte les étapes suivantes :

a) transformation d'au moins une cellule végétale d'une plante d'intérêt comprenant l'allèle (G), tel que défini dans la revendication 1, dans son génome, par un polynucléotide régulateur (Pg) non fonctionnel ou par un acide nucléique (Ng) codant pour une protéine Cm WIP1 altérée, tels que définis dans la revendication 1, ou par un acide nucléique (NG) antisens,
b) sélection des cellules transformées issues d'une plante obtenue à l'étape a) et ayant intégré dans leur génome au moins une copie d'un polynucléotide régulateur (Pg) non fonctionnel ou d'un acide nucléique (Ng) codant pour une protéine Cm WIP1 altérée, tels que définis dans la revendication 1, ou ne comportant plus l'allèle (G),
c) régénération d'une plante transformée à partir des cellules transformées obtenues à l'étape b).

**Patentansprüche**

**1.** Verwendung einer Kombination von zwei genetischen Elementen zur Kontrolle der Entwicklung des Blütentyps einer zweikeimblättrigen Pflanze, wobei die Kombination jeweils umfasst:

a) ein erstes genetisches Kontrollelement (A/a), das in der zweikeimblättrigen Pflanze in Form eines dominanten Allels (A) und eines rezessiven Allels (a) vorhanden ist, wobei:

- das dominante Allel (A) aus einer Nukleinsäure (NA) besteht, die die Expression des Proteins ACS (Aminocyclopropancarboxylatsynthase) der Sequenz SEQ ID Nr. 3 gestattet,
- das rezessive Allel (a) sich von dem dominanten Allel dadurch unterscheidet, dass das rezessive Allel (a) in der zweikeimblättrigen Pflanze nicht funktionell ist, und

b) ein zweites genetisches Kontrollelement (G/g), das in der zweikeimblättrigen Pflanze in Form eines dominanten Allels (G) und eines rezessiven Allels (g) vorhanden ist, wobei:

- das dominante Allel (G) aus einer Nukleinsäure (NG) besteht, die die Expression des Proteins CmWIPI ("C. melo-Zinkfingerprotein") der Sequenz SEQ ID Nr. 12 gestattet,
- das rezessive Allel (g) sich von dem dominanten Allel dadurch unterscheidet, dass das rezessive Allel (g) in der zweikeimblättrigen Pflanze nicht funktionell ist, wobei selbstverständlich sein sollte, dass mindestens das zweite genetische Kontrollelement künstlich in die zweikeimblättrige Pflanze eingeführt worden ist.

**2.** Verwendung einer Kombination von zwei genetischen Elementen nach Anspruch 1, **dadurch gekennzeichnet, dass**:

- das aus einer Nukleinsäure (NA) bestehende dominante Allel (A) Folgendes umfasst:

(i) ein regulatorisches Polynukleotid (PA), das in einer zweikeimblättrigen Pflanze funktionell ist, und
(ii) eine Nukleinsäure, deren Expression durch das regulatorische Polynukleotid (PA) reguliert wird, wobei diese Nukleinsäure für das Protein ACS (Aminocyclopropancarboxylatsynthase) der Sequenz SEQ ID Nr. 3 codiert,

- das nicht funktionelle rezessive Allel (a) sich von dem dominanten Allel durch eine nicht funktionelle Nukleinsäure unterscheidet und Folgendes umfasst:

(i) ein regulatorisches Polynukleotid (Pa), dessen Nukleotidsequenz eine Insertion, eine Substitution oder eine Deletion in Bezug auf die Nukleinsäure umfasst, die vom Nukleotid 1 bis zum Nukleotid 5907 der Sequenz SEQ ID Nr. 1 reicht, und zu einer verringerten oder nicht vorhandenen Expression des Proteins ACS der Sequenz SEQ ID Nr. 3 führt,
(ii) eine Nukleinsäure (Na), die für ein Protein codiert, das sich von dem Protein ACS der Sequenz SEQ ID Nr. 3 durch Substitution, Insertion oder Deletion einer oder mehrerer Aminosäuren unterscheidet und das nicht die biologische Aktivität des Proteins ACS in der zweikeimblättrigen Pflanze besitzt, und

- das aus einer Nukleinsäure (NG) bestehende dominante Allel (G) Folgendes umfasst:

(i) ein regulatorisches Polynukleotid (PG), das in einer zweikeimblättrigen Pflanze funktionell ist, und
(ii) eine Nukleinsäure, deren Expression durch das regulatorische Polynukleotid (PG) reguliert wird, wobei die Nukleinsäure für die Expression des Proteins CmWIPI ("*C. melo-Zinkfingerprotein*") der Sequenz SEQ ID Nr. 12 codiert,

- das nicht funktionelle rezessive Allel (g) sich von dem dominanten Allel (G) durch eine nicht funktionelle Nukleinsäure unterscheidet und Folgendes umfasst:

(i) ein regulatorisches Polynukleotid (Pg), dessen Nukleotidsequenz mindestens eine Mutation, eine Insertion oder eine Deletion in Bezug auf die Nukleinsäure der Sequenz SEQ ID Nr. 13 umfasst, wobei diese Nukleinsäure zu einer verringerten oder nicht vorhandenen Expression des Proteins CmWIPI der Sequenz SEQ ID Nr. 12 führt, oder
(ii) eine Nukleinsäure (Ng), die für ein Protein codiert, das sich von dem Protein CmWIPI der Sequenz SEQ ID Nr. 12 durch Substitution, Insertion oder Deletion einer oder mehrerer Aminosäuren unterscheidet und das nicht die biologische Aktivität des Proteins CmWIPI in der zweikeimblättrigen Pflanze besitzt.

**3.** Verwendung einer Kombination von zwei genetischen Elementen nach einem der Ansprüche 1 und 2, **dadurch gekennzeichnet, dass** die Nukleinsäure, die für das Protein CmWIPI codiert, vom 5'-Ende zum 3'-Ende mindestens Folgendes umfasst:

(i) eine Sequenz mit mindestens 95% Identität mit dem Polynukleotid, das vom Nukleotid 3000 bis zum Nukleotid 3617 der Sequenz SEQ ID Nr. 10 reicht, und
(ii) eine Sequenz mit mindestens 95% Identität mit dem Polynukleotid, das vom Nukleotid 5458 bis zum Nukleotid 5901 der Sequenz SEQ ID Nr. 10 reicht.

**4.** Verwendung einer Kombination von zwei genetischen Elementen nach einem der Ansprüche 2 und 3, **dadurch gekennzeichnet, dass** das regulatorische Polynukleotid (PG) die Nukleotidsequenz SEQ ID Nr. 11 umfasst.

**5.** Nukleinsäure, umfassend vom 5'-Ende zum 3'-Ende mindestens:

(i) eine Sequenz mit mindestens 98,5% Identität mit dem Polynukleotid, das vom Nukleotid 3000 bis zum Nukleotid 3617 der Sequenz SEQ ID Nr. 10 reicht, und
(ii) eine Sequenz mit mindestens 99,5% Identität mit dem Polynukleotid, das vom Nukleotid 5458 bis zum Nukleotid 5901 der Sequenz SEQ ID Nr. 10 reicht.

**6.** Nukleinsäure in Form des Allels (G) nach Anspruch 5 der Sequenz SEQ ID Nr. 10.

**7.** Nukleinsäure, umfassend eine Nukleotidsequenz, die vom Nukleotid 3000 bis zum Nukleotid 5901 der Sequenz SEQ ID Nr. 10 reicht.

**8.** Nukleinsäure, umfassend eine Nukleotidsequenz, die mindestens eine Veränderung, ausgewählt aus einer Mutation, einer Insertion oder einer Deletion, in Bezug auf die Nukleinsäure trägt, die vom Nukleotid 1 bis zum Nukleotid 2999 der Sequenz SEQ ID Nr. 10 reicht, wobei die veränderte Nukleinsäure zu einer veränderten Expression des Proteins CmWIPI der Sequenz SEQ ID Nr. 12, wenn sie die Expression dieses Proteins kontrolliert, im Vergleich zur Expression des Proteins CmWIPI, die von der Nukleinsäure kontrolliert wird, die vom Nukleotid 1 bis zum Nukleotid 2999 der Sequenz SEQ ID Nr. 10 reicht, führt.

**9.** Rekombinanter Vektor, umfassend eine Nukleinsäure zum künstlichen Einführen in eine zweikeimblättrige Pflanze, wobei die Nukleinsäure aus den folgenden ausgewählt ist:

a) einem genetischen Kontrollelement (G/g),
wobei das genetische Kontrollelement (G/g) in Form von einem dominanten Allel (G) oder einem rezessiven Allel (g) vorliegt:

- dem aus einer Nukleinsäure (NG) bestehenden dominanten Allel (G), das Folgendes umfasst:

(i) ein regulatorisches Polynukleotid (PG), das in einer zweikeimblättrigen Pflanze funktionell ist, und
(ii) eine Nukleinsäure, deren Expression durch das regulatorische Polynukleotid (PG) reguliert wird, wobei diese Nukleinsäure für die Expression des Proteins CmWIPI ("*C. melo-Zinkfingerprotein*") der Sequenz SEQ ID Nr. 12 codiert,

- dem nicht funktionellen rezessiven Allel (g), das sich von dem dominanten Allel (G) durch eine nicht funktionelle Nukleinsäure unterscheidet und Folgendes umfasst:

(i) ein regulatorisches Polynukleotid (Pg), dessen Nukleotidsequenz mindestens eine Mutation, eine Insertion oder eine Deletion in Bezug auf die Nukleinsäure der Sequenz SEQ ID Nr. 13 umfasst, wobei diese Nukleinsäure zu einer verringerten oder nicht vorhandenen Expression des Proteins CmWIPI der Sequenz SEQ ID Nr. 12 führt, oder
(ii) eine Nukleinsäure (Ng), die für ein Protein codiert, das sich von dem Protein CmWIPI der Sequenz SEQ ID Nr. 12 durch Substitution, Insertion oder Deletion einer oder mehrerer Aminosäuren unterscheidet und das nicht die biologische Aktivität des Proteins CmWIPI in der zweikeimblättrigen Pflanze besitzt,
oder

b) einer Nukleinsäure nach einem der Ansprüche 5 bis 8.

**10.** Wirtszelle, transformiert mit einem rekombinanten Vektor nach Anspruch 9, wobei die Wirtszelle in ihrem Genom eine künstlich durch den rekombinanten Vektor eingeführte Nukleinsäure umfasst, wobei die Nukleinsäure aus den folgenden ausgewählt ist:

a) einem genetischen Kontrollelement (G/g),
wobei das genetische Kontrollelement (G/g) in Form von einem dominanten Allel (G) oder einem rezessiven Allel (g) vorliegt:

- dem aus einer Nukleinsäure (NG) bestehenden dominanten Allel (G), das Folgendes umfasst:

(i) ein regulatorisches Polynukleotid (PG), das in einer zweikeimblättrigen Pflanze funktionell ist, und
(ii) eine Nukleinsäure, deren Expression durch das regulatorische Polynukleotid (PG) reguliert wird, wobei die Nukleinsäure für die Expression des Proteins CmWIPI ("*C. melo-Zinkfingerprotein"*) der Sequenz SEQ ID Nr. 12 codiert,

- dem nicht funktionellen rezessiven Allel (g), das sich von dem dominanten Allel (G) durch eine nicht funktionelle Nukleinsäure unterscheidet und Folgendes umfasst:

(i) ein regulatorisches Polynukleotid (Pg), dessen Nukleotidsequenz mindestens eine Mutation, eine Insertion oder eine Deletion in Bezug auf die Nukleinsäure der Sequenz SEQ ID Nr. 13 umfasst, wobei diese Nukleinsäure zu einer verringerten oder nicht vorhandenen Expression des Proteins CmWIPI der Sequenz SEQ ID Nr. 12 führt, oder

(ii) eine Nukleinsäure (Ng), die für ein Protein codiert, das sich von dem Protein CmWIPI der Sequenz SEQ ID Nr. 12 durch Substitution, Insertion oder Deletion einer oder mehrerer Aminosäuren unterscheidet und das nicht die biologische Aktivität des Proteins CmWIPI in der zweikeimblättrigen Pflanze besitzt, oder

b) einer Nukleinsäure nach einem der Ansprüche 5 bis 8.

**11.** Wirtszelle nach Anspruch 10, **dadurch gekennzeichnet, dass** es sich um eine Zelle einer Pflanze aus der Familie *Cucurbitaceae* und vorzugsweise der Spezies *Cucumis melo* handelt.

**12.** Pflanze aus der Familie *Cucurbitaceae,* transformiert mit einem rekombinanten Vektor nach Anspruch 9, wobei die Pflanze in ihrem Genom eine künstlich durch den rekombinanten Vektor eingeführte Nukleinsäure umfasst, wobei die Nukleinsäure aus den folgenden ausgewählt ist:

a) einem genetischen Kontrollelement (G/g),
wobei das genetische Kontrollelement (G/g) in Form von einem dominanten Allel (G) oder einem rezessiven Allel (g) vorliegt:

- dem aus einer Nukleinsäure (NG) bestehenden dominanten Allel (G), das Folgendes umfasst:

(i) ein regulatorisches Polynukleotid (PG), das in einer zweikeimblättrigen Pflanze funktionell ist, und
(ii) eine Nukleinsäure, deren Expression durch das regulatorische Polynukleotid (PG) reguliert wird, wobei die Nukleinsäure für die Expression des Proteins CmWIPI ("*C. melo-Zinkfingerprotein*") der Sequenz SEQ ID Nr. 12 codiert,

- dem nicht funktionellen rezessiven Allel (g), das sich von dem dominanten Allel (G) durch eine nicht funktionelle Nukleinsäure unterscheidet und Folgendes umfasst:

(i) ein regulatorisches Polynukleotid (Pg), dessen Nukleotidsequenz mindestens eine Mutation, eine Insertion oder eine Deletion in Bezug auf die Nukleinsäure der Sequenz SEQ ID Nr. 13 umfasst, wobei diese Nukleinsäure zu einer verringerten oder nicht vorhandenen Expression des Proteins CmWIPI der Sequenz SEQ ID Nr. 12 führt, oder
(ii) eine Nukleinsäure (Ng), die für ein Protein codiert, das sich von dem Protein CmWIPI der Sequenz SEQ ID Nr. 12 durch Substitution, Insertion oder Deletion einer oder mehrerer Aminosäuren unterscheidet und das nicht die biologische Aktivität des Proteins CmWIPI in der zweikeimblättrigen Pflanze besitzt, oder

b) einer Nukleinsäure nach einem der Ansprüche 5 bis 8.

**13.** Transformierte Pflanze nach Anspruch 12, **dadurch gekennzeichnet, dass** sie mindestens ein Allel (G) nach Anspruch 1 umfasst.

**14.** Wirtszelle, transformiert mit:

(i) einem rekombinanten Vektor, umfassend eine Nukleinsäure, ausgewählt aus:

- einer Nukleinsäure, die ein Allel A oder a bestimmt, nach Anspruch 1,
- einer Nukleinsäure, die vom 5'-Ende zum 3'-Ende mindestens Folgendes umfasst:

(i) eine Sequenz mit mindestens 95% Identität mit dem Polynukleotid, das vom Nukleotid 5907 bis zum Nukleotid 6086 der Sequenz SEQ ID Nr. 1 reicht,
(ii) eine Sequenz mit mindestens 95% Identität mit dem Polynukleotid, das vom Nukleotid 6181 bis zum Nukleotid 6467 der Sequenz SEQ ID Nr. 1 reicht, und
(iii) eine Sequenz mit mindestens 95% Identität mit dem Polynukleotid, das vom Nukleotid 7046 bis zum Nukleotid 7915 der Sequenz SEQ ID Nr. 1 reicht,

- einer Nukleinsäure in Form des Allels (A) nach Anspruch 2 der Sequenz SEQ ID Nr. 1,
- einer Nukleinsäure in Form des Allels (a) nach Anspruch 2 der Sequenz SEQ ID Nr. 2,

- einer Nukleinsäure, umfassend eine Nukleotidsequenz, die vom Nukleotid 1 bis zum Nukleotid 5906 der Sequenz SEQ ID Nr. 1 reicht,
- einer Nukleinsäure, umfassend wobei eine Nukleotidsequenz, die mindestens eine Veränderung, ausgewählt aus einer Mutation, einer Insertion oder einer Deletion, in Bezug auf die Nukleinsäure trägt, die vom Nukleotid 1 bis zum Nukleotid 5907 der Sequenz SEQ ID Nr. 1 reicht, wobei die veränderte Nukleinsäure zu einer veränderten Expression des Proteins ACS der Sequenz SEQ ID Nr. 3, wenn sie die Expression dieses Proteins kontrolliert, im Vergleich zur Expression des Proteins ACS, das von der Nukleinsäure kontrolliert wird, die vom Nukleotid 1 bis zum Nukleotid 5907 der Sequenz SEQ ID Nr. 1 reicht, führt, und
- einer Nukleinsäure, umfassend eine Sequenz, die vom Nukleotid 1 bis zum Nukleotid 3650 der Sequenz SEQ ID Nr. 2 reicht, und

(ii) einem rekombinanten Vektor nach Anspruch 9,
wobei die Wirtszelle in ihrem Genom eine künstlich durch den rekombinanten Vektor eingeführte Nukleinsäure umfasst.

**15.** Wirtszelle nach Anspruch 14, **dadurch gekennzeichnet, dass** es sich um eine Zelle einer Pflanze aus der Familie *Cucurbitaceae* und vorzugsweise der Spezies *Cucumis melo* handelt.

**16.** Nukleinsäure, verwendbar als Primer, die spezifisch mit einer Nukleinsäure nach einem der Ansprüche 5 bis 8 hybridisiert.

**17.** Verfahren zum Nachweis des Vorhandenseins eines Allels (G) oder (g) nach Anspruch 1, wobei das Verfahren die folgenden Schritte umfasst:

1) Inkontaktbringen einer Nukleotidsonde oder einer Mehrzahl von Nukleotidsonden, die spezifisch mit einer Nukleinsäure nach einem der Ansprüche 5 bis 8 hybridisiert/hybridisieren, mit der zu testenden Probe;
2) Nachweisen des gegebenenfalls gebildeten Komplexes zwischen der Sonde bzw. den Sonden und der in der Probe vorhandenen Nukleinsäure.

**18.** Verfahren zum Erhalten von Pflanzen, die künstlich in dem für das Protein CmWIPI codierenden Gen mutiert sind, umfassend die folgenden Schritte:

a) Erzeugen einer Sammlung von mutierten zweikeimblättrigen Pflanzen durch chemische Mutagenese;
b) Selektieren, aus der im Schritt a) erzeugten Sammlung von mutierten Pflanzen, derjenigen Pflanzen, die eine Mutation oder mehr als eine Mutation in dem für das Protein CmWIPI codierenden Gen besitzen.
c) Selektieren, unter den im Schritt b) selektierten mutierten Pflanzen, derjenigen Pflanzen, die den Phänotyp exprimieren, der mit dem Allel (g) des genetischen Elements (G/g) nach Anspruch 1 assoziiert ist.

**19.** Verfahren nach Anspruch 18, **dadurch gekennzeichnet, dass** die Pflanzen außerdem in dem für das Protein ACS codierenden Gen mutiert sind, und dadurch, dass:

- man im Schritt b) diejenigen Pflanzen selektiert, die

(i) eine Mutation oder mehr als eine Mutation in dem für das Protein CmWIPI codierenden Gen besitzen und
(ii) eine Mutation oder mehr als eine Mutation in dem für das Protein ACS codierenden Gen besitzen,

- man im Schritt c) unter den Pflanzen, die in dem für das Protein CmWIPI codierenden Gen und in dem für das Protein ACS codierenden Gen mutiert sind, diejenigen Pflanzen selektiert, die sowohl den mit dem Allel (g) des genetischen Elements (G/g) assoziierten Phänotyp als auch den mit dem Allel (a) des genetischen Elements (A/a) assoziierten Phänotyp exprimieren.

**20.** Verfahren zum Erhalten einer transformierten Pflanze aus der Familie *Cucurbitaceae,* **dadurch gekennzeichnet, dass** es die folgenden Schritte umfasst:

a) Transformation mindestens einer Pflanzenzelle einer Pflanze von Interesse, die nicht das Allel (G) nach Anspruch 1 in ihrem Genom umfasst, mit einer Nukleotidsequenz (NG) nach Anspruch 1 oder einem rekombinanten Vektor, umfassend eine solche Nukleinsäure;
b) Selektion der im Schritt a) erhaltenen transformierten Zellen, die (NG) in ihre Nukleinsäure integriert haben;

c) Regeneration einer transformierten Pflanze aus den im Schritt b) erhaltenen transformierten Zellen.

**21.** Verfahren zum Erhalten einer transformierten Pflanze aus der Familie *Cucurbitaceae,* **dadurch gekennzeichnet, dass** es die folgenden Schritte umfasst:

a) Transformation mindestens einer Pflanzenzelle einer Pflanze von Interesse, die das Allel (G) nach Anspruch 1 in ihrem Genom umfasst, mit einem nicht funktionellen regulatorischen Polynukleotid (Pg) oder mit einer Nukleinsäure (Ng), die für ein verändertes Protein CmWIP1 codiert, jeweils nach Anspruch 1, oder mit einer Antisense-Nukleinsäure (NG),
b) Selektion transformierter Zellen, die aus einer im Schritt a) erhaltenen Pflanze stammen und in ihr Genom mindestens eine Kopie eines nicht funktionellen regulatorischen Polynukleotids (Pg) oder einer Nukleinsäure (Ng), die für ein verändertes Protein CmWIP1 codiert, nach Anspruch 1 integriert haben oder die das Allel (G) nicht mehr tragen,
c) Regeneration einer transformierten Pflanze aus den im Schritt b) erhaltenen transformierten Zellen.

## Claims

**1.** Use of a combination of two genetic elements for controlling the development of the floral type of a dicotyledonous plant, said combination comprising respectively:

a) a first genetic control element (A/a) present in said dicotyledonous plant, in the form of a dominant allele (A), and of a recessive allele (a), in which:

- the dominant allele (A) consists of a nucleic acid (NA) permitting expression of the protein ACS (aminocyclopropane carboxylate synthase) of sequence SEQ ID No. 3,
- the recessive allele (a) differs from the dominant allele wherein the said recessive allele (a) is non-functional in said dicotyledonous plant, and

b) a second genetic control element (G/g) present in said dicotyledonous plant, in the form of a dominant allele (G), and of a recessive allele (g), in which:

- the dominant allele (G) consists of a nucleic acid (NG) permitting expression of the protein CmWIPI ("*C. melo Zinc Finger Protein*") of sequence SEQ ID No. 12,
- the recessive allele (g) differs from the dominant allele wherein said recessive allele (g) is non-functional in said dicotyledonous plant,
it being understood that at least the second genetic control element was introduced artificially into said dicotyledonous plant.

**2.** Use of a combination of two genetic elements according to Claim 1, **characterized in that**:

- the dominant allele (A) consists of a nucleic acid (NA) comprising:

(i) a regulatory polynucleotide (PA) that is functional in a dicotyledonous plant, and
(ii) a nucleic acid whose expression is regulated by the regulatory polynucleotide (PA), said nucleic acid coding for the protein ACS (aminocyclopropane carboxylate synthase) of sequence SEQ ID No. 3,

- the recessive allele (a) non-functional differs from the dominant allele by a non-functional nucleic acid and comprises:

(i) a regulatory polynucleotide (Pa) whereof the nucleotide sequence comprises an insertion, a substitution or a deletion, relative to the nucleic acid from nucleotide 1 to nucleotide 5907 of the sequence SEQ ID No. 1, leading to a reduced or suppressed expression of the protein ACS of sequence SEQ ID No. 3,
(ii) a nucleic acid (Na) encoding for a protein which differs from the ACS protein of sequence SEQ ID No. 3 by an insertion, a substitution or a deletion of one or several amino acids et which does not have the biological activity of the protein ACS in the dicotyledonous plant, and

- the dominant allele (G) consists of a nucleic acid (NG) comprising:

(i) a regulatory polynucleotide (PG) that is functional in a dicotyledonous plant, and
(ii) a nucleic acid, expression of which is regulated by the regulatory polynucleotide (PG), said nucleic acid coding for the protein CmWIPI ("C. melo Zinc Finger Protein") of sequence SEQ ID No. 12,

- the recessive allele (g), non functional, differs from the dominant allele (G) by a non-functional nucleic acid and comprises:

(i) a regulatory polynucleotide (Pg) whereof the nucleotide sequence comprises at least an insertion, a mutation or a deletion, relative to the nucleic acid of the sequence SEQ ID No. 13, said nucleic acid leading to a reduced or suppressed expression of the protein CmWIPI of sequence SEQ ID No. 12, or
(ii) a nucleic acid (Ng) encoding for a protein which differs from the CmWIPI protein of sequence SEQ ID No. 12 by an insertion, a substitution or a deletion of one or several amino acids et which does not have the biological activity of the protein CmWIPI in the dicotyledonous plant.

**3.** Use of a combination of two genetic elements according to any of the Claims 1 and 2, **characterized in that** the nucleic acid coding for the protein CmWIPI comprises, from the 5' end to the 3' end, at least:

(i) one sequence having at least 95% identity with the polynucleotide from nucleotide 3000 to nucleotide 3617 of the sequence SEQ ID No. 10, and
(ii) one sequence having at least 95% identity with the polynucleotide from nucleotide 5458 to nucleotide 5901 of the sequence SEQ ID No. 10.

**4.** Use of a combination of two genetic elements according to any of the Claims 2 and 3, **characterized in that** the regulatory polynucleotide (PG) comprises the nucleotide sequence SEQ ID No. 11.

**5.** Nucleic acid comprising, from the 5' end to the 3' end, at least:

(i) one sequence having at least 98.5% identity with the polynucleotide from nucleotide 3000 to nucleotide 3617 of the sequence SEQ ID No. 10, and
(ii) a sequence having at least 99.5% identity with the polynucleotide from nucleotide 5458 to nucleotide 5901 of the sequence SEQ ID No. 10.

**6.** Nucleic acid in the form of the allele (G), as defined in Claim 5 of sequence SEQ ID No. 10.

**7.** Nucleic acid comprising a nucleotide sequence extending from nucleotide 3000 to nucleotide 5901 of the sequence SEQ ID No. 10.

**8.** Nucleic acid comprising a nucleotide sequence bearing at least one alteration selected from a mutation, an insertion or a deletion, relative to the nucleic acid from nucleotide 1 to nucleotide 2999 of the sequence SEQ ID No. 10, said altered nucleic acid leading to altered expression of the protein CmWIPI of sequence SEQ ID No. 12, when it controls the expression of said protein, relative to the expression of the protein CmWIPI controlled by the nucleic acid from nucleotide 1 to nucleotide 2999 of the sequence SEQ ID No. 10.

**9.** Recombinant vector comprising a nucleic acid, intended to be artificially introduced into a dicotyledonous plant, wherein said nucleic acid is chosen among:

a) a genetic control element (G/g),
wherein said genetic control element (G/g) is in the form of a dominant allele (G) or of a recessive allele (g):

- the dominant allele (G) consists of a nucleic acid (NG) which comprises:

(i) a regulatory polynucleotide (PG) that is functional in a dicotyledonous plant, and
(ii) a nucleic acid, expression of which is regulated by the regulatory polynucleotide (PG), said nucleic acid coding for the protein CmWIPI ("C. melo Zinc Finger Protein") of sequence SEQ ID No. 12,

- the recessive allele (g), non functional, differs from the dominant allele (G) by a non functional nucleic acid and comprises:

(i) a regulatory polynucleotide (Pg) whereof the nucleotide sequence comprises at least an insertion, a mutation or a deletion, relative to the nucleic acid of the sequence SEQ ID No. 13, said nucleic acid leading to a reduced or suppressed expression of the protein CmWIPI of sequence SEQ ID No. 12, or
(ii) a nucleic acid (Ng) encoding for a protein which differs from the CmWIPI protein of sequence SEQ ID No. 12 by an insertion, a substitution or a deletion of one or several amino acids and which does not have the biological activity of the protein CmWIPI in the dicotyledonous plant, or

b) a nucleic acid according to any one of Claims 5 to 8.

**10.** Host cell transformed by a recombinant vector according to Claim 9, wherein said host cell comprising, in its genome, an artificially introduced nucleic acid by said recombinant vector, wherein said nucleic acid is chosen among:

a) a genetic control element (G/g),
wherein said genetic control element (G/g) is in the form of a dominant allele (G) or of a recessive allele (g):

- the dominant allele (G) consists of a nucleic acid (NG) which comprises:

(i) a regulatory polynucleotide (PG) that is functional in a dicotyledonous plant, and
(ii) a nucleic acid, expression of which is regulated by the regulatory polynucleotide (PG), said nucleic acid coding for the protein CmWIPI ("C. melo Zinc Finger Protein") of sequence SEQ ID No. 12,

- the recessive allele (g), non functional, differs from the dominant allele (G) by a non functional nucleic acid and comprises:

(i) a regulatory polynucleotide (Pg) whereof the nucleotide sequence comprises at least an insertion, a mutation or a deletion, relative to the nucleic acid of the sequence SEQ ID No. 13, said nucleic acid leading to a reduced or suppressed expression of the protein CmWIPI of sequence SEQ ID No. 12, or
(ii) a nucleic acid (Ng) coding for a protein which differs from the CmWIPI protein of sequence SEQ ID No. 12 by an insertion, a substitution or a deletion of one or several amino acids and which does not have the biological activity of the protein CmWIPI in the dicotyledonous plant, or

b) a nucleic acid according to any one of Claims 5 to 8.

**11.** Host cell according to Claim 10, **characterized in that** it is a cell of a plant belonging to the family *Cucurbitaceae,* and preferably to the species *Cucumis melo.*

**12.** Plant belonging to the family *Cucurbitaceae* transformed by a recombinant vector according to Claim 9, wherein said plant comprising, into its genome, an artificially introduced nucleic acid by said recombinant vector, wherein said nucleic acid is chosen among:

a) a genetic control element (G/g),
wherein said genetic control element (G/g) is in the form of a dominant allele (G) or of a recessive allele (g):

- the dominant allele (G) consists of a nucleic acid (NG) which comprises:

(i) a regulatory polynucleotide (PG) that is functional in a dicotyledonous plant, and
(ii) a nucleic acid, expression of which is regulated by the regulatory polynucleotide (PG), said nucleic acid coding for the protein CmWIPI ("C. melo Zinc Finger Protein") of sequence SEQ ID No. 12,

- the recessive allele (g), non functional, differs from the dominant allele (G) by a non functional nucleic acid and comprises:

(i) a regulatory polynucleotide (Pg) whereof the nucleotide sequence comprises at least an insertion, a mutation or a deletion, relative to the nucleic acid of the sequence SEQ ID No. 13, said nucleic acid leading to a reduced or suppressed expression of the protein CmWIPI of sequence SEQ ID No. 12, or
(ii) a nucleic acid (Ng) coding for a protein which differs from the CmWIPI protein of sequence SEQ ID No. 12 by an insertion, a substitution or a deletion of one or several amino acids and which does not have the biological activity of the protein CmWIPI in the dicotyledonous plant, or

b) a nucleic acid according to any one of Claims 5 to 8.

**13.** Transformed plant according to Claim 12, **characterized in that** it comprises at least one allele (G) as defined in Claim 1.

**14.** Host cell transformed by:

(i) a recombinant vector comprising a nucleic acid selected from:

- a nucleic acid determining an allele A or (a) as defined in one of Claim 1,
- a nucleic acid comprising, from the 5' end to the 3' end, at least:

(i) one sequence having at least 95% identity with the polynucleotide from nucleotide 5907 to nucleotide 6086 of the sequence SEQ ID No. 1,
(ii) one sequence having at least 95% identity with the polynucleotide from nucleotide 6181 to nucleotide 6467 of the sequence SEQ ID No. 1, and
(iii) one sequence having at least 95% identity with the polynucleotide from nucleotide 7046 to nucleotide 7915 of the sequence SEQ ID No. 1,

- a nucleic acid in the form of allele (A), as defined in Claim 2, of sequence SEQ ID No. 1,
- a nucleic acid in the form of the allele (a), as defined in Claim 2, of sequence SEQ ID No. 2,
- a nucleic acid comprising a nucleotide sequence from nucleotide 1 to nucleotide 5906 of the sequence SEQ ID No. 1,
- a nucleic acid comprising a nucleotide sequence bearing at least one alteration selected from a mutation, an insertion or a deletion, relative to the nucleic acid from nucleotide 1 to nucleotide 5907 of the sequence SEQ ID No. 1, said altered nucleic acid leading to altered expression of the protein ACS of sequence SEQ ID No. 3, when it controls the expression of said protein, relative to the expression of the protein ACS controlled by the nucleic acid from nucleotide 1 to nucleotide 5907 of the sequence SEQ ID No. 1, and
- a nucleic acid comprising a sequence extending from nucleotide 1 to nucleotide 3650 of the sequence SEQ ID No. 2, and

(ii) a recombinant vector according to Claim 9,
wherein said host cell comprises, into its genome, a nucleic acid artificially introduced by said recombinant vector.

**15.** Host cell according to Claim 14, **characterized in that** it is a cell of a plant belonging to the family *Cucurbitaceae,* and preferably to the species *Cucumis melo.*

**16.** Nucleic acid, usable as probe or primer, hybridizing specifically to a nucleic acid as defined in any one of Claims 5 to 8.

**17.** Method for detecting the presence of an allele (G) or (g) as defined in Claim 1, said method comprising the stages of:

1) contacting a nucleotide probe or a plurality of nucleotide probes according to Claims 5 to 8 with the sample to be tested;
2) detecting any complex formed between the probe or probes and the nucleic acid present in the sample.

**18.** Method for obtaining plants artificially mutated in the gene encoding the protein CmWIPI comprising the following stages:

a) generating a collection of mutant dicotyledonous plants by chemical mutagenesis;
b) selecting, from the collection of mutant plants generated in stage a), the plants possessing a mutation or more than one mutation in the gene encoding the protein CmWIPI;
c) selecting, from the mutant plants selected in stage b), the plants that express the phenotype associated to allele (g) of the genetic element (G/g) such as defined in claim 1.

**19.** Method according to Claim 18, **characterized in that** the plants are moreover mutated in the gene encoding the protein ACS and **in that**:

- the plants (i) possessing a mutation or more than one mutation in the gene encoding the protein CmWIPI and (ii) possessing a mutation or more than one mutation in the gene encoding the protein ACS are selected in stage b),
- the plants that express both the phenotype associated with the allele (g) of the genetic element (G/g) and the phenotype associated with the allele (a) of the genetic element (A/a) are selected in stage c) from the plants mutated in the gene encoding the protein CmWIPI and in the gene encoding the protein ACS.

**20.** Method for obtaining a transformed plant, belonging to the family *Cucurbitaceae,* **characterized in that** it comprises the following stages:

a) transformation of at least one vegetable cell of a plant of interest not comprising the allele (G) as defined in Claim 1, in its genome, by a nucleotide sequence (NG) as defined in Claim 1, or a recombinant vector comprising such a nucleic acid;
b) selection of the transformed cells obtained in stage a) that has integrated the nucleic acid (NG) into their genome;
c) regeneration of a transformed plant from the transformed cells obtained in stage b).

**21.** Method for obtaining a transformed plant, belonging to the family *Cucurbitaceae,* **characterized in that** it comprises the following stages:

a) transformation of at least one vegetable cell of a plant of interest comprising the allele (G) as defined in Claim 1, in its genome, by a regulatory polynucleotide (Pg) non functional or by a nucleic acid (Ng) encoding for a altered CmWIPI protein as defined in Claim 1, or by a nucleic acid (NG) antisense;
b) selection of the transformed cells issued from a plant obtained in stage a) and that have integrated in their genome at least a copy of the regulatory polynucleotide (Pg) non-functional or the nucleic acid (Ng) encoding for a altered CmWIPI protein as defined in Claim 1, or without anymore allele (G);
c) regeneration of a transformed plant from the transformed cells obtained in stage b).

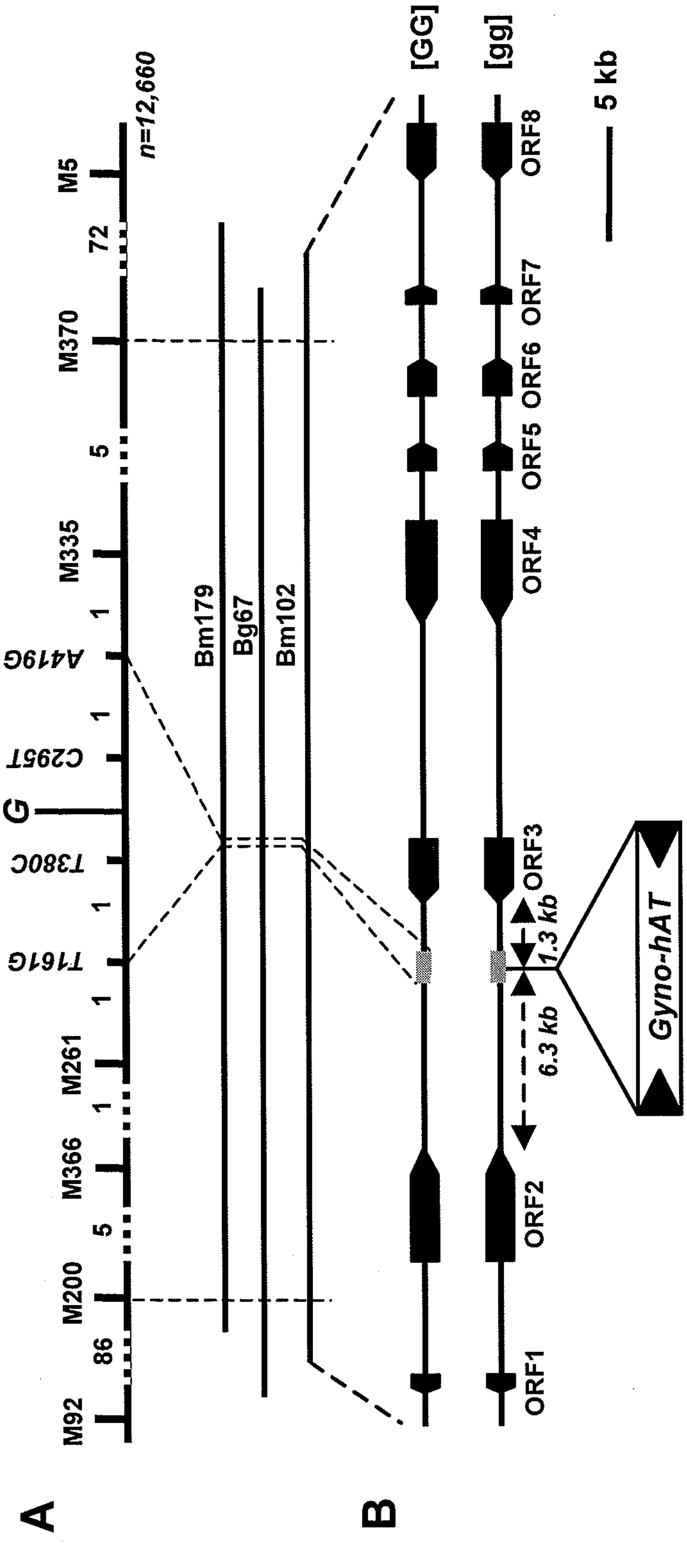


Figures 1A et 1B

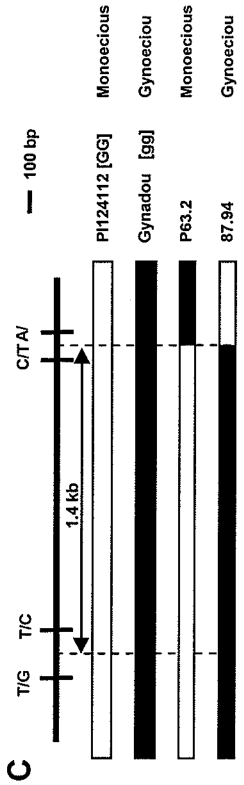
C
T/G
T/C
C/T A/
1.4 kb
100 bp
PI124112 [GG]
Gynadou [gg]
P63.2
87.94
Monoecious
Gynoeciou
Monoecious
Gynoeciou

Figure 1C

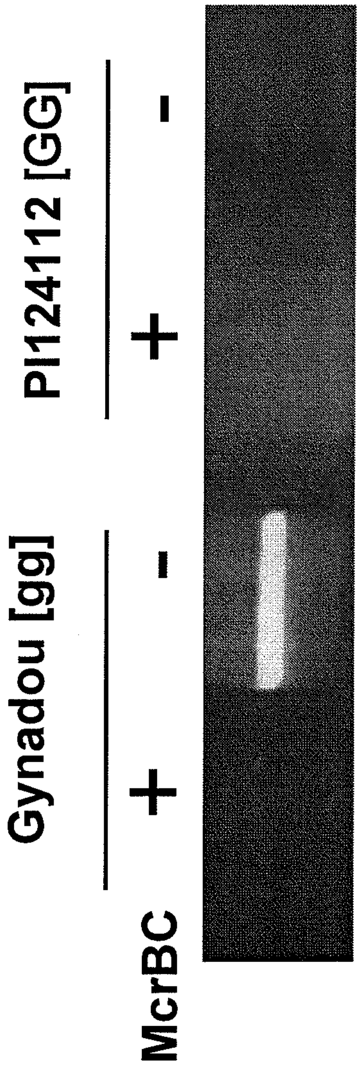


Figure 2

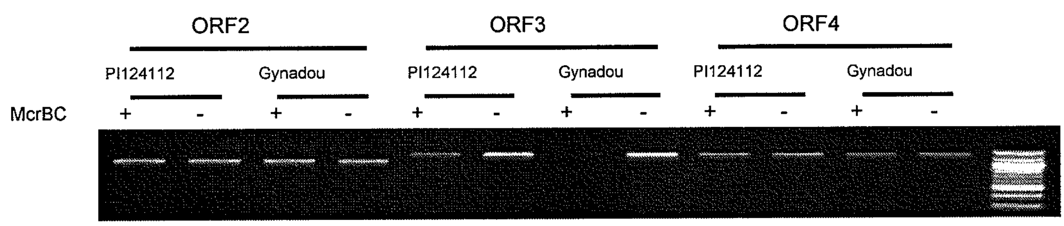


Figure 3A

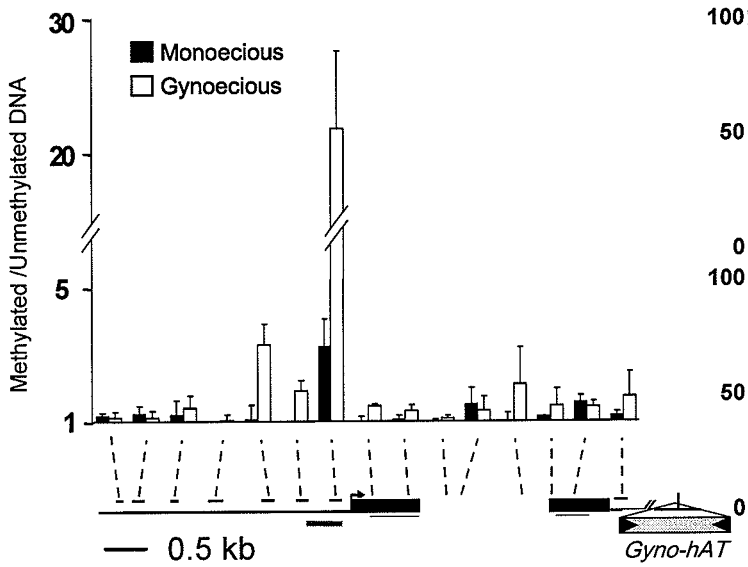


Figure 3B

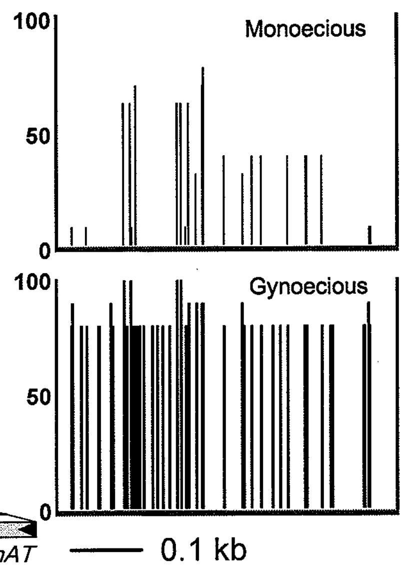


Figure 3C

Figures 3A, 3B, 3C

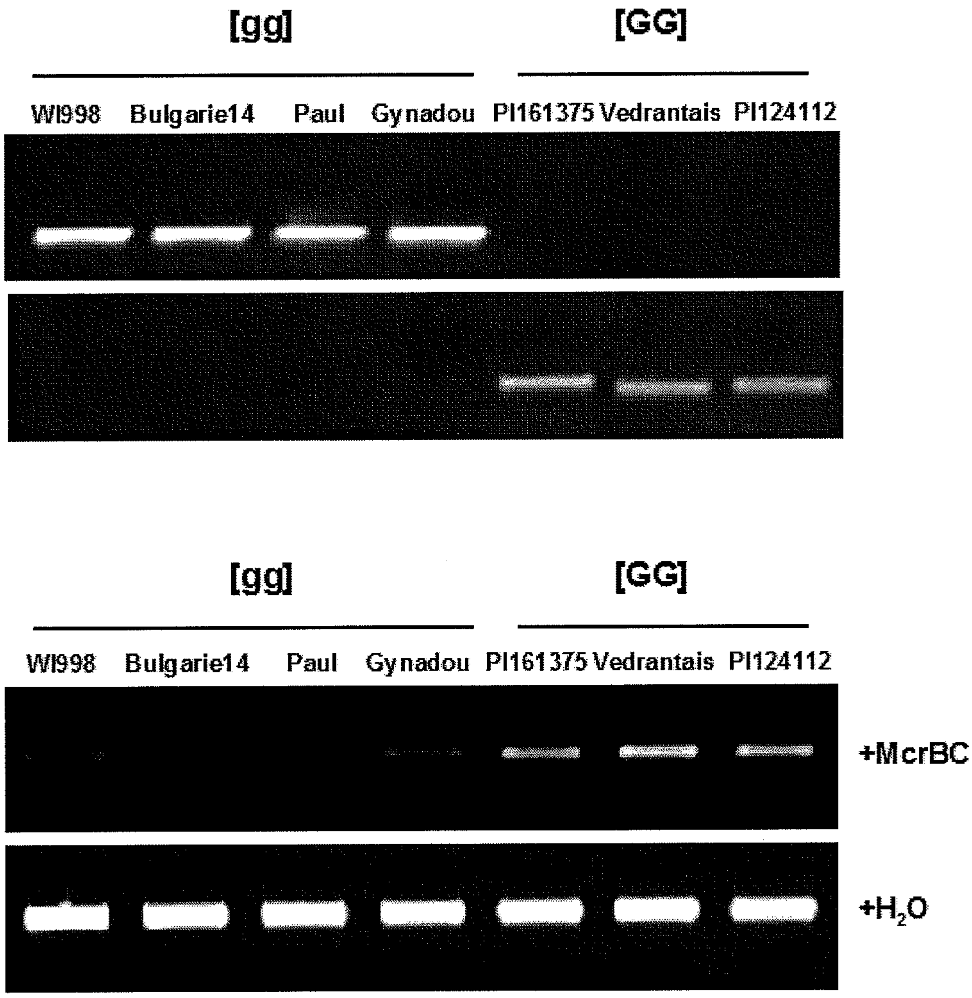
[gg]
[GG]
WI998
Bulgarie14
Paul
Gynadou
PI161375
Vedrantais
PI124112
[gg]
[GG]
WI998
Bulgarie14
Paul
Gynadou
PI161375
Vedrantais
PI124112
+McrBC
+$H_2O$

Figure 4

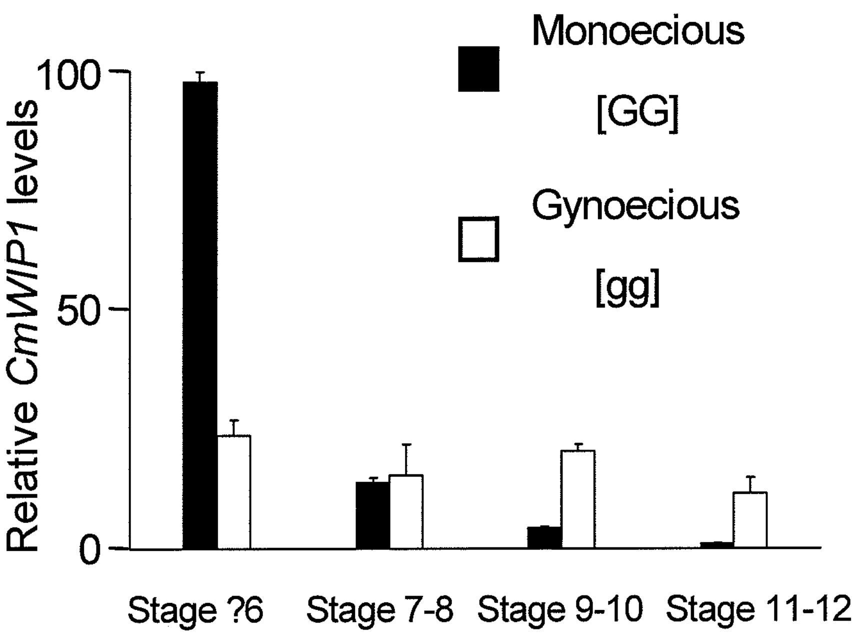
Monoecious
[GG]
Gynoecious
[gg]
100
50
0
Relative CmWIP1 levels
Stage ?6
Stage 7-8
Stage 9-10
Stage 11-12

Figure 5

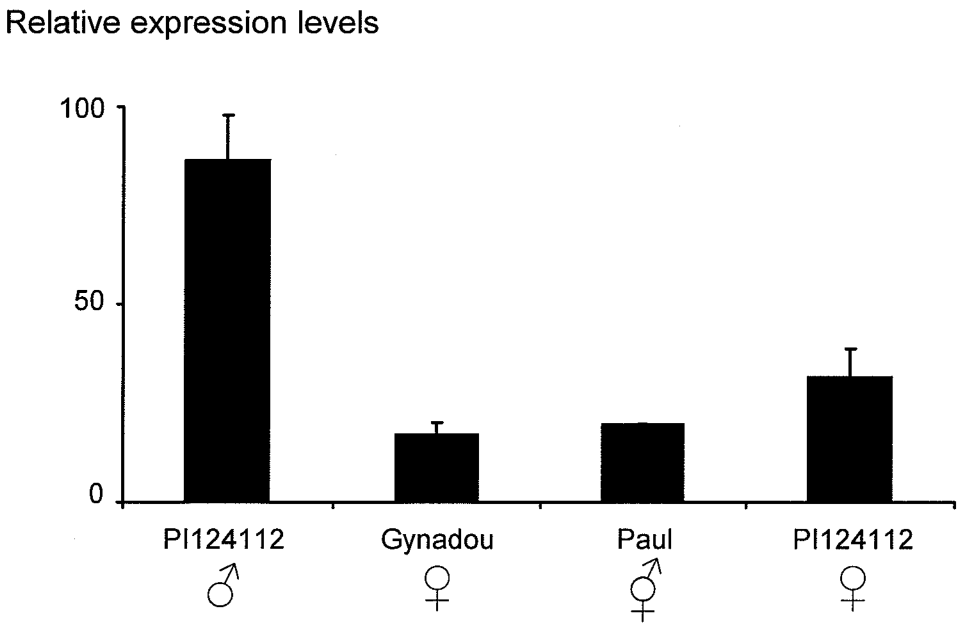

Relative expression levels
100
50
0
PI124112 ♂
Gynadou ♀
Paul ⚥
PI124112 ♀


Figure 6

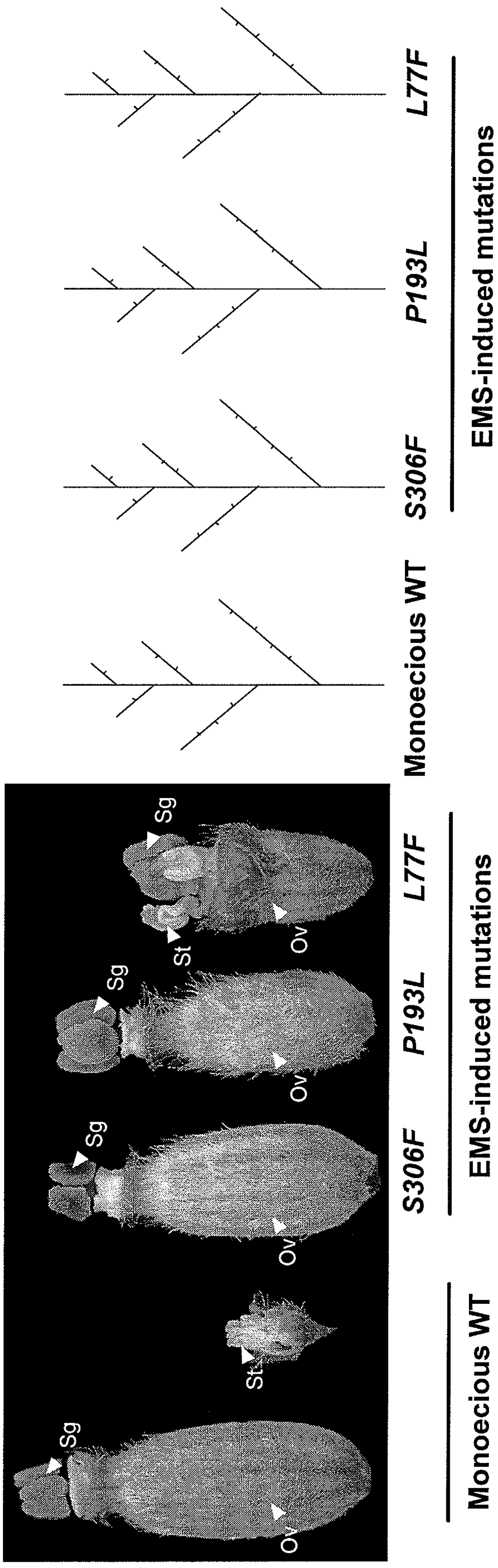
Sg
Ov
St
Monoecious WT
S306F
P193L
L77F
EMS-induced mutations

Figure 7

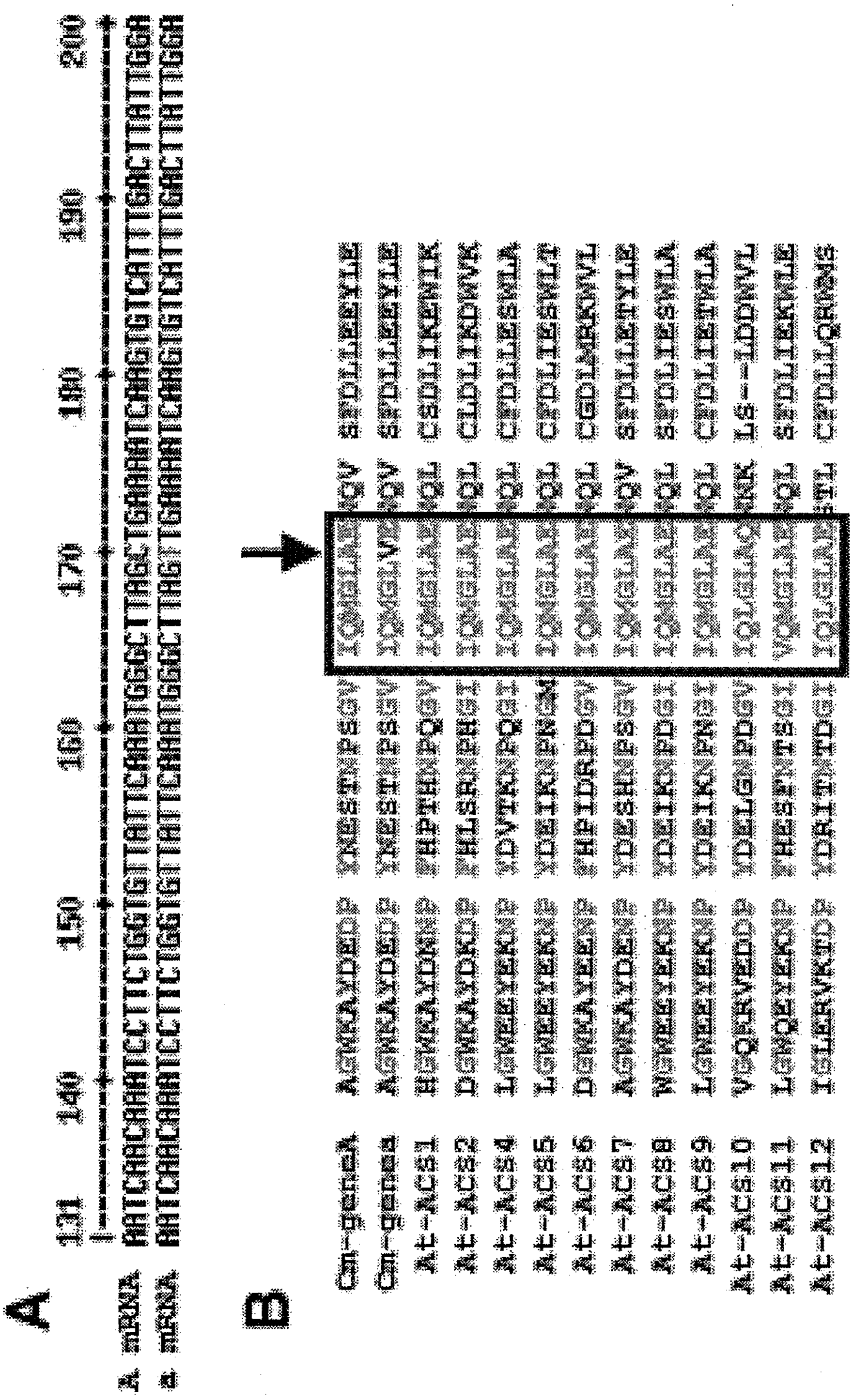

Figure 8

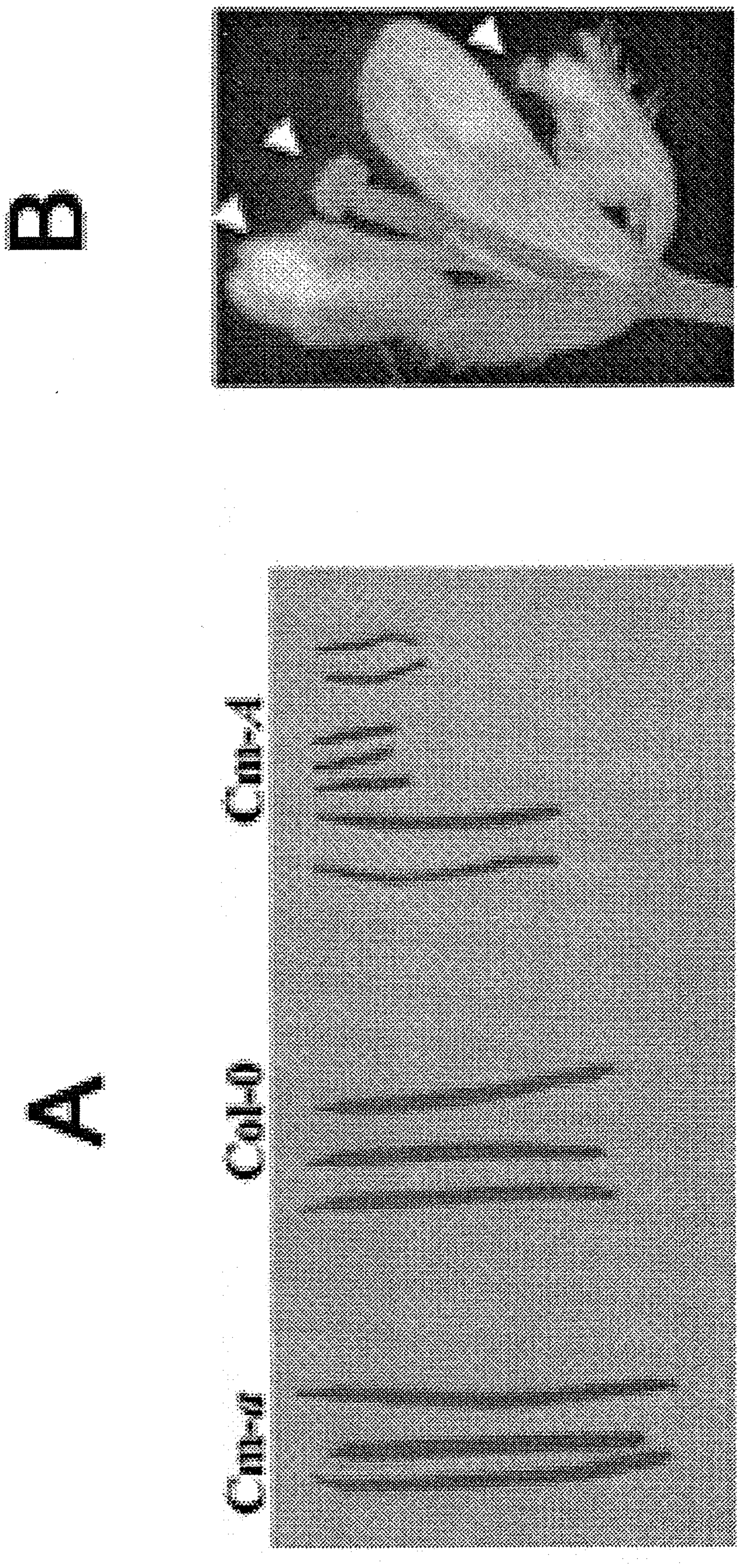


Figure 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2007125264 PCT
- WO 2007125264 A
- WO 9002172 A
- FR 2900415
- US 4946778 A
- WO 9504064 A
- EP 0707592 A
- FR 7810975
- EP 0225807 A, Chiron
- EP 672752 A, Ishida
- WO 2007125364 A


### Littérature non-brevet citée dans la description


- **MC CALLUM et al.** *Plant Physiology,* 2000, vol. 123, 439-442
- **KOORNBEEF et al.** *Mutat Res,* 1982, vol. 93, 109-123
- **MC CALLUM et al.** *Plant Physiol.,* 2000, vol. 123, 439-442
- **AN et al.** *Plant Physiol.,* 1986, vol. 81, 86-91
- **AOYAMA T et al.** *The Plant Journal,* 1997, vol. 11 (3), 605-612
- **AUSUBEL et al.** *Current protocols in molecular biology,* 1997
- **BEAUCAGE et al.** *Tetrahedron Lett.,* 1981, vol. 22, 1859-1862
- **BEVAN et al.** *Nucleic Acids Research,* vol. 12, 8711-8721
- **BROWN et al.** *Methods Enzymol.,* 1979, vol. 68, 109-151
- **CAUSSE et al.** *Molecular Breeding,* 1995, vol. 1, 259-272
- **CHRISTENSEN et al.** *Transgenic. Res.,* 1996, vol. 5, 213
- **FINER et al.** *Plant Cell Report,* 1992, vol. 11, 323-328
- **FROMM M. et al.** *Biotechnology,* 1990, vol. 8, 833-839
- Nucleic Acid Hybridization. 1984
- **GORLACH J ; VOLRATH S ; KNAUF-BEITER G ; HENGY G ; BECKHOVE U ; KOGEL KH ; OOSTENDORP M ; STAUB T ; WARD E ; KESSMANN H.** Benzothiadiazole, a novel class of inducers of systemic acquired resistance, activates gene expression and disease resistance in wheat. *Plant Cell,* 1996, vol. 8, 629-43
- DNA Cloning: A Practical Approach, Volumes I and II Oligonucleotide Synthesis. MRL Press, Ltd, 1985
- **GUERCHE et al.** *Mol Gen. Genet,* 1987, vol. 206, 382
- **HAMES ; HIGGINS.** Nucleic Acid Hybridization: a practical approach. IRL Press, 1985
- **HAJDUKIEWICZ ; P. SVAB. Z. ; MALIGA P.** *Plant Mol. Biol.,* 1994, vol. 25 (6), 989-994
- **ISHIDA et al.** *Nature biotechnology,* 1996, vol. 14, 745-750
- **JEFFERSON.** *Plant Molecular Biology Reporter,* 1987, vol. 5, 387-405
- **KAY et al.** *Science,* 1987, vol. 236, 4805
- **KAHANA, A. ; SILBERSTEIN, L. ; KESSLER, N. ; GOLDSTEIN, R. S. ; PERL-TREVES, R.** expression of ACC oxidase genes differs among sex genotypes and sex phases in cucumber. *Plant Mol Biol.,* Novembre 2000, vol. 41 (4), 517-528
- **KAMACHI, S. ; SEKIMOTO, H. ; KONDO, N. ; SAKAI, S.** Cloning of a cDNA for a 1-aminocyclopropane-1-carboxylate synthase that is expressed durig development of female flowers at the apices of Cucumis sativus L. *The Plant Cell Physiol.,* 1997, vol. 38, 1197-206
- **KOHLER G ; MILSTEIN C.** *Nature,* 1975, vol. 256, 495
- **KOOTER, JM. ; MATZKE, MA. ; MEYER, P.** Listening to the silent genes: transgene silencing, gene regulation and pathogen control. *Trends Plant Sci.,* 1999, vol. 4, 430-437
- **KOZBOR et al.** *Hybridoma,* 1983, vol. 2 (1), 7-16
- **LEGER OJ et al.** *Hum Antibodies,* 1997, vol. 8 (1), 3-16
- **MARTINEAU P et al.** *J. Mol. Biol.,* 1998, vol. 280 (1), 117-127
- **MARTINEZ, A. ; SPARKS, C. ; HART, CA. ; TOMPSON, J. ; JEPSON, I.** Ecdysone agonist inducible transcription in transgenic tobacco plants. *Plant J.,* 1999, vol. 19, 97-106
- **MCNELLIS T W.** *The Plant Journal,* 1998, vol. 14 (2), 247-257

- **MOLINA A ; HUNT MD ; RYALS JA.** Impaired fungicide activity in plants blocked in disease resistance signal transduction. *Plant Cell,* 1998, vol. 10, 1903-14
- **NARANG et al.** *Methods Enzymol.,* 1979, vol. 68, 90-98
- **NEUHAUS et al.** *Theor. Appl. Genet.,* 1987, vol. 75 (1), 30-36
- **REINMANN KA et al.** *Aids Res. Hum retroviruses,* 1997, vol. 13 (11), 933-943
- **RIDDER R. et al.** *Biotechnology (NY),* 1995, vol. 13 (3), 255-260
- **RISSER, G. ; RODE, J.C.** Induction par le nitrate d'argent de fleurs staminées chez des plantes gynoïques de melon (Cucumis melo L.). *Annales de l'Amélioration des Plantes,* 1979, vol. 29, 349-352
- **RUDICH, J. ; HALEVY, A.H. ; KEDAR, N.** Increase of femaleness of three cucurbits by treatment with Ethrel, an ethylene-releasing compound. *Planta,* 1969, vol. 86, 69-76
- **SAMBROOK et al.** Molecular Cloning A-laboratory manual. 2001
- **SANCHEZ PESCADOR.** *J. Clin. Microbiol.,* 1988, vol. 26 (10), 1934-1938
- **URDEA et al.** *Nucleic Acids Research,* 1988, vol. 11, 4937-4957
- **WASSENEGGER, M. ; PÉLISSIER, T.** A model for RNA-mediated gene silencing in higher plants. *Plant Mol. Biol.,* 1998, vol. 37, 349-362
- **WATSON et al.** ADN recombinant. De Boek Université, 1994, 273-292
- **YANG F. MOSS LG ; PHILIPS GN JR.** *Nat. Biotechnol.,* 14 Octobre 1996, vol. 10, 1246-51
- **YANG TT ; CHENG L. KAIN SR.** *Nucleic acids Res.,* 15 Novembre 1996, vol. 24 (22), 4592-3

LISTAGE DE SEQUENCES

<110> INSTITUT NATIONAL DE LA RECHERCHE AGRONOMIQUE INRA

<120> Combinaison de deux elements genetiques pour le controle du developpement du type floral d'une plante dicotyledone, et mise en oeuvre dans des procedes de detection et de selection

<130> V790FR

<160> 16

<170> PatentIn version 3.3

<210> 1
<211> 13380
<212> DNA
<213> Cucumis melo

<400> 1

```
tatacacatt ttgtaatgat aaaattagaa gactagttga ttaattgttt aggctttatt     60
atatattcat cataagtctt ttttgtagcc atttaggttt gttttcgtcg aattaatctt    120
ataaacacta tttttattcg taaattccgt tgctttctta tttactttat atcaatgctt    180
taaaacatca atctagtttt taaaaatcaa tatatatgtt tgcacacacc attattatcg    240
tatgttactc tatctattac tgacaaacgt tatgaaattt tattatattt gtaattatct    300
tttgcagttt tgtcatttaa aatcgttttt cttaaaagaa ttatgttgtt attttaaaat    360
tttggctaaa gaatcacgtg gagaattaga tatatcaaac ctttcatctt tgagatgaaa    420
gattacatca attactatta actaagctta ctttgataaa ttaaaatcat attaaaacaa    480
atagtccgta aaagaatata attttgaaaa actaaacagt catcaaacaa cgcgtgttag    540
cttttaatat atattatgat atgttaagtg aaaataaagt tgaagtgtat gaagccaaaa    600
gagaagtcgt tttcacttgt tgagttctaa tttctaggat ggttctatgt aaagtacttc    660
ctcttccaaa attggaatcc aactcactac ttataaacat catttattcg tcatcttaat    720
tacaatacca actcttattt ttgtctcatc tatcatcaca ctcactaatt aacattacca    780
ttatcttata tcattttatg aactcattat ttaacaaata aatcacttaa aagtttaact    840
tcaaaaaaaa aaaggaagaa agaaagaagg tttgaaatta cactatttgc aattaattat    900
gttttatgaa aactttctaa tactttaatt ttatgtcgaa tcgtttgtcg aatcgtttct    960
cttttatcct actacaaaaa tattataaaa tgattataaa tggctaaaat atatagtatg   1020
tgtatttcat aaatttaaga aaatgttttc gaatacagtc aaatgaacta aaatatttac   1080
aaaaatataa caaaatttca tatgtatatc gaataaaatt taaaaatttg aagactaaat   1140
ttgtaatata attacatatt aaagtaattt ttagatgtgt gggtattata taataataat   1200
gttgggaagg tgagggcatg aggcagctgg agggataagg actagggatt gttttatatc   1260
```

```
cttttttcaca tttaattttt gatgctaatt aatttgttgc caatcatttc atcacttttt   1320
tttttttttt tggttctaat ttatttactt tatatggaaa ataaataaaa gaaaaatgaa   1380
agaaagaaaa aagtggtttt caaatcaata gaaaaaacaa acaactccaa ctttaatggc   1440
ttgaaaacaa atgcattcta aaattaaacc ttatgattga tttgattttt attccccttt   1500
tttacacttt tcattttcat cataattata tcttcagtta cctgtccacc aattacacca   1560
tcaaatgtgg attattggga ttcttttttt tttttttaag attatcttac ggctttcatt   1620
tttttcgtat ctttatgacg gtttgataga cgtaaaagtg gttattgtgt tatagagatt   1680
tgtattattt tgatattatg gaaggattcg tttgagtaaa attataaaaa tcagaggggt   1740
gtcgtttaaa aatgtaagta atccaacaca aaaaaataat tatcataaaa tgtaaaaaaa   1800
agggttagat tgaaaacaaa cgaaacaaat gagttttgta ttataaatcg acctaaaatg   1860
ttcaacccaa acatggatta cgatacgacc gattcatctc attacagctc atcgatccta   1920
aaaatgtgaa gagaagtatt ggatataatt attacttaaa aagataatag aaaaaggaaa   1980
tcagcaaaat tagggttctt taataagtta taaaactcat ttatatacaa aattaattac   2040
attacaaaag gtgggaatgt ggatttagac atacaaccta taataattaa ttaaaaacaa   2100
tacacatgtt tcacaatttg agataattaa attttaatcc ccatttgata agtaatgatt   2160
ttatcttata aattagtttg ttaggtctat actttatttg tttatttatt tattcttact   2220
ctttttttaat tatattttta cttatatccc aagcttcatt aacgattaat ctaagtttga   2280
aatgattaat tacaaaatag tagtctattt tgatctatca cggactattg tgggtatttt   2340
ataatatttt gttatatttt ataaatattt ttagttcatt ttgctatatt tgaaaataac   2400
catatattat ttatatttta tttttctaga aaatattttc cataaactca agttctatat   2460
ttaaaatata tattcaaaag tttcctatta caaccctaag ttgaatactt atagaattgt   2520
aataaaataa gataattaac taaataagtc taattaaaca ctaataattt gaattaacaa   2580
cactaaacaa atattgtcaa caaaacttag ttcaattgac atctatatga agaatcgagt   2640
tccaaatctt cacacctgaa cattaacaaa attatatagt aattatctta attaattctc   2700
ctcatcgata aagtgaatat ctaattaaaa atttaaagtc aaaagtgtga atttcttgaa   2760
atatcaaatt aagacaaaat tcaaatcaat ttgaaaacat ataaacaaaa tggtaaatta   2820
gacaaaaaaa aaaaatccta aaaactacat atgaaaaggt tcattaccaa agaagttttt   2880
ccatgaaaaa aaaaaagaaa gaaagagaat aaaatattat atatagttaa taattatgaa   2940
atttttgtat aatcccataa agtttgcaac taaacttaag catatagttt atgacataat   3000
taaggtcact aataatagag aacagttaga gcaaaggtca aacatccact ttattcactc   3060
```

tctctcaatc atacaaagag atttaattga atctactcat tacaaaatcc ccaatcttat 3120
aataatatta atatcattaa tctcttatat atatatataa tatatataca tatattatct 3180
catgcacatg gattttcatg atcttcaaac cccacgtcgt tgattttcca taaaacctat 3240
atattccact aatcatttat attcattttt ttttttgggt ctaattttaa actatatgtt 3300
ttaaaactcc atagtttgat caattcaaaa aaaaaaaaaa aaaaaagtga gttatacaat 3360
ttttaaaatt tttaggacat aatcttgaca agtatcttta tctctcctac atgaaagagg 3420
gagcataaga ttagcttgac attgtctaaa attggaagtg tatatatata tatatatata 3480
tctataaatt tagaaattaa aataatgggg ttttttcatg aaatatatat taatagcttg 3540
attaaggaag gtttagaggg tgattaaagt gcaataatat tgttgattaa ttgttttttt 3600
tttcttatgt gtatcttagt ttcaaggact catgtttttt ttttcttttt tctttttggt 3660
cccatggaag agaacttttt ttcaattata ggatttgggt ttttagtttt tgggaattat 3720
tgaaaagtta taatttctgt tgctaatgat gggaaaatta tgaaaaatta tatatgcatg 3780
ggttggtggg gtcataagat ctcaaagaag cttttatttt gtcattattt ttctttagaa 3840
aatcagaatc ttaatctttt ttttttaca cattggtatt ttggtcccct ctcgtccaac 3900
ccaaatttaa aaaagatcaa aaaagaaaaa aaaaaaaaaa agaaacagaa acctaatctt 3960
aaatcaattt ccactatgca atccttaatt gtcatgttga tataaaaaaa aatagtaacg 4020
aggcaggaga ttgaaccata aaacttagct ttgtggttat taatacactt agatgatgct 4080
aattgagtta aactcttgat tgacaattaa aagaaaagtt aaatcattag ttaaataatt 4140
aaagtttaat gatcataagt taatatttga tgttgggtat taataaagga gatgcatttg 4200
actaaaaaaa tgattaggta gagactaggg taattaataa ccaatattaa taaagtatgg 4260
ttatgggggga attcatgaca aactcaagag gggatgttca tttgggtctt aatgaagtgt 4320
aggaattcaa ataatttaaa aagttattaa taattattat gattttatta ttattatttc 4380
atttgggtct acataagtat aaagaattga ttaaagaggt tgattatgca gaaagaaggg 4440
tgattagaga agtacaatta tgaagggatt ttggataaac acataggaac gaatgatttt 4500
cattgggggc cttaacaaat aatattcaat tttaaaaaaa ttgactattt gcaattaggt 4560
cttgatcatg aagatcctcg agataaatta tagtttttc ttttttcttt cgcatatgaa 4620
tttgttcgat ataacgaatt ttccgacata tcttacgtac actgataaga tattgtctgc 4680
ttaggatcta tacttgtgat ttattctatt atctaatcaa tgtgagattt tggtctcatt 4740
cctaacaatt ctctgctaat taattgaaca aaggacgatc actgaggctc cattcaaata 4800
ggaactctta tatctaggtt aattactatg ctacattaga acatatcacc tatctgatag 4860
agttcaaaca catatcacac catgagtact actttttgag gctaagctcc actacatctt 4920

```
tgtttgacac ccaaatactc tatctacacg actaggttag gagcataaac tttgatacca   4980
tctctttgag acataaactc ccgtcacttt atttttcatt tcattgatct aaaacgtctt   5040
ataccaatag agatagttgt tttcacatat atatacttat attatcctat tgcctagtga   5100
atctttatac aaagcaacat actttaattt tgattaaaca aagagtgatt acacatggag   5160
atcatagcct aattaaataa ttaaagtata attataggga gggattttga gagaaatgta   5220
attcaacaag gattttgcat aagggtctta gataaggaac taaacaacta gaaaaaaaaa   5280
tataatatat atatatataa aagggaaatg aaatcaaaga aagcatccat tctccatata   5340
tataaaaata catatatata tatggggaag agagaagaga ttacaaaact aatttaataa   5400
taaggtagtt gagggggcaa aaagcaaaat acaagagatt ttgattttg agagaagccc   5460
tttttagcaa aaaaaataaa atagattaat ataacacaca aacacacacc tactcctttt   5520
cttcaaccac cagattcgat tttgcctctc tctctctctc tctctctctc tctctctgtg   5580
gatcttaaac cccaattcaa aatatgatga caaattatta attattattc ctccaaaaat   5640
attttcccta ttaaaaaaaa taccaagaga gagaaaattc aatgattgtt ttttctcttt   5700
tacattattt ttcttttaaa gaaaaaaact tgctataaat agaggtgccc attgtaagag   5760
caacattcaa ttcaacaaat cttcagttca atttctctct ttttggctct caaaaaggga   5820
aagaaaaaaa aatcattatt attattattt cattttcttt ctttccctta aatttgagct   5880
gaaggaaaaa aaaaaaaaaa aaatcaatgg cgattgagat tgatattgag caaaatccaa   5940
cggttgaact ttcgcgaatc ggaacatcag aaacacacgg cgaagattcg ccgtattttg   6000
ctggctggaa agcgtatgat gaagatcctt ataatgaatc aacaaatcct tctggtgtta   6060
ttcaaatggg cttagctgaa aatcaagtaa gaatatataa cttttttttg ttttgttttg   6120
ctttgtaagg agattgggtt ttttttttta attgggtttg tgttggaatt tatgaaacag   6180
gtgtcatttg acttattgga ggaatatttg gaggaaaatt gtgagggaga agggaattat   6240
ttaaattctg ggtttagaga aaatgcttta tttcaagact atcatggtct tttctcattt   6300
agaagtgcaa tgggaagttt tatggaagag attagaggtg gaagagcaaa atttgaccca   6360
aatcgagttg ttttaactgc tggtgccact gctgccaatg agcttctcac tttcattctt   6420
gcaaatcctg gcgatgcttt gcttgtcccc actccttact atcctgggta agtttatcat   6480
cacctctacg ttttcgtatt tcatttcaaa aaccactctt tactgtaatt actataccct   6540
cagacattaa aattttaact ttcaaactat tcttaaagta tgagtttgag ggtatttcat   6600
atggggtttt taaatgtaaa tttatttaca tttttccact acttaagtgt cctatatttc   6660
tactaatttc ttcttgtgtt gtactcatat tttctatcgt ggggtggact acgtattttt   6720
```

acgagactat tcgtataaca tacgaatgag tgctttttaa accaaattct tcaaaatcca 6780
agtttaattt tggaaactag aaaatgggta gtttttttaaa atgttaccaa acgtgatctt 6840
tatccttaca atcaaacatt accaaggata attgcaacta ccgttagact ttatgagtgc 6900
ttttttttcc aactgttcta tatttttaca acattttgag ttgtattcat catttctgtt 6960
aaagatattt atatgtaact aagtattttt ataagacact gttggtataa tttcatgcac 7020
taataatata gtttcttttt ccagatttga cagagatttg agatggagaa caggagtgaa 7080
aattgtacca attcattgtg acagttcaaa caattttcaa ataactccaa aagcattaga 7140
agaagcttat aattcagcaa tggaaatgaa aatcaaagta agaggagttt taatcacaaa 7200
tccatcaaat ccactcggag caacgatcca acgctccaca atcgaagaca ttctagattt 7260
cgttacacgc aaaaacatcc acctcgtatc cgacgaaatc tattccggtt ccgttttctc 7320
ctccgccgag ttcacaagcg tcgctgaggt tttggaatcc cgcagctaca aaaacgccga 7380
acgtgtccac atcgtttaca gcctctccaa agatctcggc cttcccgggt ttagaatcgg 7440
cacgatctac tcatacaacg ataaagtcgt cacaaccgct cgccggatgt ctagctttac 7500
gcttatctct tcacaaacgc aacgattttt agcgtccatg ttgtcgaacc ggaagtttac 7560
ggagaaatat attaaaatga accgggacag gctcaagaaa cggtatgaaa tgattattga 7620
agggctgcga accgccggga ttgaatgttt ggaagggaat gccggtttgt tttgttggat 7680
gaatttgagc ccgttgttga aagataaaaa aaccaaagaa ggtgagattg agatatggaa 7740
gaggattttg aaggaagtga aattgaatat ttcgcccggt tcgtcgtgtc attgctctga 7800
acccggttgg ttcagggttt gttttgctaa tatgagtgaa aagactctgc atgttgccct 7860
tgatagaata cgtcggttca tggaacggat gaagaaggaa aacgaagcta attaaatata 7920
tatctatata taaatatatg aaaagaaaaa aaacatatgt agcttatttt attttatttt 7980
tttttacaat ggttgtgaga aaaaagaaaa aagaaaaaaa aaagaaaaaa aagccattgt 8040
gattcttttg tgtggacact gcccaatatt tgttagaaat ttggggtttt ttgtcttcat 8100
ttatacgtca tattttgatg atttaaactg aggaaaaaga aaaagaaatc cttgttttct 8160
tgcttttagc aaagcaagtt ttatttctca gttttatata tatatatata taaagtttct 8220
atttgtattg tcatttttat gtgatatgga atataattag tataattcgt tcttgcaatt 8280
aattacctcg aaaataaacg aaatacaaga aaaagaaaaa aaaaatctca tggagtattt 8340
tagggacaag tgtcaactca gggagagaga aaaaaatatg gtttaaattt aatagtattg 8400
gttattttca taacatgctc taaaaaggaa tataactaat aatttgactt taattaagaa 8460
aagaaaagct aataatatat aattaaaatc acttttagca acgaataaca ctttgccgac 8520
ttgtgtaatt aaccacctaa ctatccatct gacgtggaat gcaagtaatt aattaattga 8580

```
tttttttcgtt ttcaaatttt ggtcaacttg attcttggta ctaatttaat gtttccatct   8640
gtcagaaagc tacaacgttt ttcctccttc tttctttttt ttaagaatta ttttaaaaag   8700
tcaatacggt gctataatta gatttttatt tttcctcttt tttagtgtat atatatattt   8760
atataagtag agattaggaa ctaattgatt gaaaattaaa tatgctgtga cgctcaaaag   8820
atattaatcc cgcgttggtt ttatgtattt aaaaaatgta tttttctttt tttgatattt   8880
ttaaataata aaatatttaa attatttaca aaatataaca aaatttgctc gtttacattt   8940
ttattttgtg aaggacttat gcgatgtggt tcgatctaga attcttgtat tttcaaaata   9000
gatggagctt ctttttggat gaattctctc taggcttctg aagtcaaaaa ttttcaaccc   9060
aagaaaaaac tagagtttcc ttgtggtatg aggtgtatga aattgactca ttgactcaat   9120
tacatggact tttatcatat ttaactcagc taaattaagt ttattttttg gaattaatct   9180
aagtaaataa tatttaattg aaccaaaata tttaatttga tcagtcataa taaagacatg   9240
tgacatcatt ggaatcagtc aatttgtgtt taaatttaat ttgggataca tgtcaacttt   9300
tagttaatct caaatgcaat ttgtgattag ttacaaaatt tcttattcaa catacttcaa   9360
atctaaattt ggtaaattat gtttttttta aagaaattag atcaacacaa aaatataata   9420
tgttttgtga aaatgaaaat tttggtttaa tgggaggaga caaatttgaa cgacaaattt   9480
tcttagtaac ttacgatatg atcactaact aatttattat aggttggggg tttgaacctc   9540
tctcaacttt gtgctcatta tataatatat ctttaaaaga ccgaccttgc attaatgttg   9600
ttggttagtc tagtggtaga atcgtcattc tctagctctc tctaaattgt tccagcttca   9660
gtttttatat actttttttat attattttta ctgaactata aaaaattact gtcgacaaaa   9720
tttatgcttt tatcacttaa cataataatt gaactatgtt tgctttgttt tttctttttt   9780
aacgtatact atctcaaagt tttggataat gtacgtgttt gaaattttgt aacaaacaag   9840
gatacataaa tacgtaattg ttgttaatta tttcaaaatg taaatagatg atatgatgta   9900
ggggttgcta atattattgt ctaattattt ttgtaaagaa taaaataaaa taaaaatcca   9960
tatgatgcct aaggacgtgt ttagtattca atatggcgat tattatgtct ttttttcaaa  10020
tggattactt tttggatgat atgatatctt ttattttaat taaaactttt tgatgacttt  10080
taaaatttaa agggcataaa atatagcttt cttttgtaca tatatatggt tgtgcttgga  10140
ttttgtatct gcttgtcttt gtcccgagtt tctcgtcggt ttgaccttcg atctactttt  10200
tttgatatat atgttccaat atcgatttct aagctaacat atgtaaaaga tgattgcact  10260
cgtgtggtgc tgagactatc acacttgata cttgatataa gattgtatta ttcatctgaa  10320
ccaataaaaa ttaagatgga tattactcac tcttctcttt taggatcatc aaacaaggct  10380
```

```
cttttttga caatcacctg aagtcacgag taactctaca gtgttatagt tgtttatagt  10440
atttgagact ggaaatatca aattcagcca attcaattaa attttataat tgatatacat  10500
tcatatgctc gaaaagttgg cttccaaccg tccaacgctc taactttggg acatgtgtta  10560
caaaacacat aggtttaaaa aaatacctaa aaacataaaa aatacaactt tgatcatcct  10620
ttagttcaat tttattccca atttgatact tttgaattcc ctaactagag aattgtaatt  10680
gtgattgaac gtttttatag tcaaaatttg gatttgatag cttataggac cccatgtgcc  10740
aaacaaaata aaaattgcat atatatatat agagagagag ggggagtttt atattttaaa  10800
aaacaaaatt agaaaaaggc aattaatttt gttctttaaa cgacatggtt tctcacgtgt  10860
taaacgtcat tgttatcaac tgcgtcttgt cgctgaccaa ttcattgaca gcacagcaag  10920
caaaaaagaa aaaagaaaaa aaaaagtcaa aaagttaatg tggttaaaga aagcgctttt  10980
tgtgataact caaaaaaaca aaccaaccaa tggaagccct ccaggtcact ctcatgtggc  11040
tcccgtctcc acgtcatcaa tgaacacgga cgtgtttaca gctcaacaaa caacaactat  11100
tcctctgacg tggcaatttc ttattaatga atttccaact ctgccctcct attacatttt  11160
cttaaggtcg gtgaatgccg tacaatggaa gctcctagac atactttcta gttgtgatat  11220
atatatatat atattattca tccatcatat attaaactcc aatgtatgtt tgtggatttt  11280
acaaaagtta atattatttg gtagatgttg agatttcttt tttttccaat gtctagtctt  11340
tttccaagtt tggagaaagt tttttatgat gttgggagtt atttaatttc ctagtggggc  11400
cacgtagtta aataaatata ggttaaattt tacgaaatat ccatatatcg cgtgtgtggt  11460
atgatttcag ttacgtcact attttgaaaa catagttttc gtgttcttat taatgcttat  11520
agttgtaaat aacataatta aaagtatcat ttgttaaaat tgatgtcaca ccgtatgtac  11580
ataatttatt tattgattga tgttataagg ggcgttggag atatcgttgg aaaaaaatga  11640
tttgtaagga tgatcttaat tttctataat tgactcacgt atattatatt gtatacgttt  11700
ttcaaaattt acacaccaat catctcactt tcgttttcat ttttcatttt agtggaaaac  11760
aattcaataa aaaaaaattc tgacaacttt ttaaaattta aggcacagtt gaatcaatcc  11820
aaccgttcaa gatttaaaga agaaaaaaac taatttggtt gctccacttt ttgtttttgt  11880
tcgttttggt ccattaattc taaaaatgtt taatttattt gttatacttt caaatcttca  11940
caactttacc gtattgatcc cttaaaaatg aagtaaaaac aataatgaac gaactaagac  12000
aatcaccatt tgaaagttta aggaccaaat gaaccaaagt taaaagtata gaaacaaaaa  12060
taaacatcgc taaataaacc aaataaaact agaattactt aattgaaaca aaataatatg  12120
aaatggatca aatctttaga ctttagtgta tgggaaagtt ctatgaaaat gaccaccgac  12180
tatcgagaga ccaattttgg ggccaagtca atgattggta atttcaacct acatttatga  12240
```

```
tgtatgacaa tgacaatagc ttaggtcact ttgaaaatga ctataagatt ttctagttag  12300
agatatacac ttgatattag acttggtcgt tgtaataaaa actatgtgtc acggatgata  12360
tatgctaagt acatgtttta gtctttaatg tttgcgtata tttctttacg taatttaatc  12420
ttcgttaatt atatttttta aactatgttt taatctttta attcttttgt tgtgaaattg  12480
acaaataaag agaaacacga caatgtagat ggtaaacaat gaagtttgtg tagtttattg  12540
acaatatgtt ggcaatgttt acgagtagag agataattgt tcaaattaca gaacaatagg  12600
tgacaatacg tgagtttttg ttttaattta cttttgaaac tttattttga ttttcaaaac  12660
ttaaagaagt tgatactatt attgttttga gctatgaaga tgtgtgatcg aacctttcac  12720
acgtttagaa tgaaagagca tgtcaattaa ttttgagcta aacttgttta aaaaaattga  12780
ccttttgtct ttgttttaag ttttaacaaa ttaatgatgt cattgcgtaa ttttaagtca  12840
gttaggtatg aaaagccacc atcgaagaaa gaaaatttca agaagaaaag caatgtagta  12900
aatcacaaat aattgttttt ctttcccata ggttataact ataaaaaaaa aacttctttt  12960
ttagtataat aacggtaaag aaggatgatc aaaccttcta actcagtcaa ttgcaaatat  13020
gataaattca ctttgacaaa ctaaaataaa tttgaagatt tatgaaacaa aatgtacatt  13080
ttaaaagttt atattttcac acaatgttag cttcctttta aaaaaaaatt aaattttaaa  13140
agttcagaga acaaaacata catttcaact ttgctaactt caatatagaa cttatataaa  13200
atcgtgccac atagggttca aaagaactat aggattttaa aatgaaaaca tatattttaa  13260
ttgagtttta gaactaagtt aatatattta tttataaaga ataacacttc agtaaaatta  13320
agaacaacac agacatagtt caataacata aaactagtac cttttacatc aatattagaa  13380
```

<210> 2
<211> 11137
<212> DNA
<213> Cucumis melo

<400> 2

```
tagtctattt tgatctatca cgaactattg taggtatttt ataatatttt gttatatttt     60
ataaatattt ttagttcatt ttgctatatt taaaaataac catatattat ttatatttta    120
tttttctaaa aaatattttc cataaactca agttctatat ttaaaatata tattcaaaac    180
tttcttatta caaccctaag ttgaatactt atagaattgt aataaaataa gataattaac    240
taaataagtc taattaaaca ttaataattt gaattaacaa cactaaacaa atattgtcaa    300
caaaacttag ttcaattgac atctatatga agaatcgact tccaaatctt cgcacctgaa    360
cattaacaaa attatatagt aattatctta attaattatc ctcatcgata aagtgaatat    420
ctaattaaaa atttaaagtc aaaagtgtga atttcttgaa atatcaaatt aagacaaaat    480
```

```
tcaaatctct ttagagtaaa attatagaat ttgaaaacat ataaacgaaa aatggtaaat     540
tagacaaaaa aaaaatatat tttttctaaa aactacatat gaaaaggttc attaccaaag     600
aagtttttcc atgaaaaaaa aaagaaagaa agaaagaaag agaataaaat attatatata     660
gttaataatt atgaaatttt tgtataatcc cataaagttt gcaactaaac ttaagcatat     720
agtttatgac ataattaagg tcactaataa tagagaacag ttagagcaaa ggtcaaacat     780
ccactttatt cactctctct caatcataca aagagattta attgaatcta ctcattacaa     840
aatccccaat cttataataa tattaatatc attaatctct tatatatata tataatatat     900
atacatatat tatctcatgc acatggattt tcatgatctt caaaccccac gtcgttgatt     960
ttccataaaa cctatatatt ccactaatca tttatattca tttttttttt tgggtctaat    1020
tttaaactat atgttttaaa actccatagt ttgatcaatt caaaaaaaaa aaaaaaaaaa    1080
aaagtgagtt atacaatttt taaaattttt aggacataat cttgacaagt atctttatct    1140
ctcctacatg aaagagggag cataagatta gcttgacatt gtctaaaatt ggaagtgtat    1200
atatatatat atatatatct ataaatttag aaattaaaat aatggggttt tttcatgaaa    1260
tatatattaa tagcttaatt aaggaaggtt tagagggtga ttaaagtgca ataatattgt    1320
tgattaattg tttttttttc ttatgtgtat cttagtttca aggactcatg tttttttttt    1380
ctttttcttt tttggtccca tggaagagaa cttttttttca attataggat ttgggttttt    1440
agtttttggg aattattgaa aagttataat ttctgttgct aatgatggga aaattatgaa    1500
aaattatata tgcatgggtt ggtggggtca taagatctca aagaagcttt tattttgtca    1560
ttatttttct ttagaaaatc agaatcttaa tctttttttt ttacacattg gtattttggt    1620
cccctctcgt ccaacccaaa tttaaaaaag atcaaaaaag aaaaaagaaa aaaaaaaaaa    1680
gaagaagaag aaacctaatc ttaaattaat ttccactatg caatccttaa ttgtcatgtt    1740
gatataaaaa aaaatagtaa cgaggtagga gattcaacca taaaacttag ctttgtggtt    1800
attaatacac ttagatgatg ctaattgagt taaactcttg attgacaatt aaaagaaaag    1860
ttaaatcatt agttaaataa ttaaagttta atgatcataa gttaatattt gatgttgggt    1920
attaataaag gagatgcatt tgactaaaaa aatgattagg tagagactag ggtaattaat    1980
aacaaatatt aataaagtat ggttatgggg gaattcatga caaactcaag aggggatgtt    2040
catttgggtc ttaatgaagt gtaggaattc aaataattta aaaagttatt aataattatt    2100
atgattttat tattattatt tcatttgggt ctacataagt ataaagaatt gattaaagag    2160
gttgattatg cagaaagaag ggtgattaga gaagtacaat tatgaaggga ttttggataa    2220
acacatagga acgaatgatt ttcattgggg gccttaacaa ataatattca atttttaaaa    2280
```

aattgactat ttgcaattag gtcttgatca tgaagatcct cgagataaat tatagttttt 2340

tcttgttttc ttcgcatatg aatttgttcg atataacgaa ttttccgaca tatcttacgt 2400

acactgataa gatattgtct gcttaagatc tatacttgtg atttattcta ttatctaatc 2460

aatgtgagat tttggtctca ttcctaacaa ttctctgcta attaattgaa caaaggacga 2520

tcactgaggc tccattcaaa taggaactct tatatctagg ttaattacta tgctacatta 2580

gaacatatca cctatctgat agagttcaaa cacatatcac accatgagta ctacttttcg 2640

aggctaagct ccactacatc tttgtttgac acccaaatac tctatctaca cgactaggtt 2700

aggagcataa actttgatac catctctttg agacataaac tcccgtcact ttatttttca 2760

tttcactgat ctaaaacgtc ttataccaat agagatagtt gttttcacat atatatactt 2820

atattatcct attacctagt gaatctttct gcaaagcaca tactttaatt ttgattaaac 2880

aaaaagtgat tacacatgga tatcatagcc taattaaata attaaagtat aattatgggg 2940

gggggggat tttgagagaa atgtaattca acaaggattt tgcataaggg tcttagataa 3000

ggaactaaac aactagaaaa aaaatataat atatatatat ataaaaaagg gaaatgaaat 3060

caaagaaagc atccattctc catatatata aaaatacata tatatatatg gggaagagag 3120

aagagattac aaaactaatt taataataag gtagttgagg gggcaaaaag caaaatacaa 3180

gagattttga tttttgagag aagccctttt tagcaaaaaa aataaaatag attaatataa 3240

cacacaaaca cacacctact ccttttcttc aaccaccaga ttcgattttg cctctctctc 3300

tctctctctc tctctctctc tctctctgga tcttaaaccc caattcaaaa tatgatgaca 3360

aattattaat tattattcct ccaaaaatat tttccctatt aaaaaaaata ccaagagaga 3420

gaaaattcaa tgattgtttt ttctctttta cattattttt tttttaaaga aaaaaacttg 3480

ctataaatag aggtgcccat tgtaagagca acattcaatt caacaaatct tcagttcaat 3540

ttctctcttt ttggctctca aaaagggaaa gaaaaaaaaa tcattattat tattatttca 3600

ttttctttct ttcccttaaa tttgagctga aggaaaaaaa aaaaaaatca atggcgattg 3660

agattgatat tgagcaaaat ccaacggttg aactttcgcg aatcggaaca tcagaaacac 3720

acggcgaaga ttcgccgtat tttgctggct ggaaagcgta tgatgaagat ccttataatg 3780

aatcaacaaa tccttctggt gttattcaaa tgggcttagt tgaaaatcaa gtaagaatat 3840

ataacttttt tttgttttgt tttgctttgt aaggagattg gggttttttt ttaattgggt 3900

ttgtgttgga atttatgaaa caggtgtcat ttgacttatt ggaggaatat ttggaggaaa 3960

attgtgaggg agaagggaat tatttaaatt ctgggtttag agaaaatgct ttatttcaag 4020

actatcatgg tcttttctca tttagaagtg caatgggaag ttttatggaa gagattagag 4080

gtggaagagc aaaatttgac ccaaatcgag ttgttttaac tgctggtgcc actgctgcca 4140

atgagcttct cactttcatt cttgcaaatc ctggcgatgc tttgcttgtc cccactcctt 4200

actatcctgg gtaagtttat catcacctct acgttttcgt atttcatttc aaaaaccact 4260

ctttactgta attactatac cctcagacat taaaatttta actttcaaac tattcttaaa 4320

gtatgagttt gagggtattt catatggggt ttttaaatgt aaatttattt acatttttcc 4380

actacttaag tgtcctatat ttctactcat ttcttcttgt gttgtactca tattttctat 4440

cgtggggtgg actacgtatt tttacgagac tattcgtata acatacgaat gagtgctttt 4500

taaaccaaat tcttcaaaat ccaagtttaa ttttggaaac tagaaaatgg gtagtttttt 4560

aaaatgttac caaacgtgat ctttatcctt acaatcaaac attatcaagg ataattgcaa 4620

ctatcattag actttatgag tgcttttttt ttccaactgt tctatatttt tacaacattt 4680

tgagttatat tcatcacttc tgttaaagat atttatatgt aactaagtat ttttataaga 4740

cactgttggt ataatttcat gcactaataa tatagtttct ttttccagat ttgacagaga 4800

tttgagatgg agaacaggag tgaaaattgt accaattcat tgtgacagtt caaacaattt 4860

tcaaataact ccaaaagcat tagaagaagc ttataattca gcaatggaaa tgaaaatcaa 4920

agtaagagga gttttaatca caaatccatc aaatccactc ggagcaacga tccaacgctc 4980

cacaatcgaa gacattctag atttcgttac acgcaaaaac atccacctcg tatccgacga 5040

aatctattcc ggttccgttt tctcctccgc cgagttcaca agcgtcgctg aggttttgga 5100

atcccgcagc tacaaaaacg ccgaacgtgt ccacatcgtt tacagcctct ccaaagatct 5160

cggccttccc gggtttagaa tcggcacgat ctactcatac aacgataaag tcgtcacaac 5220

cgctcgccgg atgtctagct ttacgcttat ctcttcacaa acgcaacgat ttttagcgtc 5280

catgttgtcg aaccggaagt ttacggagaa atatattaaa atgaaccggg acaggctcaa 5340

gaaacggtat gaaatgatta ttgaagggct gcgaaccgct gggattgaat gtttggaagg 5400

gaatgccggt ttgttttgtt ggatgaattt gagcccgttg ttgaaagata aaaaaaccaa 5460

agaaggtgag attgagatat ggaagaggat tttgaaggaa gtgaaattga atatttcgcc 5520

cggttcgtcg tgtcattgct ctgaacccgg ttggttcagg gtttgttttg ctaatatgag 5580

tgaaaagact ctgcatgttg cccttgatag aatacgtcgg ttcatggaac ggatgaagaa 5640

ggaaaacgaa gctaattaaa tatatatata tatatatata aatatatgaa aagaaaaaaa 5700

acatatgtag cttattttat tttatttttt ttttacaatg gttgtgagaa aaaagaaaaa 5760

agaaaaaaga aaaaaaaaaa gaaaaaaaag ccattgtgat tcttttgtgt ggacactgcc 5820

caatatttgt tagaaatttg gggttttttg tcttcattta tacgtcatat tttgatgatt 5880

taaactgagg aaaaagaaaa agaaatcctt gttttcttgc ttttagcaaa gcaagtttta 5940

tttctcagtt ttatatatat atataaagtt tctatttgta ttgtcatttt tatgtgatat 6000

ggaatataat tagtataatt cgttcttgca attaattacc tcgaaaataa acgaaataca 6060

agaaaaagaa aaaaaaaatc tcatggagta ttttagggac aagtgtcaac tcagggagag 6120

agaaaaaaat atggtttaaa tttaatagta ttggttattt tcataacatg ctctaaaaag 6180

gaatataact aataatttga ctttaattaa gaaaagaaaa gctaataata tataattaaa 6240

atcactttta gcaacgaata acactttgcc gacttgtgta attaaccacc taactatcca 6300

tctgacgtgg aatgcaagta attaattaat tgattttttc gttttcaaat tttggtcaac 6360

ttgattcttg gtactaattt aatgtttcca tctgtcagaa agctacaacg tttttcctcc 6420

ttctttcttt tttttaagaa ttattttaaa aagtcaatac ggtgctataa ttagattttt 6480

atttttcctc ttttttagtg tatatatata tttatataag tagagattag gaactaattg 6540

attgaaaatt aaatatgctg tgacgctcaa aagatattaa tcccgcgttg gttttatgta 6600

tttaaaaaat gtatttttttc ttttttgata tttttaaata ataaaatatt taaattattt 6660

acaaaatata acaaaatttg ctcgtttaca tttttatttt gtgaaggact tatgcgatgt 6720

ggttcgatct agaattcttg tattttcaaa atagatggag cttctttttg gatgaattct 6780

ctctaggctt ctgaagtcaa aaattttcaa cccaagaaaa aactagagtt tccttgtggt 6840

atgaggtgta tgaaattgac tcattgactc aattacatgg acttttatca tatttaactc 6900

agctaaatta agtttatttt ttggaattaa tctaagtaaa taatatttaa ttgaaccaaa 6960

atatttaatt tgatcagtca taataaagac atgtgacatc attggaatca gtcaatttgt 7020

gtttaaattt aatttgggat acatgtcaac ttttagttaa tctcaaatgc aatttgtgat 7080

tagttacaaa atttcttatt caacatactt caaatctaaa tttggtaaat tatgtttttt 7140

ttaaagaaat tagatcaaca caaaaatata atatgttttg tgaaaatgaa aattttggtt 7200

taatgggagg agacaaattt gaacgacaaa ttttcttagt aacttacgat atgatcacta 7260

actaatttat tctaggttgg gggtttgaac ctctctcaac tttgtgctca ttatataata 7320

tatctttaaa agaccgacct tgcattaatg ttgttggtta gtctagtggt agaatcgtca 7380

ttctctagct ctctctaaat tgttccagct tcagttttta tatacttttt tatattattt 7440

ttactgaact ataaaaaatt actgtcgaca aaatttatgc ttttatcact taacataata 7500

attgaactat gtttgctttg ttttttcttt tttaacgtat actatctcaa agttttggat 7560

aatgtacgtg tttgaaattt tgtaacaaac aaggatacat aaatacgtaa ttgttgttaa 7620

ttatttcaaa atgtaaatag atgatatgat gtaggggttg ctaatattat tgtctaatta 7680

tttttgtaaa gaataaaata aaataaaaat ccatatgatg cctaaggacg tgtttagtat 7740

tcaatatggc gattattatg tctttttttc aaatggatta ctttttggat gatatgatat 7800

cttttatttt aattaaaact ttttgatggc ttttaaaatt taaagggcat aaaatatagc 7860
tttcttttgt acatatatat ggttgtgctt ggattttgta tctgcctgtc tttgtcccga 7920
gtttctcgtc ggtttgacct ccgatctact tttcttgata tatatgttcc aatatcgatt 7980
tctaagctaa catatgtaag agatgattgc actcgtgtgg tgtcgagact atcacacttg 8040
atacttgata taagattgta ttattcatct gaaccaataa aaattaagat ggatattact 8100
cactcttctc ttttaggatc atcaaacaat gctctttttt tgacaatcac ctgaagtcat 8160
gagtaactct acagtgttat agttgtttat agtatttgag actagaaata acaaattcag 8220
ccaattcaat taaattttat aattgatata cattcatatg ctcgaaaagt tggcttccaa 8280
ccgtccaacg ccctaacttt gggacatgtg ttacaaaaca cataggttta aaaaaatacc 8340
taaaaacata aaaaaaaata caattttgat catcctttag ttcaatttta ttcccaattt 8400
gatacttttg aattccctaa ctagagaatt gtaattgtga ttgaacgttt ttatagtcaa 8460
aatttggatt tgatagctta tagaacccca tgtgccaaac aaaataaaaa ttgcatacat 8520
atatatatat atatatatat atatatatat atatatatat atatatatat atatagaggg 8580
agttttatat tttcaaaaac aaaattagaa aaaggcaatt aattttgttc tttaaacgac 8640
atggtttctc acgtgttaaa cgtcattgtt atcaactgcg tcttgtcgct gaccaattca 8700
ttgacagcac agcaagcaaa aaagaaaaaa aaaaaaaaaa aagtcaaaaa gttaatgtgg 8760
ttaaagaaag cgctttttgt gataactcaa aaaaacaaac caaccaatgg aagccctcca 8820
ggtcactctc atgtggctcc cgtctccacg tcatcaatga acacggacgt gtttacagct 8880
caacaaacaa caaccattcc tctgacgtgg caatttctta ttaatgaatt tccaactctg 8940
ccctcctatt acattttctt aaggtcggtg aatgccgtac aatggaagct cctagacata 9000
ctttctagtt gtgatatata tatatatata tatatattat tcatccatca tatattaaac 9060
tccaatgtat gtttgtggat tttacaaaag ttaatattat ttggtagatg ttgagatttc 9120
ttttttttcc aatgtctagt ctttttccaa gtttggagaa agtttttttat gatgttggga 9180
gttatttaat ttcctagtgg ggccacgtag ttaaataaat ataggttaaa ttttacgaaa 9240
tatccatata tcgcgtgtgt ggtatgattt cagttacgtc actattttga aaacatagtt 9300
ttcgtgttct tattaatgct tatagttgta aataacataa ttaaaagtat catttgttaa 9360
aattgatgtc acaccgtatg tacataattt atttattgat tgatgttata aggggcgttg 9420
gagatatcgt tggaaaaaaa tgatttgtaa ggatgatctt aattttctat aattgactca 9480
cgtatattat attgtatacg tttttcaaaa tttacacacc aatcatctca ctttcgtttt 9540
catttttcat tttagtggaa aacaattcaa aaaaaaaaaa aattctgaca actttttaaa 9600

```
atttaaggca cagttgaatc aatccaaccg ttcaagattt aaagaagaaa aaaactaatt    9660
tggttgctcc actttttgtt tttgttcgtt ttggtccatt aattctaaaa atgtttaatt    9720
tatttgttat actttcaaat cttcacaact ttaccgtatt gatcccttaa aaatgaagta    9780
agtcaatcac catttgaaag tttaaggacc aaatgaacca aagttaaaag tataaaaaca    9840
aaaataaaca tcgctaaata aaccaaataa aactagaatt acttaattga aacaaaataa    9900
tatgaaatgg atcaaatctt tagactttag tgtatgggaa agttctatga aaatgaccac    9960
cgactatcga gagaccaatt ttggggccaa gtcaatgatt ggtaatttca acctacattt   10020
atgatgtatg acaatgacaa tagcttaggt cactttgaaa atgactataa gattttctag   10080
ttagagatat acacttgata ttagacttgg tcgttgtaat aaaaactatg tgtcacggat   10140
gatatatgct aagtacatgt tttagtcttt aatgtttgcg tatatttctt tacgtaattt   10200
aatcttcgtt aattatattt tttaaactat gttttaatct tttaattctt ttgttgtgaa   10260
attgatacaa ataaagaggt ttctctttat tgacaatatg ttgcgtagtt tattgacaat   10320
atgttggcaa tgtttacgag tagagagata attgttcaaa ttacagaaca ataggtgaca   10380
atacgtgagt ttttgtttta atttagtttt gaaactttat tttgattttc aaaacttaaa   10440
gaagttgata ctattattgt tttgagctat gaagatgtgt gatcgaacct ttcacacgtt   10500
tagaatgaaa gagcatgtca attaattttg agctaaactt gtttaaaaaa attgaccttt   10560
tgtctttgtt ttaagtttta acaaattaat gatgtcattg cgtaatttta agtcagttag   10620
gtatgaaaag ccaccatcga agaaagaaaa tttcaagaag aaaagcaatg tagtaaatca   10680
caaataattg tttttctttc ccataggtta taactataaa aaaaaacttc ttttttagta   10740
taataacggt aaagaaggat gatcaaacct tctaactcag tcaattgcaa atatgataaa   10800
ttcactttga caaactaaaa taaatttgaa gatttatgaa acaaaatgta cattttaaaa   10860
gtttatattt tcacacaatg ttagcttcct tttaaaaaaa atttaaattt taaaagttca   10920
gagaacaaaa catacatttc aactttgcta acttcaatat agaacttata taaaatcgtg   10980
ccacataggg ttcaaaagaa ctataggatt ttaaaatgaa aacatatatt ttaattgagt   11040
tttagaacta agttaatata tttatttata aagaataaca cttcagtaaa attaagaaca   11100
acacagacat agttcaataa cataaaacta gaggatc                            11137
```

<210> 3
<211> 445
<212> PRT
<213> Cucumis melo

<400> 3

Met Ala Ile Glu Ile Asp Ile Glu Gln Asn Pro Thr Val Glu Leu Ser

```
1               5                   10                  15

Arg Ile Gly Thr Ser Glu Thr His Gly Glu Asp Ser Pro Tyr Phe Ala
            20                  25                  30

Gly Trp Lys Ala Tyr Asp Glu Asp Pro Tyr Asn Glu Ser Thr Asn Pro
        35                  40                  45

Ser Gly Val Ile Gln Met Gly Leu Ala Glu Asn Gln Val Ser Phe Asp
    50                  55                  60

Leu Leu Glu Glu Tyr Leu Glu Glu Asn Cys Glu Gly Glu Gly Asn Tyr
65                  70                  75                  80

Leu Asn Ser Gly Phe Arg Glu Asn Ala Leu Phe Gln Asp Tyr His Gly
                85                  90                  95

Leu Phe Ser Phe Arg Ser Ala Met Gly Ser Phe Met Glu Glu Ile Arg
            100                 105                 110

Gly Gly Arg Ala Lys Phe Asp Pro Asn Arg Val Val Leu Thr Ala Gly
        115                 120                 125

Ala Thr Ala Ala Asn Glu Leu Leu Thr Phe Ile Leu Ala Asn Pro Gly
    130                 135                 140

Asp Ala Leu Leu Val Pro Thr Pro Tyr Tyr Pro Gly Phe Asp Arg Asp
145                 150                 155                 160

Leu Arg Trp Arg Thr Gly Val Lys Ile Val Pro Ile His Cys Asp Ser
                165                 170                 175

Ser Asn Asn Phe Gln Ile Thr Pro Lys Ala Leu Glu Glu Ala Tyr Asn
            180                 185                 190

Ser Ala Met Glu Met Lys Ile Lys Val Arg Gly Val Leu Ile Thr Asn
        195                 200                 205

Pro Ser Asn Pro Leu Gly Ala Thr Ile Gln Arg Ser Thr Ile Glu Asp
    210                 215                 220

Ile Leu Asp Phe Val Thr Arg Lys Asn Ile His Leu Val Ser Asp Glu
225                 230                 235                 240

Ile Tyr Ser Gly Ser Val Phe Ser Ser Ala Glu Phe Thr Ser Val Ala
                245                 250                 255
```

```
Glu Val Leu Glu Ser Arg Ser Tyr Lys Asn Ala Glu Arg Val His Ile
            260                 265                 270

Val Tyr Ser Leu Ser Lys Asp Leu Gly Leu Pro Gly Phe Arg Ile Gly
        275                 280                 285

Thr Ile Tyr Ser Tyr Asn Asp Lys Val Val Thr Thr Ala Arg Arg Met
    290                 295                 300

Ser Ser Phe Thr Leu Ile Ser Ser Gln Thr Gln Arg Phe Leu Ala Ser
305                 310                 315                 320

Met Leu Ser Asn Arg Lys Phe Thr Glu Lys Tyr Ile Lys Met Asn Arg
                325                 330                 335

Asp Arg Leu Lys Lys Arg Tyr Glu Met Ile Ile Glu Gly Leu Arg Thr
            340                 345                 350

Ala Gly Ile Glu Cys Leu Glu Gly Asn Ala Gly Leu Phe Cys Trp Met
        355                 360                 365

Asn Leu Ser Pro Leu Leu Lys Asp Lys Lys Thr Lys Glu Gly Glu Ile
    370                 375                 380

Glu Ile Trp Lys Arg Ile Leu Lys Glu Val Lys Leu Asn Ile Ser Pro
385                 390                 395                 400

Gly Ser Ser Cys His Cys Ser Glu Pro Gly Trp Phe Arg Val Cys Phe
                405                 410                 415

Ala Asn Met Ser Glu Lys Thr Leu His Val Ala Leu Asp Arg Ile Arg
            420                 425                 430

Arg Phe Met Glu Arg Met Lys Lys Glu Asn Glu Ala Asn
        435                 440                 445
```

<210> 4
<211> 19
<212> DNA
<213> artificial sequence

<220>
<223> amorce

<400> 4
gatgagtcct gagtaagta 19

<210> 5

<211> 19
<212> DNA
<213> artificial sequence

<220>
<223> amorce

<400> 5
gatgagtcct gagtaatgt 19

<210> 6
<211> 18
<212> DNA
<213> artificial sequence

<220>
<223> sonde

<400> 6
gactgcgtac atgcagca 18

<210> 7
<211> 27
<212> DNA
<213> artificial sequence

<220>
<223> sonde

<400> 7
cgtattttgc tggctggaaa gcgtatg 27

<210> 8
<211> 27
<212> DNA
<213> artificial sequence

<220>
<223> sonde

<400> 8
cgatagaaaa tatgagtaca acacaag 27

<210> 9
<211> 16177
<212> DNA
<213> artificial sequence

<220>
<223> Vecteur pEC2 avecinsertion du g鈩e ACS

<400> 9

```
ggaaacagct atgaccatga ttacgccaag ctcggaatta accctcacta aagggaacaa     60
aagctggagc tccaccgcgg tggccggggc cgctctagcc acagacagct ccgtagccct    120
```

cgttctcctt ggagttcttc gggaaatgga tctttcgatt cccgatgatg tctctcttat 180
ctgctttgac gacgccgact ggacatccgc tataacgccg ccattgaccg tgatttcgca 240
acctgtcagg gatctcgcga cggctgccac agaagacctg atcgcccgct taaagggcga 300
gacttcagcc ccacccaagg aaactcttct cccggcggtt ctcatagagc gcggttccgt 360
aagcggttct tcgcaaggtc ggggttgcat accgaactcg cgaaacgtcg gcgactgagc 420
tcccgaggcg cgttgacaag atgccacgaa gggaatggaa gacagccgat attgcaattg 480
tcttcgtgga ctgctttcgg gacgtaaggc gcaagccatc atcaccgccg tcctaaacaa 540
acatacctcc acacaaattt atctacctga ccacaagata tatcctgtca cacgatttat 600
taaacgctgc acttggctag aactagtgga tccgcggccg catgcctgca ggtcgactct 660
agttgattaa ttgtttaggc tttattatat attcatcata agtctttttt gtagccattt 720
aggtttgttt tcgtcgaatt aatcttataa acactatttt tattcgtaaa ttccgttgct 780
ttcttattta ctttatatca atgctttaaa acatcaatct agtttttaaa aatcaatata 840
tatgtttgca cacaccatta ttatcgtatg ttactctatc tattactgac aaacgttatg 900
aaattttatt atatttgtaa ttatcttttg cagttttgtc atttaaaatc gtttttctta 960
aaagaattat gttgttattt taaaattttg gctaaagaat cacgtggaga attagatata 1020
tcaaaccttt catctttgag atgaaagatt acatcaatta ctattaacta agcttacttt 1080
gataaattaa aatcatatta aaacaaatag tccgtaaaag aatataattt tgaaaaacta 1140
aacagtcatc aaacaacgcg tgttagcttt taatatatat tatgatatgt taagtgaaaa 1200
taaagttgaa gtgtatgaag ccaaaagaga agtcgttttc acttgttgag ttctaatttc 1260
taggatggtt ctatgtaaag tacttcctct tccaaaattg gaatccaact cactacttat 1320
aaacatcatt tattcgtcat cttaattaca ataccaactc ttatttttgt ctcatctatc 1380
atcacactca ctaattaaca ttaccattat cttatatcat tttatgaact cattatttaa 1440
caaataaatc acttaaaagt ttaacttcaa aaaaaaaaag gaagaaagaa agaaggtttg 1500
aaattacact atttgcaatt aattatgttt tatgaaaact ttctaatact ttaattttat 1560
gtcgaatcgt ttgtcgaatc gtttctcttt tatcctacta caaaaatatt ataaaatgat 1620
tataaatggc taaaatatat agtatgtgta tttcataaat ttaagaaaat gttttcgaat 1680
acagtcaaat gaactaaaat atttacaaaa atataacaaa atttcatatg tatatcgaat 1740
aaaatttaaa aatttgaaga ctaaatttgt aatataatta catattaaag taatttttag 1800
atgtgtgggt attatataat aataatgttg ggaaggtgag ggcatgaggc agctggaggg 1860
ataaggacta gggattgttt tatatccttt ttcacattta atttttgatg ctaattaatt 1920

tgttgccaat catttcatca cttttttttt tttttttggt tctaatttat ttactttata 1980
tggaaaataa ataaaagaaa aatgaaagaa agaaaaaagt ggttttcaaa tcaatagaaa 2040
aaacaaacaa ctccaacttt aatggcttga aaacaaatgc attctaaaat taaaccttat 2100
gattgatttg atttttattc ccctttttta cacttttcat tttcatcata attatatctt 2160
cagttacctg tccaccaatt acaccatcaa atgtggatta ttgggattct tttttttttt 2220
tttaagatta tcttacggct ttcatttttt tcgtatcttt atgacggttt gatagacgta 2280
aaagtggtta ttgtgttata gagatttgta ttattttgat attatggaag gattcgtttg 2340
agtaaaatta taaaaatcag aggggtgtcg tttaaaaatg taagtaatcc aacacaaaaa 2400
aataattatc ataaaatgta aaaaaaaggg ttagattgaa aacaaacgaa acaaatgagt 2460
tttgtattat aaatcgacct aaaatgttca acccaaacat ggattacgat acgaccgatt 2520
catctcatta cagctcatcg atcctaaaaa tgtgaagaga agtattggat ataattatta 2580
cttaaaaaga taatagaaaa aggaaatcag caaaattagg gttctttaat aagttataaa 2640
actcatttat atacaaaatt aattacatta caaaaggtgg gaatgtggat ttagacatac 2700
aacctataat aattaattaa aaacaataca catgtttcac aatttgagat aattaaattt 2760
taatccccat ttgataagta atgattttat cttataaatt agtttgttag gtctatactt 2820
tatttgttta tttatttatt cttactcttt tttaattata tttttactta tatcccaagc 2880
ttcattaacg attaatctaa gtttgaaatg attaattaca aaatagtagt ctattttgat 2940
ctatcacgga ctattgtggg tattttataa tattttgtta tattttataa atatttttag 3000
ttcattttgc tatatttgaa aataaccata tattatttat attttatttt tctagaaaat 3060
attttccata aactcaagtt ctatatttaa aatatatatt caaaagtttc ctattacaac 3120
cctaagttga atacttatag aattgtaata aaataagata attaactaaa taagtctaat 3180
taaacactaa taatttgaat taacaacact aaacaaatat tgtcaacaaa acttagttca 3240
attgacatct atatgaagaa tcgagttcca aatcttcaca cctgaacatt aacaaaatta 3300
tatagtaatt atcttaatta attctcctca tcgataaagt gaatatctaa ttaaaaattt 3360
aaagtcaaaa gtgtgaattt cttgaaatat caaattaaga caaaattcaa atcaatttga 3420
aaacatataa acaaaatggt aaattagaca aaaaaaaaaa atcctaaaaa ctacatatga 3480
aaaggttcat taccaaagaa gtttttccat gaaaaaaaaa aagaaagaaa gagaataaaa 3540
tattatatat agttaataat tatgaaattt ttgtataatc ccataaagtt tgcaactaaa 3600
cttaagcata tagtttatga cataattaag gtcactaata atagagaaca gttagagcaa 3660
aggtcaaaca tccactttat tcactctctc tcaatcatac aaagagattt aattgaatct 3720
actcattaca aaatccccaa tcttataata atattaatat cattaatctc ttatatatat 3780

atataatata tatacatata ttatctcatg cacatggatt ttcatgatct tcaaacccca 3840
cgtcgttgat tttccataaa acctatatat tccactaatc atttatattc attttttttt 3900
ttgggtctaa ttttaaacta tatgttttaa aactccatag tttgatcaat tcaaaaaaaa 3960
aaaaaaaaaa aagtgagtta tacaattttt aaaattttta ggacataatc ttgacaagta 4020
tctttatctc tcctacatga aagagggagc ataagattag cttgacattg tctaaaattg 4080
gaagtgtata tatatatata tatatatcta taaatttaga aattaaaata atggggtttt 4140
ttcatgaaat atatattaat agcttgatta aggaaggttt agagggtgat taaagtgcaa 4200
taatattgtt gattaattgt tttttttttc ttatgtgtat cttagtttca aggactcatg 4260
tttttttttt ctttttcttt tttggtccca tggaagagaa cttttttttca attataggat 4320
ttgggttttt agtttttggg aattattgaa aagttataat ttctgttgct aatgatggga 4380
aaattatgaa aaattatata tgcatgggtt ggtggggtca taagatctca aagaagcttt 4440
tattttgtca ttatttttct ttagaaaatc agaatcttaa tctttttttt tttacacatt 4500
ggtattttgg tcccctctcg tccaacccaa atttaaaaaa gatcaaaaaa gaaaaaaaaa 4560
aaaaaaagaa acagaaacct aatcttaaat caatttccac tatgcaatcc ttaattgtca 4620
tgttgatata aaaaaaaata gtaacgaggc aggagattga accataaaac ttagctttgt 4680
ggttattaat acacttagat gatgctaatt gagttaaact cttgattgac aattaaaaga 4740
aaagttaaat cattagttaa ataattaaag tttaatgatc ataagttaat atttgatgtt 4800
gggtattaat aaaggagatg catttgacta aaaaaatgat taggtagaga ctagggtaat 4860
taataaccaa tattaataaa gtatggttat gggggaattc atgacaaact caagagggga 4920
tgttcatttg ggtcttaatg aagtgtagga attcaaataa tttaaaaagt tattaataat 4980
tattatgatt ttattattat tatttcattt gggtctacat aagtataaag aattgattaa 5040
agaggttgat tatgcagaaa gaagggtgat tagagaagta caattatgaa gggattttgg 5100
ataaacacat aggaacgaat gattttcatt gggggcctta acaaataata ttcaatttta 5160
aaaaaattga ctatttgcaa ttaggtcttg atcatgaaga tcctcgagat aaattatagt 5220
tttttctttt tttcttcgca tatgaatttg ttcgatataa cgaattttcc gacatatctt 5280
acgtacactg ataagatatt gtctgcttag gatctatact tgtgatttat tctattatct 5340
aatcaatgtg agattttggt ctcattccta acaattctct gctaattaat tgaacaaagg 5400
acgatcactg aggctccatt caaataggaa ctcttatatc taggttaatt actatgctac 5460
attagaacat atcacctatc tgatagagtt caaacacata tcacaccatg agtactactt 5520
tttgaggcta agctccacta catctttgtt tgacacccaa atactctatc tacacgacta 5580

ggttaggagc ataaactttg ataccatctc tttgagacat aaactcccgt cactttattt 5640
ttcatttcat tgatctaaaa cgtcttatac caatagagat agttgttttc acatatatat 5700
acttatatta tcctattgcc tagtgaatct ttatacaaag caacatactt taattttgat 5760
taaacaaaga gtgattacac atggagatca tagcctaatt aaataattaa agtataatta 5820
tagggaggga ttttgagaga aatgtaattc aacaaggatt ttgcataagg gtcttagata 5880
aggaactaaa caactagaaa aaaaaatata atatatatat atataaaagg gaaatgaaat 5940
caaagaaagc atccattctc catatatata aaaatacata tatatatatg gggaagagag 6000
aagagattac aaaactaatt taataataag gtagttgagg gggcaaaaag caaaatacaa 6060
gagattttga tttttgagag aagccctttt tagcaaaaaa aataaaatag attaatataa 6120
cacacaaaca cacacctact ccttttcttc aaccaccaga ttcgattttg cctctctctc 6180
tctctctctc tctctctctc tctgtggatc ttaaacccca attcaaaata tgatgacaaa 6240
ttattaatta ttattcctcc aaaaatattt tccctattaa aaaaaatacc aagagagaga 6300
aaattcaatg attgtttttt ctcttttaca ttatttttct tttaaagaaa aaaacttgct 6360
ataaatagag gtgcccattg taagagcaac attcaattca acaaatcttc agttcaattt 6420
ctctcttttt ggctctcaaa aagggaaaga aaaaaaaatc attattatta ttatttcatt 6480
ttctttcttt cccttaaatt tgagctgaag gaaaaaaaaa aaaaaaaaat caatggcgat 6540
tgagattgat attgagcaaa atccaacggt tgaactttcg cgaatcggaa catcagaaac 6600
acacggcgaa gattcgccgt attttgctgg ctggaaagcg tatgatgaag atccttataa 6660
tgaatcaaca aatccttctg gtgttattca aatgggctta gctgaaaatc aagtaagaat 6720
atataacttt tttttgtttt gttttgcttt gtaaggagat tgggtttttt tttttaattg 6780
ggtttgtgtt ggaatttatg aaacaggtgt catttgactt attggaggaa tatttggagg 6840
aaaattgtga gggagaaggg aattatttaa attctgggtt tagagaaaat gctttatttc 6900
aagactatca tggtcttttc tcatttagaa gtgcaatggg aagttttatg gaagagatta 6960
gaggtggaag agcaaaattt gacccaaatc gagttgtttt aactgctggt gccactgctg 7020
ccaatgagct tctcactttc attcttgcaa atcctggcga tgctttgctt gtccccactc 7080
cttactatcc tgggtaagtt tatcatcacc tctacgtttt cgtatttcat ttcaaaaacc 7140
actctttact gtaattacta taccctcaga cattaaaatt ttaactttca aactattctt 7200
aaagtatgag tttgagggta tttcatatgg ggtttttaaa tgtaaattta tttacatttt 7260
tccactactt aagtgtccta tatttctact aatttcttct tgtgttgtac tcatattttc 7320
tatcgtgggg tggactacgt atttttacga gactattcgt ataacatacg aatgagtgct 7380
ttttaaacca aattcttcaa aatccaagtt taattttgga aactagaaaa tgggtagttt 7440

tttaaaatgt taccaaacgt gatctttatc cttacaatca aacattacca aggataattg 7500
caactaccgt tagactttat gagtgctttt ttttccaact gttctatatt tttacaacat 7560
tttgagttgt attcatcatt tctgttaaag atatttatat gtaactaagt atttttataa 7620
gacactgttg gtataatttc atgcactaat aatatagttt ctttttccag atttgacaga 7680
gatttgagat ggagaacagg agtgaaaatt gtaccaattc attgtgacag ttcaaacaat 7740
tttcaaataa ctccaaaagc attagaagaa gcttataatt cagcaatgga aatgaaaatc 7800
aaagtaagag gagttttaat cacaaatcca tcaaatccac tcggagcaac gatccaacgc 7860
tccacaatcg aagacattct agatttcgtt acacgcaaaa acatccacct cgtatccgac 7920
gaaatctatt ccggttccgt tttctcctcc gccgagttca caagcgtcgc tgaggttttg 7980
gaatcccgca gctacaaaaa cgccgaacgt gtccacatcg tttacagcct ctccaaagat 8040
ctcggccttc ccgggtttag aatcggcacg atctactcat acaacgataa agtcgtcaca 8100
accgctcgcc ggatgtctag ctttacgctt atctcttcac aaacgcaacg atttttagcg 8160
tccatgttgt cgaaccggaa gtttacggag aaatatatta aaatgaaccg ggacaggctc 8220
aagaaacggt atgaaatgat tattgaaggg ctgcgaaccg ccgggattga atgtttggaa 8280
gggaatgccg gtttgttttg ttggatgaat ttgagcccgt tgttgaaaga taaaaaaacc 8340
aaagaaggtg agattgagat atggaagagg attttgaagg aagtgaaatt gaatatttcg 8400
cccggttcgt cgtgtcattg ctctgaaccc ggttggttca gggtttgttt tgctaatatg 8460
agtgaaaaga ctctgcatgt tgcccttgat agaatacgtc ggttcatgga acggatgaag 8520
aaggaaaacg aagctaatta aatatatatc tatatataaa tatatgaaaa gaaaaaaaac 8580
atatgtagct tattttattt tatttttttt tacaatggtt gtgagaaaaa agaaaaaaga 8640
aaaaaaaaag aaaaaaaagc cattgtgatt cttttgtgtg gacactgccc aatatttgtt 8700
agaaatttgg ggtttttttgt cttcatttat acgtcatatt ttgatgattt aaactgagga 8760
aaaagaaaaa gaaatccttg ttttcttgct tttagcaaag caagttttat ttctcagttt 8820
tatatatata tatatataaa gtttctattt gtattgtcat ttttatgtga tatggaatat 8880
aattagtata attcgttctt gcaattaatt acctcgaaaa taaacgaaat acaagaaaaa 8940
gaaaaaaaaa atctcatgga gtattttagg gacaagtgtc aactcaggga gagagaaaaa 9000
aatatggttt aaatttaata gtattggtta ttttcataac atgctctaaa aaggaatata 9060
actaataatt tgactttaat taagaaaaga aaagctaata atatataatt aaaatcactt 9120
ttagcaacga ataacacttt gccgacttgt gtaattaacc acctaactat ccatctgacg 9180
tggaatgcaa gtaattaatt aattgatttt ttcgttttca aattttggtc aacttgattc 9240

ttggtactaa tttaatgttt ccatctgtca gaaagctaca acgtttttcc tccttctttc 9300
ttttttttaa gaattatttt aaaaagtcaa tacggtgcta taattagatt tttatttttc 9360
ctcttttta gtgtatatat atatttatat aagtagagat taggaactaa ttgattgaaa 9420
attaaatatg ctgtgacgct caaaagatat taatcccgcg ttggttttat gtatttaaaa 9480
aatgtatttt ttctttttg atatttttaa ataataaaat atttaaatta tttacaaaat 9540
ataacaaaat ttgctcgttt acatttttat tttgtgaagg acttatgcga tgtggttcga 9600
tctagaattc ttgtattttc aaaatagatg gagcttcttt ttggatgaat tctctctagg 9660
cttctgaagt caaaaatttt caacccaaga aaaaactaga gtttccttgt ggtatgaggt 9720
gtatgaaatt gactcattga ctcaattaca tggactttta tcatatttaa ctcagctaaa 9780
ttaagtttat tttttggaat taatctaagt aaataatatt taattgaacc aaaatattta 9840
atttgatcag tcataataaa gacatgtgac atcattggaa tcagtcaatt tgtgtttaaa 9900
tttaatttgg gatacatgtc aacttttagt taatctcaaa tgcaatttgt gattagttac 9960
aaaatttctt attcaacata cttcaaatct aaatttggta aattatgttt tttttaaaga 10020
aattagatca acacaaaaat ataatatgtt ttgtgaaaat gaaaattttg gtttaatggg 10080
aggagacaaa tttgaacgac aaattttctt agtaacttac gatatgatca ctaactaatt 10140
tattataggt tgggggtttg aacctctctc aactttgtgc tcattatata atatatcttt 10200
aaaagaccga ccttgcatta atgttgttgg ttagtctagt ggtagaatcg tcattctcta 10260
gctctctcta aattgttcca gcttcagttt ttatatactt ttttatatta tttttactga 10320
actataaaaa attactgtcg acaaaattta tgcttttatc acttaacata ataattgaac 10380
tatgtttgct ttgtttttttc ttttttaacg tatactatct caaagttttg gataatgtac 10440
gtgtttgaaa ttttgtaaca aacaaggata cataaatacg taattgttgt taattatttc 10500
aaaatgtaaa tagatgatat gatgtagggg ttgctaatat tattgtctaa ttatttttgt 10560
aaagaataaa ataaaataaa aatccatatg atgcctaagg acgtgtttag tattcaatat 10620
ggcgattatt atgtcttttt ttcaaatgga ttactttttg gatgatatga tatcttttat 10680
tttaattaaa actttttgat gacttttaaa atttaaaggg cataaaatat agctttcttt 10740
tgtacatata tatggttgtg cttggatttt gtatctgctt gtctttgtcc cgagtttctc 10800
gtcggtttga ccttcgatct actttttttg atatatatgt tccaatatcg atttctaagc 10860
taacatatgt aaaagatgat tgcactcgtg tggtgctgag actatcacac ttgatacttg 10920
atataagatt gtattattca tctgaaccaa taaaaattaa gatggatatt actcactctt 10980
ctcttttagg atcatcaaac aaggctcttt ttttgacaat cacctgaagt cacgagtaac 11040
tctacagtgt tatagttgtt tatagtattt gagactggaa atatcaaatt cagccaattc 11100

aattaaattt tataattgat atacattcat atgctcgaaa agttggcttc caaccgtcca 11160
acgctctaac tttgggacat gtgttacaaa acacataggt ttaaaaaaat acctaaaaac 11220
ataaaaaata caactttgat catcctttag ttcaatttta ttcccaattt gatacttttg 11280
aattccctaa ctagagaatt gtaattgtga ttgaacgttt ttatagtcaa aatttggatt 11340
tgatagctta taggacccca tgtgccaaac aaaataaaaa ttgcatatat atatatagag 11400
agagaggggg agttttatat tttaaaaaac aaaattagaa aaaggcaatt aattttgttc 11460
tttaaacgac atggtttctc acgtgttaaa cgtcattgtt atcaactgcg tcttgtcgct 11520
gaccaattca ttgacagcac agcaagcaaa aaagaaaaaa gaaaaaaaaa agtcaaaaag 11580
ttaatgtggt taaagaaagc gctttttgtg ataactcaaa aaaacaaacc aaccaatgga 11640
agccctccag gtcactctca tgtggctccc gtctccacgt catcaatgaa cacggacgtg 11700
tttacagctc aacaaacaac aactattcct ctgacgtggc aatttcttat taatgaattt 11760
ccaactctgc cctcctatta cattttctta aggtcggtga atgccgtaca atggaagctc 11820
ctagacatac tttctagttg tgatatatat atatatatat tattcatcca tcatatatta 11880
aactccaatg tatgtttgtg gattttacaa aagttaatat tatttggtag atgttgagat 11940
ttcttttttt tccaatgtct agtctttttc caagtttgga gaaagttttt tatgatgttg 12000
ggagttattt aatttcctag tggggccacg tagttaaata aatataggtt aaattttacg 12060
aaatatccat atatcgcgtg tgtggtatga tttcagttac gtcactattt tgaaaacata 12120
gttttcgtgt tcttattaat gcttatagtt gtaaataaca taattaaaag tatcatttgt 12180
taaaattgat gtcacaccgt atgtacataa tttatttatt gattgatgtt ataaggggcg 12240
ttggagatat cgttggaaaa aaatgatttg taaggatgat cttaattttc tataattgac 12300
tcacgtatat tatattgtat acgtttttca aaatttacac accaatcatc tcactttcgt 12360
tttcattttt cattttagtg gaaaacaatt caataaaaaa aaattctgac aactttttaa 12420
aatttaaggc acagttgaat caatccaacc gttcaagatt taaagaagaa aaaaactaat 12480
ttggttgctc cactttttgt ttttgttcgt tttggtccat taattctaaa aatgtttaat 12540
ttatttgtta tactttcaaa tcttcacaac tttaccgtat tgatccctta aaaatgaagt 12600
aaaaacaata atgaacgaac taagacaatc accatttgaa agtttaagga ccaaatgaac 12660
caaagttaaa agtatagaaa caaaaataaa catcgctaaa taaaccaaat aaaactagaa 12720
ttacttaatt gaaacaaaat aatatgaaat ggatcaaatc tttagacttt agtgtatggg 12780
aaagttctat gaaaatgacc accgactatc gagagaccaa ttttggggcc aagtcaatga 12840
ttggtaattt caacctacat ttatgatgta tgacaatgac aatagcttag gtcactttga 12900

aaatgactat aagattttct agttagagat atacacttga tattagactt ggtcgttgta 12960
ataaaaacta tgtgtcacgg atgatatatg ctaagtacat gttttagtct ttaatgtttg 13020
cgtatatttc tttacgtaat ttaatcttcg ttaattatat tttttaaact atgttttaat 13080
cttttaattc ttttgttgtg aaattgacaa ataaagagaa acacgacaat gtagatggta 13140
aacaatgaag tttgtgtagt ttattgacaa tatgttggca atgtttacga gtagagagat 13200
aattgttcaa attacagaac aataggtgac aatacgtgag tttttgtttt aatttacttt 13260
tgaaacttta ttttgatttt caaaacttaa agaagttgat actattattg ttttgagcta 13320
tgaagatgtg tgatcgaacc tttcacacgt ttagaatgaa agagcatgtc aattaatttt 13380
gagctaaact tgtttaaaaa aattgacctt ttgtctttgt tttaagtttt aacaaattaa 13440
tgatgtcatt gcgtaatttt aagtcagtta ggtatgaaaa gccaccatcg aagaaagaaa 13500
atttcaagaa gaaaagcaat gtagtaaatc acaaataatt gtttttcttt cccataggtt 13560
ataactataa aaaaaaaact tcttttttag tataataacg gtaaagaagg atgatcaaac 13620
cttctaactc agtcaattgc aaatatgata aattcacttt gacaaactaa aataaatttg 13680
aagatttatg aaacaaaatg tacattttaa aagtttatat tttcacacaa tgttagcttc 13740
cttttaaaaa aaaattaaat tttaaaagtt cagagaacaa aacatacatt tcaactttgc 13800
taacttcaat atagaactta tataaaatcg tgccacatag ggttcaaaag aactatagga 13860
ttttaaaatg aaaacatata ttttaattga gttttagaac taagttaata tatttattta 13920
taaagaataa cacttcagta aaattaagaa caacacagac atagttcaat aacataaaac 13980
tagctagagg atccccgggt accatggata tctagaattc gcggccgcaa gcttgatccc 14040
ccgggctgca ggaattcgat atcaagctag agatctagta acatagatga caccgcgcgc 14100
gataatttat cctagtttgc gcgctatatt ttgttttcta tcgcgtatta aatgtataat 14160
tgcgggactc taatcataaa aacccatctc ataaataacg tcatgcatta catgttaatt 14220
attacatgct taacgtaatt caacagaaat tagatgataa tcatcgcaag accggcaaca 14280
ggattcaatc ttaagaaact ttattgccaa atgtttgaac gatctgcagg tcgatcctag 14340
acgcgtgaga tcagatctcg gtgacgggca ggaccggacg gggcggtacc ggcaggctga 14400
agtccagctg ccagaaaccc acgtcatgcc agttcccgtg cttgaagccg gccgcccgca 14460
gcatgccgcg gggggcatat ccgagcgcct cgtgcatgcg cacgctcggg tcgttgggca 14520
gcccgatgac agcgaccacg ctcttgaagc cctgtgcctc cagggacttc agcaggtggg 14580
tgtagagcgt ggagcccagt cccgtccgct ggtggcgggg ggagacgtac acggtcgact 14640
cggccgtcca gtcgtaggcg ttgcgtgcct tccaggggcc cgcgtaggcg atgccggcga 14700
cctcgccgtc cacctcggcg acgagccagg gatagcgctc ccgcagacgg acgaggtcgt 14760

```
ccgtccactc ctgcggttcc tgcggctcgg tacggaagtt gaccgtgctt gtctcgatgt  14820
agtggttgac gatggtgcag accgccggca tgtccgcctc ggtggcacgg cggatgtcgg  14880
ccgggcgtcg ttctgggctc atggatcgac ctgcaggtct gtcctctcca aatgaaatga  14940
acttccttat atagaggaag ggtcttgcga aggatagtgg gattgtgcgt catcccttac  15000
gtcagtggag atatcacatc aatccacttg ctttgaagac gtggttggaa cgtcttcttt  15060
ttccacgatg ctcctcgtgg gtgggggtcc atctttggga ccactgtcgg cagaggcatc  15120
ttcaacgatg gcctttcctt tatcgcaatg atggcatttg taggagccac cttccttttc  15180
cactatcttc acaataaagt gacagatagc tgggcaatgg aatccgagga ggtttccgga  15240
tattaccctt tgttgaaaag tctcaattgc cctttggtct tctgagactg tatctttgat  15300
atttttggag tagacaagcg tgtcgtgctc caccatgttg acggatctct agcttatcga  15360
taccgtcggc tattggtaat aggacactgg gattcgtctt ggacaacttt ccttctcatc  15420
taagcgtaga caaccctcaa ctggaaacgg gccggactcc agggcgtgtg ccaggtgccc  15480
acggaatagt tttggccaga cccttgaaaa tccgattcag tacaatcgat tgccctcatt  15540
tttacgttgg catatatcct gccaaacagc caacaacgcg cgtgcggtga ataggaaagc  15600
gtttgagttg cttgctcata tcgtgacggt tgacagcaca ggttgaccgc ttgatgattc  15660
gtacgagccg ccaaacattg gctgtcgtaa tgatatacca tgtcagaaca gcaatccgat  15720
ggggcggaaa gcattatctt aatgcacacg gaaatggcgc gtcggtgggt ggaatacacc  15780
gacatagagg ccgtaagttc tgcatggtca tcgtcggaaa ggtggcagca ggcgcacggc  15840
tgtggcctct tgctctttca gcgtgaaatg cgtgttgaaa gaataatcga agagagcgtc  15900
cgctcgacac cttcaattat gccgatttga tcgatgaact gatcgagctc tgaaatcgaa  15960
ggggcttcga taatcgcaat caaatcaaaa gtgccactca cagaatgaag agcgataacg  16020
gccgtgacct tcccaaggga ggccgtcacc tgtgaaagcg ccttcgtaat ggtgatcaga  16080
atatgggctc gaaccaagct cgagacctcg aggggggccc cggtacccaa ttcgccctat  16140
agtgagtcgt attacaattc actggccgtc gttttac                           16177
```

<210> 10
<211> 7621
<212> DNA
<213> Cucumis melo

<400> 10

```
ttcttatcct ctctctctct cttgcctttt atagtagtgg ggttgggtta gatttttggc     60
catggtggaa agtatgaata agaaaagtag ggagtttgag ttattttgtt gtttgatatg    120
aagttggaca aagtgaatat acaatatatt gctatggaga aaaagaattg aggttgtaat    180
```

gttggaaaaa ggatcttatc tcattgttat gattatctag gccccaaata aaataatgga 240

gcatcttgaa agaagaaaag cactttttct tatttttttgg ctttttaact ttgctaatta 300

ttttaggaaa aatatgagtc agccaacccc atttcccaaa atccaattct ttttaataag 360

taatttctgt aagaggatga ttttaattat agcatttatt ttctttaaaa gaaaaggaaa 420

agtattttcg tcaatcattg actgaattca taatgctata agttgcttta catgatgatg 480

aatttgaata ccaatctaaa attatggttc tctgctttga atatttaagt aatagattag 540

attttaaacc tttttccatg aaatgttatt tactgatgtt actaattgaa tatattttga 600

tcctcacata attcagtaag accaaaaatg ggtgattaga aaatattatg taataaaact 660

aaaccaatta agttgtggaa gacagttcaa tttaaactat ggaaatagac tccacaaata 720

atagttatag gctaataaat aagaaatctt taaagaattt gaaaagtaag aatttaactt 780

attgcctaaa gtcttatcaa accttatctc aaaacttacg tacggaataa cataaatcat 840

ttcactttgg ttggaaagct gatattaaaa ttgggtatgt atgtttattt ttatttttat 900

tattgctatt aatattacta ttattattac tattattatt gttgttggtt ggacctaaat 960

tcaaaaatac ccctgaatta ataggatatt ttaaaataat tggtgaactt agagaagtgt 1020

tatttattat tattattatt atcattttaa tttatgttga tttctactgt atataatcta 1080

aaatttggga aaatattaat ttaaactttg aattaaatgt tttatcagca cattaactat 1140

tgcatttcat ttgaaaaaat tgtgtaaatt ttataataat ttaatgaact ttcataaata 1200

aatcaatttt ggcttttagt tataatcgta agaatcgtta cgtccatttt gtaaaattga 1260

cttttgaaca agtctacaca tgtatgaaag agaagcgata gtacgactat ttttaaagtt 1320

gatcataatt aatggtctaa aattcattga cttatgaaaa tttatgagtt taattaactc 1380

aaaccataaa tttcatgcgg agaaatgtat aaattgctga actttcaaat aaaatcagat 1440

aggagttata aatcgtaaag agagtcgtgg atgatattag gtgtcgtttt caactttatt 1500

gttattatta agattaacat gttggtattt atgaaacatg agcactgagg tgtttaggta 1560

gaaatttaaa tgaagaatta aggatggagg cagcaattag aaaagaaaga ttgaaaacac 1620

agagagacag agaagaaata tcgcaattag ttttcacatt aaaactagac attaccaata 1680

aatcataatt aatatcactt atcttttttct ttcctaactt ttgtgaatat tcgaatcaat 1740

ttacatacag tatgattaat tttacgggac tatttacatg atcttatgat tttttttttc 1800

ttttaacgct catgagtatt ttagttaact tacattcact tcgactaatc ttcgagaacc 1860

atgtacctga ttctccaacg tttaaaccta aaccttaata aaataattta tatatatata 1920

cagtgtttta tgtgtggaat tgtgtgtatt atggaggagt agggacaatg gtgagcgttg 1980

```
gcaaattaaa aaaaaaagaa gagtagtgga attaggcgac aaggaaacca agacgtgacc   2040
accaaagcaa atgttggcct tttcacactt ttcatttgat catttccaaa gaactatcaa   2100
aaaaattttc ttatcttctc ttttggcaaa tttggcaaat acctacggaa ttaccattac   2160
ggtctcctaa tacattctcc aatgttagtc acttactttg cccatttctt cccctttttc   2220
tcaactctca tctctttttt tcacacttgc acacttactt ctagtttatg ttctctcctt   2280
ttgcacgtga atttcaatcg acacttctaa tattatgtct ccgatccaat aaacgtgttg   2340
gaatttggaa gttaaaccga attagtgtat catctagtaa gatttgaaat agtatgtata   2400
gcttttcttt ctaaaaaatt ttaagaacga caagtcaaac ctcgttaaaa ccaaaaacgt   2460
ataacaaaga aaaatcatca tttaagactt ggtgacatct cattagggta aacacttttc   2520
caacatgcat cacgaccttc ctcctcaaac ataagaaata agaaaaacaa aacaaaacaa   2580
aattaacaca gcaaaataaa aaataaagtt acaaaattac aagaacagta gatgaatttg   2640
ttttatctac gtgaagaggg ttatgatgtt tttttaatat atatatatat atagtcttct   2700
tcacactatt caatgagtca tttgttcttt caaatcccaa aaattgtgat gtctctctca   2760
ccacacacac acaccctttg tttccccctc tcttttcttt ccccttctca ttctttagtc   2820
attatagcaa gtgatcttat tcttggcttt gtcctttatg ttaggagttt ctctttaatt   2880
attttatttt tatttctcca atctctctct atttctccct atattataag ggtgttgttg   2940
taaacagtcc tctcccatca aaaaaaaaaa aaaaaaaaaa aaggaaaaaa gaaaaaaaga   3000
tgggtgatcc taatctctcc atcaataata ataacaatac ttgttttagt ttatcccctt   3060
ttcaacatta ctcttcttct tcttcttctt cttcctcttc ttataactct cattatcatc   3120
atgatcatct cttctcactt tcttactcca atgataataa cagcaatacc ctaaaaaaca   3180
acatgactac ctctcattat aatccttctt cttcttctca agttcttctt cctcttttga   3240
gtttaagtcc tgcaagagta gaacaagatc atcatcatca tcatcatcaa aatattattg   3300
atcatgatca taatattatt gattatgatc aaaatgatgt tactgttgct ttgcatttag   3360
ggcttccaac tccttcctct tcttctaata ataattctga tttgattttg aggctttctt   3420
caactgagat ctcagatcaa gaagatcaca cccatcaatt gcaagaactt tcatctaata   3480
attctattgc tagtaatagt aatggagtta ataagggtca atattggatt ccaactccta   3540
ctcagattct cattggtcca actcaattct cttgccctct ttgcttcaag actttcaata   3600
gatacaacaa catgcaggta cttttttttt tttcctttca atttttatgt gtttattttg   3660
tcactttttt actcttttc ttttctttca tgatataatt ttactgaaaa agtggaaaaa    3720
aaattcacga taattttggt taatttattt ttttagtacc aatatatttg aaccattgat   3780
ttcaaagtcc tttaacacat acaaatgtga tttgagctat atatatatat atatatatat   3840
```

atatatatat atatatatac tctcttctgt ttttttaggg ttttggttgt tctttttata 3900
cgtatatatg gtcgtcaaca cgtacaaatg tcaatgtcaa tgttttcttc ttctacttct 3960
tttttttttt ttttttttga attttttcat gatgatatta tatattttttg gtgcatggtt 4020
taaaattatt gtagtttttg gggattttca aaagatcaga acaaatcttc atctctggtg 4080
tgtgtatttc agaatgtaaa atccctttct ttatgtgatt gaacaacaca cagcagccag 4140
tttgctgtat atttgacaca ttttgaactt cgtgtgttat ttaatcatct ttggcattcg 4200
tgtgaaatga aatctaactc taattttagt taactcaaac acaaatacat atatatttaa 4260
aaaaaaaatc cataacctga gtcagtagtt gcacctgatg aaatttcatt atatattatt 4320
tcttctaatt atatattcat gtttgttaat ttccaacaag tctaatgcaa atttggagat 4380
ttatattctt ggtatatttg tacttcaaca ctaaaaggaa tcccaagttt ttgagaacaa 4440
caaaagcttt tttttttttt tttttttttt tttttttttt ttttttgaac tctttgcttt 4500
tccaatttct ccctttgacc accataataa ttattgactc aggagggttt gatatcattt 4560
attaagttga aaactcctga atcttttgcc taaaaagaac tgatagagaa atgtgatgat 4620
atatctacaa gaaaaatttg taacaaagta actcaacttc ttcatcagat ttgccccctc 4680
attgtgcaac aactttatta taatataaca tgtttaattt caaacccttt actaattcat 4740
acacatgttt tgaaaaaata tcataatttt ttaaattaat taggatcatt atatagtatt 4800
atggatctgt atggaaaacc aagaatgtgg ttgatagtga tcgctcatca tcgtttaggg 4860
tgtggtttct tttttaagcg tacttgtaat tttgtcgttc actttctggt gtgtatgttg 4920
cggtggattc ttttaatcca taggttcact ttctggtgca tatcgaaaac taactctcat 4980
gtgtcattaa gtatataaat ttctataaaa aatacttaag gatatgaggg ttagtggtgt 5040
tgcctattgg aagattgatt ataccccttgt tggatatcct ttggtcttat ggaggattgc 5100
attgtgttgt tgtttcaaaa atgcttttta ttattaaaaa gaattcaaaa tttaattgaa 5160
aatcataagt ttttatcgtt taaaagaaaa tacctgagaa tacgaaggtt ggtcgtaaat 5220
ataaatacga atatctcaac taggtagaca cgtacattat tcattagtat actctcatcg 5280
tactttgtca actatagatt agagcattga cctatattcg tgttagcacg tgttcaacat 5340
tgcatgcatt gaaatggtag tctaagcttt tttattgtta cggtttcatt aattttatga 5400
ttaattaact gttgagttat attattatgc tgacatgttt agcattcgga ataacagatg 5460
catatgtggg ggcatggatc acaatacagg aaagggccac aatcactaag aggaacacaa 5520
ccaacagcaa tgttgaggct gccatgttac tgttgtgcaa taggatgcag aaacaacata 5580
gaccatccaa gatcaaagcc attaaaagac ttcagaacac ttcaaacaca ttacaaaaga 5640

aaacatggga tgaagccatt tacatgtaga aaatgtggga aagcttttgc tgttagagga 5700
gactggagaa cacatgagaa gaattgtggg aaactttggc attgcacatg tggctctgat 5760
tttaagcata aaaggtctct taaagatcat atcaaagcct ttggacttgg ccatgctgct 5820
tatgggattg atgatcatca taatcatcat cattcttttg ataatgaaga tgatgaccca 5880
gcttctgata ttgaaacatg actatcataa tatatataat ataattatga acattattat 5940
tgatatatcc ccaatgttct ttttttttcc cccctttta acaaatttca taccttattt 6000
tagctgtttg tactgttaaa ttggagttgt tttcaatcat tattgttctt atctttaatg 6060
agttcatgct agctaattta atatcaagct atatactcaa attaaaattg atattcattg 6120
ttcattttta ttttatttac atttagattt ggttgataga agttctaatg acgtcttttg 6180
cctatcaaca aaggtctttt cttgactttt tgtgttaaca aagatctaga ccacaatcgt 6240
ggtagtgatg gagttgagat ataatgagag tgtgaaaaaa tatgtcaacc tatttagaat 6300
atattagtgt gtctactaac cacctatcta tcatatttgc tttctaaaaa tagaattaca 6360
acttttcatt gaaacccata attttgaact tcttaccata taggtgagga atgtagttta 6420
ttacttccat ataattacaa aatgccaata caataatatg gtttaaatta tgataggcta 6480
tatgtgtatc atttaggtat aagaaataca attagatgtg taatataatg cttaattaca 6540
agattgtttg atttgaaatc aaactaattt ggttgaatga ttattggaaa ctatttggtt 6600
gaataataat ggtttccaaa ttaaaataaa agggcaaagc agcttaagct taattaacac 6660
gcaactgaaa cgattttaca caacaacaca acccctccat cttaaattct atacttgttg 6720
agatgtccca tcatgaccct catcacgatt caaaattaca cccaaaatgc ttatattcct 6780
cttgtactta gtatccatta ataaataaat aacatccgta ttatatttat actccatatc 6840
aaaacgtcta attctcatca ttccgtctct tatttgtgtg ccggcgcgtc ctatctatta 6900
tacctaagat ttaaaagcaa agaatatgta tacgtatatc aaataaaata gagcaacaaa 6960
agaattcaaa aaaaccaaaa gaaagtgata aaaagaacaa cgaagaggag ttttcaatct 7020
cacggttgaa catgttaata gaaaaattag ggtttttaat gtatcctaac tcatcgactt 7080
aatattttag gccaattaat caaaataaca tcatttttct tttaatgtaa ttaattatta 7140
gatgaatgga agattgggaa tatattaaat agaagagagg tttgaaggag tgagatggta 7200
aaaaaagaag ggtgtgtagg gattttcatt gagatgtcat ctcaaagggg aagaaggggt 7260
ttttaggtcc agaaagagga ctgttacaat agaaaaaatg gaacagttat agtagttatt 7320
agggggaaga aaaagggatt tttgaaaaga gtagaaggta ctccaaatga atggctttga 7380
tttaacacaa cactcaaaat aaccaataaa taaataatgc tattgttttc ttttcttctt 7440
aaatctataa tatatatata tatatatata tatatatata gtctcttaca atttcccagt 7500

gttctgtctt gcagcactaa taacaatctg tctgccattc agacatgatg gatccatgta 7560

atttcacctc tttttctttt tcttctctct tttcttttct tttcttttct ttttaaggtt 7620

t 7621

<210> 11
<211> 1062
<212> DNA
<213> Cucumis melo

<400> 11

atgggtgatc ctaatctctc catcaataat aataacaata cttgttttag tttatcccct 60

tttcaacatt actcttcttc ttcttcttct tcttcctctt cttataactc tcattatcat 120

catgatcatc tcttctcact ttcttactcc aatgataata acagcaatac cctaaaaaac 180

aacatgacta cctctcatta taatccttct tcttcttctc aagttcttct tcctcttttg 240

agtttaagtc ctgcaagagt agaacaagat catcatcatc atcatcatca aaatattatt 300

gatcatgatc ataatattat tgattatgat caaaatgatg ttactgttgc tttgcattta 360

gggcttccaa ctccttcctc ttcttctaat aataattctg atttgatttt gaggctttct 420

tcaactgaga tctcagatca agaagatcac acccatcaat tgcaagaact ttcatctaat 480

aattctattg ctagtaatag taatggagtt aataagggtc aatattggat tccaactcct 540

actcagattc tcattggtcc aactcaattc tcttgccctc tttgcttcaa gactttcaat 600

agatacaaca acatgcagat gcatatgtgg gggcatggat cacaatacag gaaagggcca 660

caatcactaa gaggaacaca accaacagca atgttgaggc tgccatgtta ctgttgtgca 720

ataggatgca gaaacaacat agaccatcca agatcaaagc cattaaaaga cttcagaaca 780

cttcaaacac attacaaaag aaaacatggg atgaagccat ttacatgtag aaaatgtggg 840

aaagcttttg ctgttagagg agactggaga acacatgaga agaattgtgg gaaactttgg 900

cattgcacat gtggctctga ttttaagcat aaaaggtctc ttaaagatca tatcaaagcc 960

tttggacttg gccatgctgc ttatgggatt gatgatcatc ataatcatca tcattctttt 1020

gataatgaag atgatgaccc agcttctgat attgaaacat ga 1062

<210> 12
<211> 353
<212> PRT
<213> Cucumis melo

<400> 12

Met Gly Asp Pro Asn Leu Ser Ile Asn Asn Asn Asn Asn Thr Cys Phe
1 5 10 15

```
Ser Leu Ser Pro Phe Gln His Tyr Ser Ser Ser Ser Ser Ser Ser Ser
            20                  25                  30

Ser Ser Tyr Asn Ser His Tyr His His Asp His Leu Phe Ser Leu Ser
        35                  40                  45

Tyr Ser Asn Asp Asn Asn Ser Asn Thr Leu Lys Asn Asn Met Thr Thr
    50                  55                  60

Ser His Tyr Asn Pro Ser Ser Ser Ser Gln Val Leu Leu Pro Leu Leu
65                  70                  75                  80

Ser Leu Ser Pro Ala Arg Val Glu Gln Asp His His His His His His
                85                  90                  95

Gln Asn Ile Ile Asp His Asp His Asn Ile Ile Asp Tyr Asp Gln Asn
            100                 105                 110

Asp Val Thr Val Ala Leu His Leu Gly Leu Pro Thr Pro Ser Ser Ser
        115                 120                 125

Ser Asn Asn Asn Ser Asp Leu Ile Leu Arg Leu Ser Ser Thr Glu Ile
    130                 135                 140

Ser Asp Gln Glu Asp His Thr His Gln Leu Gln Glu Leu Ser Ser Asn
145                 150                 155                 160

Asn Ser Ile Ala Ser Asn Ser Asn Gly Val Asn Lys Gly Gln Tyr Trp
                165                 170                 175

Ile Pro Thr Pro Thr Gln Ile Leu Ile Gly Pro Thr Gln Phe Ser Cys
            180                 185                 190

Pro Leu Cys Phe Lys Thr Phe Asn Arg Tyr Asn Asn Met Gln Met His
        195                 200                 205

Met Trp Gly His Gly Ser Gln Tyr Arg Lys Gly Pro Gln Ser Leu Arg
    210                 215                 220

Gly Thr Gln Pro Thr Ala Met Leu Arg Leu Pro Cys Tyr Cys Cys Ala
225                 230                 235                 240

Ile Gly Cys Arg Asn Asn Ile Asp His Pro Arg Ser Lys Pro Leu Lys
                245                 250                 255
```

```
Asp Phe Arg Thr Leu Gln Thr His Tyr Lys Arg Lys His Gly Met Lys
            260                 265                 270

Pro Phe Thr Cys Arg Lys Cys Gly Lys Ala Phe Ala Val Arg Gly Asp
        275                 280                 285

Trp Arg Thr His Glu Lys Asn Cys Gly Lys Leu Trp His Cys Thr Cys
    290                 295                 300

Gly Ser Asp Phe Lys His Lys Arg Ser Leu Lys Asp His Ile Lys Ala
305                 310                 315                 320

Phe Gly Leu Gly His Ala Ala Tyr Gly Ile Asp Asp His His Asn His
                325                 330                 335

His His Ser Phe Asp Asn Glu Asp Asp Asp Pro Ala Ser Asp Ile Glu
            340                 345                 350

Thr
```

<210> 13
<211> 2999
<212> DNA
<213> Cucumis melo

<400> 13

```
ttcttatcct ctctctctct cttgcctttt atagtagtgg ggttgggtta gatttttggc     60
catggtggaa agtatgaata agaaaagtag ggagtttgag ttattttgtt gtttgatatg    120
aagttggaca aagtgaatat acaatatatt gctatggaga aaaagaattg aggttgtaat    180
gttggaaaaa ggatcttatc tcattgttat gattatctag gccccaaata aaataatgga    240
gcatcttgaa agaagaaaag cactttttct tattttttgg ctttttaact ttgctaatta    300
ttttaggaaa aatatgagtc agccaacccc atttcccaaa atccaattct ttttaataag    360
taatttctgt aagaggatga ttttaattat agcatttatt ttctttaaaa gaaaaggaaa    420
agtattttcg tcaatcattg actgaattca taatgctata agttgcttta catgatgatg    480
aatttgaata ccaatctaaa attatggttc tctgctttga atatttaagt aatagattag    540
attttaaacc tttttccatg aaatgttatt tactgatgtt actaattgaa tatattttga    600
tcctcacata attcagtaag accaaaaatg ggtgattaga aaatattatg taataaaact    660
aaaccaatta agttgtggaa gacagttcaa tttaaactat ggaaatagac tccacaaata    720
atagttatag gctaataaat aagaaatctt taaagaattt gaaaagtaag aatttaactt    780
attgcctaaa gtcttatcaa accttatctc aaaacttacg tacggaataa cataaatcat    840
```

```
ttcactttgg ttggaaagct gatattaaaa ttgggtatgt atgtttattt ttatttttat    900
tattgctatt aatattacta ttattattac tattattatt gttgttggtt ggacctaaat    960
tcaaaaatac ccctgaatta ataggatatt tttaaaataat tggtgaactt agagaagtgt   1020
tatttattat tattattatt atcattttaa tttatgttga tttctactgt atataatcta   1080
aaatttggga aaatattaat ttaaactttg aattaaatgt tttatcagca cattaactat   1140
tgcatttcat ttgaaaaaat tgtgtaaatt tttataataat ttaatgaact ttcataaata   1200
aatcaatttt ggcttttagt tataatcgta agaatcgtta cgtccatttt gtaaaattga   1260
cttttgaaca agtctacaca tgtatgaaag agaagcgata gtacgactat ttttaaagtt   1320
gatcataatt aatggtctaa aattcattga cttatgaaaa tttatgagtt taattaactc   1380
aaaccataaa tttcatgcgg agaaatgtat aaattgctga actttcaaat aaaatcagat   1440
aggagttata aatcgtaaag agagtcgtgg atgatattag gtgtcgtttt caactttatt   1500
gttattatta agattaacat gttggtattt atgaaacatg agcactgagg tgtttaggta   1560
gaaatttaaa tgaagaatta aggatggagg cagcaattag aaaagaaaga ttgaaaacac   1620
agagagacag agaagaaata tcgcaattag ttttcacatt aaaactagac attaccaata   1680
aatcataatt aatatcactt atctttttct ttcctaactt ttgtgaatat tcgaatcaat   1740
ttacatacag tatgattaat tttacgggac tatttacatg atcttatgat tttttttttc   1800
ttttaacgct catgagtatt ttagttaact tacattcact tcgactaatc ttcgagaacc   1860
atgtacctga ttctccaacg tttaaaccta aaccttaata aaataattta tatatatata   1920
cagtgtttta tgtgtggaat tgtgtgtatt atggaggagt agggacaatg gtgagcgttg   1980
gcaaattaaa aaaaaaagaa gagtagtgga attaggcgac aaggaaacca agacgtgacc   2040
accaaagcaa atgttggcct tttcacactt ttcatttgat catttccaaa gaactatcaa   2100
aaaaattttc ttatcttctc ttttggcaaa tttggcaaat acctacggaa ttaccattac   2160
ggtctcctaa tacattctcc aatgttagtc acttactttg cccatttctt cccctttttc   2220
tcaactctca tctctttttt tcacacttgc acacttactt ctagtttatg ttctctcctt   2280
ttgcacgtga atttcaatcg acacttctaa tattatgtct ccgatccaat aaacgtgttg   2340
gaatttggaa gttaaaccga attagtgtat catctagtaa gatttgaaat agtatgtata   2400
gcttttcttt ctaaaaaatt ttaagaacga caagtcaaac ctcgttaaaa ccaaaaacgt   2460
ataacaaaga aaaatcatca tttaagactt ggtgacatct cattagggta aacacttttc   2520
caacatgcat cacgaccttc ctcctcaaac ataagaaata agaaaaacaa aacaaaacaa   2580
aattaacaca gcaaaataaa aaataaagtt acaaaattac aagaacagta gatgaatttg   2640
```

```
tttatctac gtgaagaggg ttatgatgtt tttttaatat atatatatat atagtcttct   2700
tcacactatt caatgagtca tttgttcttt caaatcccaa aaattgtgat gtctctctca   2760
ccacacacac acaccctttg tttccccctc tcttttcttt ccccttctca ttctttagtc   2820
attatagcaa gtgatcttat tcttggcttt gtcctttatg ttaggagttt ctctttaatt   2880
attttatttt tatttctcca atctctctct atttctccct atattataag ggtgttgttg   2940
taaacagtcc tctcccatca aaaaaaaaaa aaaaaaaaaa aaggaaaaaa gaaaaaaag    2999
```

<210> 14
<211> 8255
<212> DNA
<213> Cucumis melo

<400> 14

```
atttttgagta ggggtgtaat tgggtcgggt cgggtcgggt cttttttttt tttttttttt     60
tttttttttt tttttttttt tcctccttcc ttagcaagga ggagattttc acaaggggga    120
gaaaatgttc cggggagtgc atcatttgaa caatacttga atagaaatgt agatcatcat    180
aactttttgt aagtgcattt tccctatgtt agtacatact catcaagaag atgttaccat    240
gaaatcaatt ctatatccat agttgtcatt cattttggtc cttacttgtc atcaaaccaa    300
gcataagata ttttgtttac aaaaactgca cataactgct gtcaattaaa tattaaccga    360
aaataggaaa caagaaatga gaccaaaagc acggatatac cgcactacac ttgctacatc    420
aaggtcgtct ttacttatta caaagtgaag gattaaaagt ttcatttcac agtataactg    480
attagggaag ggagctaatt cagaaaactg acggtatgtc tgctaaaatt tttgaagttc    540
tctaagccat tggaaagatc tcctcaacag atgaacgtaa tggtactaca taaaatctta    600
aattaaactg ccttcacctt tatgccacag atacagctgc aactacaata catcagtaaa    660
gcccattatc aactatggga tgaagtcttg tgtagcaatt attctgtagg aaaacttatc    720
ttataagcta tgacactaac agcacaagta taggcaattc aagtgccatc aagaggaaag    780
agcagacgga tccaagcaga atccagagcc acagttatgt gcacgagaaa gacaggaaag    840
gcacaaagaa aaatgaaaaa tactaaaatg ttatgttcac actctacttg taagcatgct    900
gcagggaatc tatagtattt atttaatatt caagaatgct ataacaatag ctggagacct    960
gcaataaata agtgtaacct attagagagg atggttaaac aaatctaaac ttctgtcata   1020
tgggaaagga taacaaatac aactaattca aacctatttt ttcctgacat gcagtgcacc   1080
agaacccgag ccttgtctct cacattgctc tgaaaatcac aagcaatatc aggaaagtta   1140
gacttttgga ataagtgaat gatagaaagc taatgacacg ttcttaacca aaagtcattt   1200
gttcagtgtc atcttatagt tcatttggga gaatataaag aaaggccacc tagaaattca   1260
```

actgcgtcat caaaaggtaa agtcttatca tattggagac agtgatacgt gaaggagttc 1320
ttgtagagat tttggcaagc aggcacagtc tgcaaaaata gtgtggagaa aaccaaatgt 1380
cagtcattta ttttcagatc acccaataca ttttagaggc aagtgatgat gcatgacttc 1440
tacaacatcc gagagagata accatgatat gtattctaca atgaaactgc tttttagtat 1500
aaccatcaac ccaataaaac ttatttttat atctacgaaa gccaacaact tgatatcatt 1560
gcattcaata tagattatac atcataatag attagaaaaa tagttataag cataaaatac 1620
tcagagtcca aggtaacctg agtctccagg caattcattc tggcatttgc catattacca 1680
aaaccttaat aggctagtag tacagtccat ttacggcaat tatgtgctgg tgctgttatt 1740
tgttagtatc agagctttca atttataaat aaaatccata agcaggctaa aagcaagatt 1800
ccaagatgta acgaagccgt aagcataaat cagaatagta gtcacacctg ggcagcatac 1860
gtccgttgga atggaaatta cattggcctc aaatattcaa gtagtgaact taaggacttt 1920
gacaattacg aataaagaag cagaacctga caaaatgtga tccaggatct gtaaatatac 1980
ctttgaaata ccatatccct attgcttttg cttcctgtcc ccaactttga acccaattca 2040
ttctcttggg tattgctaga tttagcagaa acaacatcag ctacttgatt agccaccgga 2100
gccttgccaa cctcgcccgc attcgctaaa tccatgtttc agatagattg aaagtagaga 2160
gtataaaaga taacaaagga atttggaaaa cactggggaa cttttacaaa cgcatggaag 2220
acaataaaaa ccaaaccaat aatcagtaag ttaccggttc aacgaaacca ccatgaaatc 2280
aactaaaaca aaaaacaaaa caaacaaaca accaataatc tctcagccct tcttttgttt 2340
cttttatata aagaaggtta accaaggaac ctcatgaaca aagtttacaa ttgggaaggt 2400
atgaattaat gaacatcttc acgagaccac cttgttcgta taacccaaag tgcttccatc 2460
attgagcttc aggactctaa aaaaacaaga ttcgatattt aataagttaa taattttaaa 2520
aaaatgtaaa taaagatttg agagtaagga tttaaaccat agagtcatta ttcaaattct 2580
caaatgcagc ttccatctcc tttcctatgt ttatgaattc tagatcaaaa tgacaaacag 2640
ttagcttata tgggataatt gaaaatttta aatgcgtact tcaaaaagaa tattaccttc 2700
atcaatttct tcagccccgt caatttcttc agtcatgtca tccaaaggtt tagactgaat 2760
ccaattctga gcacaaatga gtgcctctgc agtttgagga gttaaagaac ttcgaaaaga 2820
atctaacacc cgtcctccag tgctaaaggc ggactcagaa ggcacagttg atataggaat 2880
actgtagatg tccctagcta cttggctaat gatcttaaat cgagaggcat tcaccttcca 2940
ccaagttagc aaatctaaat attcatcgcc catacaatct atacgagcct catccagata 3000
acgagtcacc tctgttttag catcatctag acatgttttg ttactttgtt taaatctatc 3060
atgaacagta gcacgtgcct tgtaagatcc actagatgag atagaaggta tttcactttg 3120

actttgaaag ccaaatcctt cgataggtgt acatgattgt gtttgtgaat atttttcttt 3180
tgacattctc atataataat catcacacaa tcgacgaaat gcttcttcaa ccttatttgt 3240
ccatattttt gcacaatctt cctccaaaaa ttcattaaaa caataattca cataagctag 3300
cttgtatcta gggtcaagaa ctacagaaac atacaataat aaattggtct tctcacttgt 3360
agttataccc caatacttgt tgaattttgt ctgcatgctt aatgtcattt gactcaataa 3420
tgcattctca tacgatgagt attcacgaat tatttcttgg atcaaacaaa gttcatgaaa 3480
aaatatattt gaagtcacag acatagatgc agaaaacttc attgttacct ctgaaaaagt 3540
ctttaggaac tttacaaaca cttttgcatt atcccaatct tcagtagtag gaatatcatc 3600
ctttggcaaa taactagggt catgctcctc caatctttca aaagtctttt gacacttaat 3660
tgctccatcc aacatagtaa aagtagaatt ccatcgtgtc ggaacatcca ttgtaagaca 3720
atttttgtt gacatcttat cttctttagc aaaatcttta aatatttgca atctagcagg 3780
agatgaccta acatacttca cagcatttct gattcgaatg atagacacat gcaaatcttt 3840
taaggcatca ctaacaatta aattaagaat atgagcacaa catctaatgt gaataaattc 3900
accatccaac accaacccat ttctgccttt aaacttttta accaagtagg caatggctac 3960
atcatttgaa ctcgcattat caaccgttac agtaaagagc ctatcaatac cccaaccttc 4020
taagcacttt tcaatggctc tacctatggt atctccttta tgattagcta cttgacaaaa 4080
gttcaaaatt cttttgtgca agttccaatc atcatcaatg aaatgagccg ttataaccat 4140
ataattaata ttttgcacag aagtccacgt atccgttgtt aaacaaactc tttggccact 4200
tcgagttaat gcatttttta acttttttt ctccttcata tacatttgaa aacaatcttt 4260
tgcaacagtt actcttgatg gaatcacaaa ctttggattt aatgcccgac aaaattggtg 4320
aaacccttca ctttctacga acttaaatgg caattcatcc aagataacca tcctagcaag 4380
catttttcta caattttctt gagtgaatga tgcagtcatc aaattacttt cagaatctcc 4440
ctctccttca acattatctt ccaatggatt tacatacatc ttacatttct ctaaatgtct 4500
ttttaaatta gtggtaccat ttcttttgga atcacaagca tatgaagccc cacaatgttt 4560
acaagcagcc ctaggatatt taggatcaca tccttctact tttataaaat gttcccatac 4620
cgatgatggt ggtttaaccg gttttctttt tcctaacact ggactagaac aactctgatt 4680
tgaagtctcg tctaccgaga atgaagtcat cctaataatc atcaaacaaa acacataaaa 4740
aaaatatcaa tttttatggc ttattaactt catgcacagc agactcaacg actaaacaac 4800
tccaccccta aaaagatata tggactgaaa taatatttta aatatcccta aactatgtaa 4860
actaggagaa cctaactgta atttaatttc tttgatgcaa ctaacccaaa ataaatttat 4920

tacacaataa tgcaagatta gtacaatttt ttaggtcgag tttgattaga tgttttcaag 4980

ttcatgaacc tgtttaagtt ttagcaactt aatgtgatcg aagactgttg gtttctatct 5040

tcaagacttt atccatataa ggttaaacat atgacacact cccacccaat caccactaca 5100

tcttccactc atatttgata atttttttta gacaatttga acagaaaaaa attggtagct 5160

gttatttatc atatttgata aacatttttt tagataattt gaaccaataa ttgaagcata 5220

atcaagttgg acaaagttcg agagctatca aactagaaaa gagagataga aaactcgaaa 5280

caaaaccatt ttagatagga ttaattagcg aggttactag ttgttaaata ttaaataatc 5340

aaagcctacc ttcgagatcg ggatctcaac tatgtgcaac atcgccaaga cctaagattt 5400

ggaaagacta tttccaaaaa ccaacgtatt agtccttttg agtattcttt cttcacttac 5460

attctttgtc atagaaagat tcatagactt taaaatattt gagaaaggat gtcctaaacc 5520

tctaaaggcc aattaaataa aaatttctaa atataaaact ctaattgata gaacctacaa 5580

ctattgtaca taaaaatgaa caagaccgaa tccaccctct agagaacctt atgtttgctg 5640

gaaatgtata tggacttaaa tgtaaagcaa gatgaacata attcaaatta aaggtgcagc 5700

ctaatagatt caaaatccac taatttgctt acttaatcat aaaattcttc taaaaacatg 5760

caagattggt caatttaatt cttagtccat gtactcaaga aagcttaagt tggatacatg 5820

tcaattggaa aattaaggca ttttgcataa aagaaaatta agaaaaaaag aggtacattt 5880

gaaactattt agaaaaattg ggctattttt tttttttaaa catcaaattc ttaccaaacg 5940

agtggttagc aattacaatc ctggttgatc ccttctgttt tgcaccacaa gcaagagctt 6000

tgccaactcc accagcacca atgacaacaa cgagctgagc tttaccagct aagcatgaac 6060

cgactgtgga gctgtcgtta tgcaagcctg caatataaga gttgggtata aaccatcttg 6120

gttgaaaata atatttatga agaaacagaa gaaaaatatg catacaaacc aggtaatcca 6180

acttcaatgg ctgaaatagc accataacaa tctgtgttat agccacataa tttttcatca 6240

gattgtcttc tagttaagaa caaaaagtta acagctccaa ttggctgcaa aagtttaaaa 6300

ctctatcatc caagtattgt ggaaactaag aggtctatat atatctgggg gaaccttttg 6360

ttttaggatt caagacatgt agatagataa agcgtgtaga gccttacaat tttgttgaaa 6420

tatgatgaaa atttgtatat taatagctaa atgtgtttta tgatgtgaac cgtaccatcc 6480

tgatctacgg ctgaatgtag ttttatactg ctatatatct actaataatc catattacta 6540

actttttat aaaactaatc cctatattat ttactatcca atcaccgaca tgtttcttct 6600

tggtctgtag gcttatgata ttgatacact gtcagatgaa cagaggtatg ctatcaagga 6660

ccttgctgat ctgggactag ttaagcttca gcaggttttc ggctagcctc tgctgcttta 6720

ttgaatacga cctttgctca aagttttact ttattttaac tttacctttt tctttttctg 6780

ttgttccttt tgttgttgat ttgtccaggg tagaaaagag agttggttca tacctactaa 6840

attagccaca aatctttcaa tgagtttggc agattcctct tcaaggaaac aagtgagtgt 6900

ttctgaatgc aaattatttt tagattggta tatttctatt cagttccatt ttttttatgc 6960

ttaccaagtt tttagagggt gtttggctcg cgaagttgga gataagagag tgtttgtgcc 7020

caaggaaaac atggatccca caaactaaaa aaccaccaaa atttcatgca tcgttaactc 7080

ttatgatccc catgacttca caactcccta gacatcataa cttcactctt ggcctcaaac 7140

ccccttagtg ttttgtgtgt ttttgataat tgcttttcca taattatttt ttagttttat 7200

actgctatat atccgctccg cgatacttct tatgtaaatt ttatttgata gaatcttctt 7260

tctgcaatta aataattcgg ttgagtgaag tttttttttt ctcttggggg gggggggtt 7320

taattctcag tttagaggct ctatgaagtt gcttgctctt cctaattttc tttctgttac 7380

atgaccaggg atttgttgtt gtggagacaa atttcaggat gtatgcttat tcttcttcca 7440

aactacattg tgaaatatta cggtacatcg tgaggcagca gaagcaagct ggttatcaaa 7500

gttagtcaat ctattggcta gattaggcat ttagtaagtg ttgtgaaaat gagtcgatag 7560

cgtgaagttt gtgcagagga accccaaact gggaatgagt ataaagacaa ttataggcaa 7620

ttgctgaggc actggacaaa atgagctgta aactaaagac taagcttctc aaacagtctg 7680

gtatttttca atctacaaac aagaatttca tattatgtac acttaaacca gaacccataa 7740

acaagaaccc agatggcaat aaccccaaat aaacagaaaa aaaacccaaa ataatacaag 7800

ctgtaattta aaaattaaag agatgataat aaggagaaag ggacagacag aaagataaag 7860

caatatatgg gtcagtgtgg aacttacagg cgatgacgat ggagtggcga agtgatgaag 7920

acgaagagat gccgacgccg atgaacacga agagatagat gccgacgctg atgcagacga 7980

agaggtagat gccgacgcca aacgatggaa caaaatggtt tttgaagagg aggaagacgc 8040

cagacgaaat gctgaagact gaagaagaat cgaggagagg aaatgaatta tacctaatcg 8100

ggtattttaa acccgacccg acccgaaccg aattaaatcg ggtcggttcg gtccagtaac 8160

attgttgtta ctaaccgatc caacccaaac cgaaccgaat cgggtcgggt cgggtcatcg 8220

ggtcgggtcg gtttttttca cccctaattt tgagt 8255

<210> 15
<211> 15447
<212> DNA
<213> Cucumis melo

<400> 15

gctcggtacc cggggatctt atctcattgt tatgattatc taggccccaa ataaaataat 60

ggagcatctt gaaagaagaa aagcactttt tcttattttt tggcttttta actttgctaa 120

```
ttattttagg aaaaatatga gtcagccaac cccatttccc aaaatccaat ttttttttaa    180
taagtaattt ctgtaagagg atgattttaa ttatagcatt tattttcttt aaaagaaaag    240
gaaaagtatt ttcgtcaatc attgactgaa ttcataatgc tacaagttgc tttacatgat    300
gatgaatttg aataccaatc taaaattatg gttctctgct ttgaatattt aagtaataga    360
ttagattttt aacctttttc catgaaatgt tatttactga tgttactaat tgaatatatt    420
ttgatcctca cataattcag taagaccaaa aatgggtgat tagaaaatat tatgtaataa    480
aactaaacca attaagttgt ggaagacagt tcaatttaaa ctatggaaat agactccaca    540
aataatagtt ataggctaat aaataagaaa tctttaaaga atttgaaaag taagaattta    600
acttattgcc taaagtctta tcaaacctta tctcaaaact tacgtacgga ataacataaa    660
tcatttcact ttggttggaa agctgatatt aaaattgggt atgtatgttt atttttattt    720
ttattattgc tattaatatt actattatta ttactattac tattattatt gttgttggtt    780
ggacctaaat tcaaaaatac ccctgaatta ataggatatt ttaaaataat tggtgaactt    840
agagaagtgt tatttattat tattattatt atcattttaa tttatgttga tttctactgt    900
atataattta aaatttggga aaatattaat ttaaactttg aattatatgt tttatcagca    960
cattaactat tgcatttcat ttgaaaaaat tgtgtaaatt ttataataat ttaatgaact   1020
ttcataaata aatcaatttt ggcttttagt tataattgta agaatcgtta cgtccatttt   1080
gtaaaattga cttttgaaca agtctacaca tgtatgaaag agaagcgata gtacgactat   1140
ttttaaagtt gatcataatt aatggtctaa aattcattga cttatgaaaa ttcatgagtt   1200
taattaactc aaaccataaa tttcatgcgg agaaatgtat aaattgctga acttacaaat   1260
aaaatcagat aggagttata aatcgtaaag agagtcgtag atgatattag gtgtcgtttt   1320
caactttatt gttattatta agattaacat gttggtattt atgaaacatg agcactgagg   1380
tgtttaggta gaaatttaaa tgaagaatta aggatggagg cagcaattag aaaagaaaga   1440
ttgaaaacac agagagacag agaagaaata tcgcaattag ttttcacatt aaaactagac   1500
attaccaata aatcataatt aatatcactt atctttttct gaatattcga atcaatttac   1560
agtttgataa attttacggg actatttaca tgatcttatg atttttttct tttaacgctc   1620
atgagtattt tagttaactt acattcactt cgactaatct tcgaaaacca tgtacctgat   1680
tctctaacgt ttgaacctaa accttaataa aataatttac atatatatat acagtgtttt   1740
atgtgtggaa ttgtgtgtat tatggaggag tagggacaat ggtgagcgtt ggcaaattaa   1800
aaaaaaagaa gagtagtgga attaggcgac aaggaaacca agacgtgacc accaaagcaa   1860
atgttggcct tttcacactt ttcatttgat catttccaaa gaactatcaa aattcttttt   1920
```

cttatcttct cttttggcaa atttggcaaa tacctacgga attaccatta cggtctccta 1980
atacattctc caatgttaat cacttacttt gcccatttct tcccctttttt ctcaactctc 2040
atctcttttt ttcacacttg cacacttact tctagtttat gttctctcct tttgcacgtg 2100
aatttcaatc gacacttcta atattatgtc tccgatccaa taaacgtgtt ggaatttgga 2160
agttaaaccg aattagtgta tcatctagta agatttgaaa tggtatgtgt agcttttctt 2220
tctaaaaagt tttaagaacg acaagtcaaa cttcgttaaa accaaaaacg tataacaaag 2280
aaaaatcatc atttaagact tggtgacatc tcattagggt aaacactttt ccaacatgca 2340
tcacgacctt cctcctcaaa cataagaaat aagaaaaaca aaacaaaaca aaattaacac 2400
agcaaaataa aaaataaagt tacaaaatta caagaacagt agatgaattt gttttatcta 2460
cgtgggaaga gggttatgat gtttttttaa tatatatata tatagtcttc ttcacactat 2520
tcaatgagtc atttgttctt tcaaatccca aaaattgtga tgtctctctc accacacaca 2580
cacacccttt gtttccccct ctcttttctt tccccttctc attctttagt cattatagca 2640
agtgatctta ttcttggctt tgtcctttat gttaggagtt tctctttaat tattttattt 2700
ttatttctcc aatctctctc tatttctccc tatattataa gggtgttgtt gtaaacagtc 2760
ctctcccatc aaaaaaaaaa aaaaaaaaaa aaaaaaagga aaaaagaaaa aagatgggtg 2820
atcctaatct ctccatcaat aataataaca atacttgttt tagtttatcc ccttttcaac 2880
attactcttc ttcttcttct tcttcctctt cttataactc tcattatcat catgatcatc 2940
tcttctcact ttcttactcc aatgataata acagcaatac cctaaaaaac aacatgacta 3000
cctctcatta taatccttct tcttcttctc aagttcttct tcctcttttg agtttaagtc 3060
ctgcaagagt agaacaagat catcatcatc atcatcatca aaatattatt gatcatgatc 3120
ataatattat tgattatgat caaaatgatg ttactgttgc tttgcattta gggcttccaa 3180
ctccttcctc ttcttctaat aataattctg atttgatttt gaggctttct tcaactgaga 3240
tctcagatca agaagatcac acccatcaat tgcaagaact ttcatctaat aattctattg 3300
ctagtaatag taatggagtt aataagggtc aatattggat tccaactcct actcagattc 3360
tcattggtcc aactcaattc tcttgccctc tttgcttcaa gactttcaat agatacaaca 3420
acatgcaggt acttttttttt ttttcctttc aatttttatg tgtttatttt gtcacttttt 3480
tactcttttt cttttctttc atgatataat tttactgaaa aagtggaaaa aaaattcacg 3540
ataattttgg ttaatttatt tttttagtac caatatattt gaaccattga tttcaaagtc 3600
ctttaacaca tacaaatgtg atttgagcta tatatatata tatatatata tactctcttc 3660
tgttttttta gggttttggt tgttcttttt atacgtatat atggtcgtca acacgtacaa 3720
atgtcaatgt caatgttttc ttcttctact tttttttttt tttttttttt gaatttttc 3780

atgatgatat tatatatttt tggtgcatgg tttaaaatta ttgtagtttt tggggatttt 3840
caaaagatca gaacaaatct tcatctctgg tgtgtgtatt tcagaatgta aaatcccttt 3900
ctttatgtga ttgaacaaca cacagcagcc agtttgctgt atatttgaca cattttgaac 3960
ttcgtgtgtt atttaatcat ctttggcatt cgtgtgaaat gaaatctaac tctaatttta 4020
gtttacatat atatttaaaa aaaaaatcca taacctgagt cagtagttgc acctgatgaa 4080
atttcattat atattatttc ttctaattat atattcatgt ttgttaattt ccaacaagtc 4140
taatgcaaat ttggagattt atattcttgg tatatttgta cttcaacact aaaaggaatc 4200
ccaagttttt gagaacaaca aaagcttttt tctttttttt ttcttttttt tttttttttg 4260
aactctttgc ttttccaatt tctccctttg accaccataa taattattga ctcaggaggg 4320
tttgatatca tttattaagt tgaaaactcc tgaatctttt gcctaaaaag aactgataga 4380
gaaatgtgat gatatatcta caagaaaaat ttgtaacaaa gtaactcaac ttcttcatca 4440
gatttgcccc ctcattgtgc aacaacttta ttataatata acatgtttaa tttcaaaccc 4500
tttactaatt catacacatg ttttgaaaaa atatcataat tttttaaatt aattaggatc 4560
attatatagt attatggatc tgtatggaaa accaagaatg tagttgatag tgatcgctca 4620
tcatcgttta gggtgtggtt tctttttttaa gcgtacttgt aattttgtcg ttcactttct 4680
ggtgtgtatg ttgcggtaga ttcttttaat ccataggttc actttctggt gcatatcgaa 4740
aactaactct catgtgtcat taagtatata aatttctata aaaaatactt aaggatatga 4800
gggttagtgg tgttgcctat tggaagattg attataccct tgttggatat cctttggtct 4860
tatggaggat tgcattgtgt tgttgtttca aaaatgcttt ttattattaa aaagaattca 4920
aaatttaatt gaaaatcata agttttaatc gtttaaaaga aaatacctga gaatacgaag 4980
gttggtcgta aatataaata cgaatatctc aactaggtag acacgtacat tattcattag 5040
tatactctca tcgtactttg tcaactatag attagagcat tgacctatat tcgtgttagc 5100
acgtgttcaa cattgcatgc attgaaatgg tagtctaagc ttttttattg ttacggtttc 5160
attaatttta tgattaatta actgttgagt tatattatta tgctgacatg tttagcattc 5220
ggaataacag atgcatatgt gggggcatgg atcacaatac aggaaagggc cacaatcact 5280
aagaggaaca caaccaacag caatgttgag gctgccatgt tactgttgtg caataggatg 5340
cagaaacaac atagaccatc caagatcaaa gccattaaaa gacttcagaa cacttcaaac 5400
acattacaaa agaaaacatg ggatgaagcc atttacatgt agaaaatgtg ggaaagcttt 5460
tgctgttaga ggagactgga gaacacatga gaagaattgt gggaaacttt ggcattgcac 5520
atgtggctct gattttaagc ataaaaggtc tcttaaagat catatcaaag cctttggact 5580

```
tggccatgct gcttatggga ttgatgatca tcataatcat catcattctt ttgataatga   5640
agatgatgac ccagcttctg atattgaaac atgactatca taatatatat aatataatta   5700
tgaacattat tattgatata tccccaatgt tctttttttt tccctttttt aacaaatttc   5760
ataccttatt ttagctgttt gtactgttaa atcggagttg ttttcaatca ttattgttct   5820
tatctttaat gagttcatgc tagttaattt aatatcaagc tatatactca aattaaaatt   5880
gatattcatt gttcattttt attttattca catttagatt tggttgatag aagttctaat   5940
gacgtctttt gcctatcaac aaaggtcttt tcttgacttt ttttgtgtta acaaagatct   6000
agaccacaat cgtggtagtg atggagttga gatataatga gtgtgaaaaa atatgttaac   6060
ctatttagaa tatattagtg tgtctactaa ccacctatct atcatatttg ctttctaaaa   6120
atagaattgc aacttttcat tgaaacccat aattttgaac ttcttaccat aggtgagaaa   6180
tgtagtttat tacttccata taattacaaa atgccaatac agtaatatgg tttaaattat   6240
gataggctat atgtgtatca tttaggtata agaaatacaa ttagatgtgt aatataatgc   6300
ttaattacaa gattgtttga tttgaaatca aactaatttg gttgaatgat tattggaaac   6360
tatttggttg aataataatg gtgtccaaat taaaataaaa gggcaaagca gcttaagctt   6420
aattaacacg caactgaaac gattttacac aacacaaccc ctccatctta aattctatac   6480
ttgttgagat gtcccatcat gaccctcatc atgattcaaa attacaccca aaatgcttat   6540
attcctcttg tacttagtat ccattaataa ataaataaca tccatattat atttatactc   6600
catatcaaaa cgtctaattc tcatcattcc gtctcttatt cgtgtgcccg cgcgtcctat   6660
ctattatacc taagatttaa aagcaaagaa tatgtatacg tatatcaaat aaaatagagc   6720
aacaaaggaa ttcaaaaaaa ccaaaagaaa gtgataaaaa gaacaacgaa gaggagtttt   6780
caatctcacg gttgaacatg ttaatagaaa aattagggtt tttaatgtac cctaactcat   6840
cgacttaata ttttaggcca attaatcaaa ataacatcat ttttctttta atgtaattaa   6900
ttattagatg aatggaagat tgggaatata ttaaatagaa gagaggtttg aagaagtgag   6960
atggtaaaaa aagaagggtg tgtagggatt ttcattgaga tgtcatctca aaggggaaga   7020
aggggttttt aggtccagaa agaggactgt tacaatagaa aaaatggaac agttatagta   7080
gttattaggg ggaagaaaaa gggatttttg aaaagagtag aaggtactcc aaatgaatgg   7140
ctttgattta acacaacact caaaattagg ggtgaaaaaa accgacccga cccgatgacc   7200
cgacccgacc cgattcggtt cggtttgggt tggatcggtt agtaacaaca atgttactgg   7260
accgaaccga cccgatttaa ttcggttcgg gtcgggtcgg gtttaaaata cccgattagg   7320
tataattcat ttcctctcct cgattcttct tcagtcttca gcatttcgtc tggcgtcttc   7380
ctcctcttca aaaaccattt tgttccatcg tttggcgtcg gcatctacct cttcgtctgc   7440
```

atcagcgtcg gcatctatct cttcgtgttc atcggcgtcg gcatctcttc gtcttcatca 7500

cttcgccact ccatcgtcat cgcctgtaag ttccacactg acccatatat tgctttatct 7560

ttctgtctgt ccctttctcc ttattatcat ctctttaatt tttaaattac agcttgtatt 7620

attttgggtt ttttttctgt ttatttgggg ttattgccat ctgggttctt gtttatgggt 7680

tctggtttaa gtgtacataa tatgaaattc ttgtttgtag attgaaaaat accagactgt 7740

ttgagaagct tagtctttag tttacagctc attttgtcca gtgcctcagc aattgcctat 7800

aattgtcttt atactcattc ccagtttggg gttcctctgc acaaacttca cgctatcgac 7860

tcattttcac aacacttact aaatgcctaa tctagccaat agattgacta actttgataa 7920

ccagcttgct tctgctgcct cacgatgtac cgtaatattt cacaatgtag tttggaagaa 7980

gaataagcat acatcctgaa atttgtctcc acaacaacaa atccctggtc atgtaacaga 8040

aagaaaatta ggaagagcaa gcaacttcat agagcctcta aactgagaat taaacccccc 8100

cccccccaag agaaaaaaaa aacttcactc aaccgaatta tttaattgca gaaagaagat 8160

tctatcaaat aaaatttaca taagaagtat cgcggagcgg atatatagca gtataaaact 8220

aaaaaataat tatggaaaag caattatcaa aaacacacaa aacactaagg gggtttgagg 8280

ccaagagtga agttatgatg tctagggagt tgtgaagtca tggggatcat aagagttaac 8340

gatgcatgaa attttggtgg ttttttagtt tgtgggatcc atgttttcct tgggcacaaa 8400

cactctctta tctccaactt cgcgagccaa acaccctcta aaaacttggt aagcataaaa 8460

aaaatggaac tgaatagaaa tataccaatc taaaaataat ttgcattcag aaacactcac 8520

ttgtttcctt gaagaggaat ctgccaaact cattgaaaga tttgtggcta atttagtagg 8580

tatgaaccaa ctctcttttc taccctggac aaatcaacaa caaaaggaac aacagaaaaa 8640

gaaaaaggta aagttaaaat aaagtaaaac tttgagcaaa ggtcgtattc aataaagcag 8700

cagaggctag ccgaaaacct gctgaagctt aactagtccc agatcagcaa ggtccttgat 8760

agcatacctc tgttcatctg acagtgtatc aatatcataa gcctacagac caagaagaaa 8820

catgtcggtg attggatagt aaataatata gggattagtt ttataaaaaa gttagtaata 8880

tggattatta gtagatatat agcagtataa aactacattc agccgtagat caggatggta 8940

cggttcacat cataaaacac atttagctat taatatacaa attttcatca tatttcaaca 9000

aaattgtaag gctctacacg ctttatctat ctacatgtct tgaatcctaa aacaaaaggt 9060

tcccccagat atatatagac ctcttagttt ccacaatact tggatgatag agttttaaac 9120

ttttgcagcc aattggagct gttaactttt tgttcttaac tagaagacaa tctgatgaaa 9180

aattatgtgg ctataacaca gattgttatg gtgctatttc agccattgaa gttggattac 9240

ctggtttgta tgcatatttt tcttctgttt cttcataaat attattttca accaagatgg 9300
tttataccca actcttatat tgcaggcttg cataacgaca gctccacagt cggttcatgc 9360
ttagctggta aagctcagct cgttgttgtc attggtgctg gtggagttgg caaagctctt 9420
gcttgtggtg caaaacagaa gggatcaacc aggattgtaa ttgctaacca ctcgtttggt 9480
aagaatttga tgtttaaaaa aaaaaaatag cccaattttt ctaaatagtt tcaaatgtac 9540
ctcttttttt cttaattttc ttttatgcaa aatgccttaa ttttccaatt gacatgtatc 9600
caacttaagc tttcttgagt acatggacta agaattaaat tgaccaatct tgcatgtttt 9660
tagaagaatt ttatgattaa gtaagcaaat tagtggattt tgaatctatt aggctgcacc 9720
tttaatttga attatgttca tcttgcttta catttaagtc catatacatt tccagcaaac 9780
ataaggttct ctagagggtg gattcggtct tgttcatttt tatgtacaat agttgtaggt 9840
tctatcaatt agagttttat atttagaaat ttttatttaa ttggccttta gaggtttagg 9900
acatcctttc tcaaatattt taaagtctat gaatctttct atgacaaaga atgtaagtga 9960
agaaagaata ctcaaaagga ctaatacgtt ggtttttgga aatagtcttt ccaaatctta 10020
ggtcttggcg atgttgcaca tagttgagat cccgatctcg aaggtaggct ttgattattt 10080
aatatttaac aactagtaac ctcgctaatt aatcctatct aaaatggttt tgtttcgagt 10140
tttctatctc tcttttctag tttgatagct ctcgaacttt gtccaacttg attatgcttc 10200
aattattggt tcaaattatc taaaaaaatg tttatcaaat atgataaata acagctacca 10260
atttttttct gttcaaattg tctaaaaaaa attatcaaat atgagtggaa gatgtagtgg 10320
tgattgggtg ggagtgtgtc atatgtttaa ccttatatgg ataaagtctt gaagatagaa 10380
accaacagtc ttcgatcaca ttaagttgct aaaacttaaa caggttcatg aacttgaaaa 10440
catctaatca aactcgacct aaaaaattgt actaatcttg cattattgtg taataaattt 10500
attttgggtt agttgcatca aagaaattaa attacagtta ggttctccta gtttacatag 10560
tttagggata tttaaaatat tatttcagtc catatatctt tttaggggtg gagttgttta 10620
gtcgttgagt ctgctgtgca tgaagttaat aagccataaa aattgatatt ttttttatgt 10680
gttttgtttg atgattatta ggatgacttc attctcggta gacgagactt caaatcagag 10740
ttgttctagt ccagtgttag gaaaaagaaa accggttaaa ccaccatcat cggtatggga 10800
acattttata aaagtagaag gatgtgatcc taaatatcct agggctgctt gtaaacattg 10860
tggggcttca tatgcttgtg attccaaaag aaatggtacc actaatttaa aaagacattt 10920
agagaaatgt aagatgtatg taaatccatt ggaagataat gttgaaggag agggagattc 10980
tgaaagtaat ttgatgactg catcattcac tcaagaaaat tgtagaaaaa tgcttgctag 11040
gatggttatc ttggatgaat tgccatttaa gttcgtagaa agtgaagggt ttcaccaatt 11100

ttgtcgggca ttaaatccaa agtttgtgat tccatcaaga gtaactgttg caaaagattg 11160
ttttcaaatg tatatgaagg agaaaaaaaa gttaaaaaat gcattaactc gaagtggcca 11220
aagagtttgt ttaacaacgg atacgtggac ttctgtgcaa aatattaatt atatggttat 11280
aacggctcat ttcattgatg atgattggaa cttgcacaaa agaattttga acttttgtca 11340
agtagctaat cataaaggag ataccatagg tagagccatt gaaaagtgct tagaaggttg 11400
gggtattgat aggctcttta ctgtaacggt tgataatgcg agttcaaatg atgtagccat 11460
tgcctacttg gttaaaaagt ttaaaggcag aaatgggttg gtgttggatg gtgaatttat 11520
tcacattaga tgttgtgctc atattcttaa tttaattgtt agtgatgcct taaaagattt 11580
gcatgtgtct atcattcgaa tcagaaatgc tgtgaagtat gttaggtcat ctcctgctag 11640
attgcaaata tttaaagatt ttgctaaaga agataagatg tcaacaaaaa attgtcttac 11700
aatggatgtt ccgacacgat ggaattctac ttttactatg ttggatggag caattaagtg 11760
tcaaaagact tttgaaagat tggaggagca tgaccctagt tatttgccaa aggatgatat 11820
tcctactact gaagattggg ataatgcaaa agtgtttgta aagttcctaa agactttttc 11880
agaggtaaca atgaagtttt ctgcatctat gtctgtgact tcaaatatat tttttcatga 11940
actttgtttg atccaagaaa taattcgtga atactcatcg tatgagaatg cattattgag 12000
tcaaatgaca ttaagcatgc agacaaaatt caacaagtat tggggtataa ctacaagtga 12060
gaagaccaat ttattattgt atgtttctgt agttcttgac cctagataca agctagctta 12120
tgtgaattat tgttttaatg aatttttgga ggaagattgt gcaaaaatat ggacaaataa 12180
ggttgaagaa gcatttcgtc gattgtgtga tgattattat atgagaatgt caaaagaaaa 12240
atattcacaa acacaatcat gtacacctat cgaaggattt ggctttcaaa gtcaaagtga 12300
aataccttct atctcatcta gtggatctta caaggcacgt gctactgttc atgatagatt 12360
taaacaaagt aacaaaacat gtctagatga tgctaaaaca gaggtgactc gttatctgga 12420
tgaggctcgt atagattgta tgggcgatga atatttagat ttgctaactt ggtggaaggt 12480
gaatgcctct cgatttaaga tcattagcca agtagctagg gacatctaca gtattcctat 12540
atcaactgtg ccttctgagt ccgcctttag cactggagga cgggtgttag attcttttcg 12600
aagttcttta actcctcaaa ctgcagaggc actcatttgt gctcagaatt ggattcagtc 12660
taaacctttg gatgacatga ctgaagaaat tgacggggct gaagaaattg atgaaggtaa 12720
tattcttttt gaagtacgca tttaaaattt tcaattatcc catataagct aactgtttgt 12780
cattttgatc tagaattcat aaacatagga aaggagatgg aagctgcatt tgagaatttg 12840
aataatgact ctatggttta aatccttact ctcaaatctt tatttacatt tttttaaaat 12900

tattaactta ttaaatatcg aatcttgttt ttttagagtc ctgaagctca atgatggaag 12960

cactttgggt tatacgaaca aggtggtctc gtgaagatgt tcattaattc ataccttccc 13020

aattgtaaac tttgttcatg aggttccttg gttaaccttc tttatataaa agaaacaaaa 13080

gaagggctga gagattattg gttgtttgtt tgttttgttt tttgttttag ttgatttcat 13140

ggtggtttcg ttgaaccggt aacttactga ttattggttt ggtttttatt gtcttccatg 13200

cgtttgtaaa agttccccag tgttttccaa attcctttgt tatcttttat actctctact 13260

ttcaatctat ctgaaacatg gatttagcga atgcgggcga ggttggcaag gctccggtgg 13320

ctaatcaagt agctgatgtt gtttctgcta aatctagcaa tacccaagag aatgaattgg 13380

gttcaaagtt ggggacagga agcaaaagca atagggatat ggtatttcaa aggtatattt 13440

acagatcctg gatcacattt tgtcaggttc tgcttcttta ttcgtaattg tcaaagtcct 13500

taagttcact acttgaatat ttgaggccaa tgtaatttcc attccaacgg acgtatgctg 13560

cccaggtgtg actactattc tgatttatgc ttacggcttc gttacatctt ggaatcttgc 13620

ttttagcctg cttatggatt ttatttataa attgaaagct ctgatactaa caaataacag 13680

caccagcaca taattgccgt aaatggactg tactactagc ctattaaggt tttggtaata 13740

tggcaaatgc cagaatgaat tgcctggaga ctcaggttac cttggactct gagtatttta 13800

tgcttataac tatttttcta atctattatg atgtataatc tatattgaat gcaatgatat 13860

caagttgttg gctttcgtag atataaaaat aagttttatt gggttgatgg ttatactaaa 13920

aagcagtttc attgtagaat acatatcatg gttatctctc tcggatgttg tagaagtcat 13980

gcatcatcac ttgcctctaa aatgtattgg gtgatctgaa aataaatgac tgacatttgg 14040

ttttctccac actatttttg cagactgtgc ctgcttgcca aaatctctac aagaactcct 14100

tcacgtatca ctgtctccaa tatgataaga ctttaccttt tgatgacgca gttgaatttc 14160

taggtggcct ttctttatat tctcccaaat gaactataag atgacactga acaaatgact 14220

tttggttaag aacgtgtcat tagctttcta tcattcactt attccaaaag tctaactttc 14280

ctgatattgc ttgtgatttt cagagcaatg tgagagacaa ggctcgggtt ctggtgcact 14340

gcatgtcagg aaaaaatagg tttgaattag ttgtatttgt tatcctttcc catatgacag 14400

aagtttagat ttgtttaacc atcctctcta ataggttaca cttatttatt gcaggtctcc 14460

agctattgtt atagcattct tgaatattaa ataaatacta tagattccct gcagcatgct 14520

tacaagtaga gtgtgaacat aacattttag tatttttcat ttttctttgt gcctttcctg 14580

tctttctcgt gcacataact gtggctctgg attctgcttg gatccgtctg ctctttcctc 14640

ttgatggcac ttgaattgcc tatacttgtg ctgttagtgt catagcttat aagataagtt 14700

ttcctacaga ataattgcta cacaagactt catcccatag ttgataatgg gctttactga 14760

```
tgtattgtag ttgcagctgt atctgtggca taaaggtgaa ggcagtttaa tttaagattt  14820
tatgtagtac cattacgttc atctgttgag gagatctttc caatggctta gagaacttca  14880
aaaattttag cagacatacc gtcagttttc tgaattagct cccttcccta atcagttata  14940
ctgtgaaatg aaacttttaa tccttcactt tgtaataagt aaagacgacc ttgatgtagc  15000
aagtgtagtg cggtatatcc gtgcttttgg tctcatttct tgtttcctat tttcggttaa  15060
tatttaattg acagcagtta tgtgcagttt ttgtaaacaa aatatcttat gcttggtttg  15120
atgacaagta aggaccaaaa tgaatgacaa ctatggatat agaattgatt tcatggtaac  15180
atcttcttga tgagtatgta ctaacatagg gaaaatgcac ttacaaaaag ttatgatgat  15240
ctacatttct attcaagtat tgttcaaatg atgcactccc cggaacattt tctcccccctt  15300
gtgaaaatct cctccttgct aaggaaggag gaaaaaaaaa aaaaaaaaaa aaaaaaaaaa  15360
aaaaaaaaaa agacccgacc cgacccgacc caattacacc cctactcaaa ataaccaata  15420
aataaataat gctattgttt tcttttc                                      15447
```

<210> 16
<211> 352
<212> PRT
<213> Cucumis melo

<400> 16

```
Met Gly Asp Pro Asn Leu Ser Ile Asn Asn Asn Asn Asn Thr Cys Phe
1               5                   10                  15

Ser Leu Ser Pro Phe Gln His Tyr Ser Ser Ser Ser Ser Ser Ser Ser
            20                  25                  30

Ser Tyr Asn Ser His Tyr His His Asp His Leu Phe Ser Leu Ser Tyr
        35                  40                  45

Ser Asn Asp Asn Asn Ser Asn Thr Leu Lys Asn Asn Met Thr Thr Ser
    50                  55                  60

His Tyr Asn Pro Ser Ser Ser Ser Gln Val Leu Leu Pro Leu Leu Ser
65                  70                  75                  80

Leu Ser Pro Ala Arg Val Glu Gln Asp His His His His His His Gln
                85                  90                  95

Asn Ile Ile Asp His Asp His Asn Ile Ile Asp Tyr Asp Gln Asn Asp
            100                 105                 110
```

```
Val Thr Val Ala Leu His Leu Gly Leu Pro Thr Pro Ser Ser Ser Ser
        115                 120                 125

Asn Asn Asn Ser Asp Leu Ile Leu Arg Leu Ser Ser Thr Glu Ile Ser
    130                 135                 140

Asp Gln Glu Asp His Thr His Gln Leu Gln Glu Leu Ser Ser Asn Asn
145                 150                 155                 160

Ser Ile Ala Ser Asn Ser Asn Gly Val Asn Lys Gly Gln Tyr Trp Ile
                165                 170                 175

Pro Thr Pro Thr Gln Ile Leu Ile Gly Pro Thr Gln Phe Ser Cys Pro
            180                 185                 190

Leu Cys Phe Lys Thr Phe Asn Arg Tyr Asn Asn Met Gln Met His Met
        195                 200                 205

Trp Gly His Gly Ser Gln Tyr Arg Lys Gly Pro Gln Ser Leu Arg Gly
    210                 215                 220

Thr Gln Pro Thr Ala Met Leu Arg Leu Pro Cys Tyr Cys Cys Ala Ile
225                 230                 235                 240

Gly Cys Arg Asn Asn Ile Asp His Pro Arg Ser Lys Pro Leu Lys Asp
                245                 250                 255

Phe Arg Thr Leu Gln Thr His Tyr Lys Arg Lys His Gly Met Lys Pro
            260                 265                 270

Phe Thr Cys Arg Lys Cys Gly Lys Ala Phe Ala Val Arg Gly Asp Trp
        275                 280                 285

Arg Thr His Glu Lys Asn Cys Gly Lys Leu Trp His Cys Thr Cys Gly
    290                 295                 300

Ser Asp Phe Lys His Lys Arg Ser Leu Lys Asp His Ile Lys Ala Phe
305                 310                 315                 320

Gly Leu Gly His Ala Ala Tyr Gly Ile Asp Asp His His Asn His His
                325                 330                 335

His Ser Phe Asp Asn Glu Asp Asp Asp Pro Ala Ser Asp Ile Glu Thr
            340                 345                 350
```